# EUROPEAN PATENT APPLICATION

(11) **EP 3 401 336 A1**
(43) Date of publication of application: **14.11.2018**
(21) Application number: 16883432.3
(22) Date of filing: 26.12.2016
(51) Int. Cl.: C07K 16/40, C07K 16/46, C12N 15/13, A61K 39/395, A61P 3/10, A61P 9/00, A61P 9/10, A61P 25/28

(54) **PCSK9 ANTIBODY, ANTIGEN-BINDING FRAGMENT THEREOF, AND MEDICAL USES THEREOF**

(30) Priority: 05.01.2016 CN 201610010241
(71) Applicant: Jiangsu Hengrui Medicine Co., Ltd., Jiangsu 222047 (CN); Shanghai Hengrui Pharmaceutical Co., Ltd., Shanghai 200245 (CN)
(72) Inventor: QU, Xiangdong, Shanghai 200245 (CN); YE, Xin, Shanghai 200245 (CN); JIN, Houcong, Shanghai 200245 (CN); CUI, Dongbing, Shanghai 200245 (CN); HU, Qiyue, Shanghai 200245 (CN); TAO, Weikang, Shanghai 200245 (CN); ZHANG, Lianshan, Shanghai 200245 (CN); SUN, Piaoyang, Lianyungang Jiangsu 222047 (CN)
(74) Representative: Gevers & Orès
(86) International application number: PCT/CN2016/112075
(87) International publication number: WO 2017/118307

(57) **Abstract**

The present invention provides a PCSK9 antibody, an antigen-binding fragment thereof, and medical uses thereof.

## Description

### FIELD OF THE INVENTION

The present invention relates to a PCSK9 antibody, antigen-binding fragment thereof, a chimeric antibody and humanized antibody comprising CDR regions of the PCSK9 antibody, a pharmaceutical composition comprising the PCSK9 antibody and the antigen-binding fragment thereof, as well as its use as a medicament for lowering the level of blood lipid.

### BACKGROUND OF THE INVENTION

Hypercholesterolemia is a disease with abnormal metabolism of lipid, characterized in an increased level of serum cholesterol. Its main manifestation is the increased level of serum cholesterol, which causes cholesterol aggregated in vessels and consequently results in atherosclerosis formed. Abundant clinical and experimental research results have demonstrated that the abnormal metabolism of lipid is closely correlated with occurrence and development of coronary heart disease. Therefore, reducing the concentration of cholesterol in blood becomes a main means for treating and preventing atherosclerosis.

With the rapid improvement of the national standard of living in China, dyslipidemia is becoming a main factor endangering urban and rural residents of China. According to the statistic results in 2012, about 40% of deaths per year in China were attributed to cardiovascular disease. The morbidity of dyslipidemia in adults in China is 18.6%, and it is estimated now that 160 million of people have dyslipidemia. The morbidities of different types of dyslipidemia are as follows: 2.9% for hypercholesterolemia, 11.9% for hypertriglyceridemia, 7.4% for low, high density lipoproteinemia, and 3.9% for marginally increased blood cholesterol level. It was mentioned that there are 33 million of people having hypercholesterolemia in China, however, for local areas, the morbidity of dyslipidemia is far more serious than the above data, Chronic Disease Prevention and Control China Expert Consensus, by Chronic Disease Prevention and Control Branch from Disease Prevention and Control Committee, Ministry of Public Health, 2012.

At present, the medicaments clinically used for controlling lipid levels are mainly focused on statins. Liptor, as a most widely used and a best-selling cholesterol-lowering medicament, reduces the production of cholesterol by blocking the effect of cholesterol-producing enzyme in liver, and therefore increases the uptake of cholesterol from blood into liver, so that reduces the concentration of cholesterol in blood. However, Liptor has some disadvantages. Firstly, it will be understood from data, that Liptor can reduce low density lipoprotein by 30% to 40%, however, an effectively reduced blood lipid level still cannot be achieved in many patients (low density lipoprotein<50mg/dL). Secondly, there is racial difference among patients in response rate to Liptor. Because of these reasons, the patients need a more effective medicine for reducing blood lipid.

Familial hypercholesterolemia (FM) is an autosomal single-gene dominant hereditary disease, the clinical features of which are significantly increased total cholesterol (TC) and low density lipoprotein-cholesterol (LDL-c) in blood, xanthelasmata, corneal arcus and premature cardiovascular disease. Mutation in low density lipoprotein receptor (LDL receptor, LDLR) gene causes LDLR deficiency or absence, consequently LDL-c will not be transported to liver to be eliminated, and hence the level of LDL-c in blood is increased. Currently it is clear that 3 genes are corrected with occurrence of FM, which are LDLR gene, apolipoprotein B100 gene and proprotein convertase subtilisin/kexin type 9 (PCSK9) gene, respectively.

Proprotein convertase subtilisin/kexin type 9 (PCSK9) is a proprotein convertase, which is a subfamily of protease K belonging to a secretory Bacillus subtilis family. The encoded protein is synthesized as a soluble proenzyme, and is intra-molecularly processed in the endoplasmic reticulum by self-catalyzing. According to experimental results, PCSK9 promotes degradation of LDLR and thus increases the amount of LDL cholesterol in plasma, while LDL receptor mediates endocytosis process of LDL in the liver, and the latter is a main pathway to remove LDL from the circulating system. Researchers have found that PCSK9 gene mutations were detected in 12.5% hypercholesterolemia (ADH) patients. There are various types of PCSK9 mutations. According to different influences of mutations on LDL-c level regulated by PCSK9, there are two types of mutations, including loss-of-function and gain-of-function. Loss-of-function mutations are associated with low blood cholesterol level and have effect on preventing occurrence of atherosclerotic heart disease. The rates of PCSK9 mutations associated with low cholesterol are higher in population of Africans than those in other races. PCSK9 gain-of-function mutations raise plasma cholesterol level by increasing the function of PCSK9 and reducing LDLR's expression, which will cause serious hypercholesterolemia and premature coronary atherosclerotic heart disease. It is found at present that PCSK9 gain-of-function mutations include D374Y, S127R, F216L, N157K, R306S and so on. In comparison with PCSK9 wild type, the LDLR on cell surface in D374Y mutant was decreased by 36%, and in S127R mutation was decreased by 10%.

As a potential new target, PCSK9 has become a hot topic in research of hypercholesterolemia. It is important for us to further understand the mechanism of cholesterol metabolism and find new therapeutic strategy. Many multinational pharmaceutical companies are developing monoclonal antibodies against PCSK9, which increase the concentration of LDLR on the liver surface and reduce the concentration of LDL in the blood by neutralizing PCSK9 in the blood. The relevant patents are WO2011111007, WO2011072263, WO2013170367, WO2013169886, WO2013148284, WO2013091103, WO2013039958, WO2013039969, WO2013016648, WO2013008185, WO2012170607, WO2012168491, WO2012154999, WO2012109530, WO2012101251, WO2012088313, US8829165 B2, US8030457B2, US8563698B2, US8859741B2, US8871913B2, US8871914B2, US8883983B2, WO2012058137 and WO2012054438

This present invention provides PCSK9 antibodies with higher affinity, higher selectivity and higher bioactivity.

### SUMMARY OF THE INVENTION

The present invention provides a PCSK9 antibody specifically binding to PCSK9 or antigen-binding fragment thereof, comprising variable region containing at least one or more CDR regions selected from:
i) HCDR1 of GYX¹IH (SEQ ID NO: 43), where X¹ is T, D or E;
ii) HCDR2 of X²IX³PSX⁴TYTKFNQKFKD (SEQ ID NO: 44), wherein X² is Y or E; X³ is N, L, I or V; X⁴ is S, G or A;
iii) HCDR3 of AREX⁵IX⁶X⁷NYWFFDX⁸ (SEQ ID NO: 45), wherein X⁵ is R or N; X⁶ is Y or F; X⁷ is S or F; X⁸ is V or R;
iv) LCDR1 of KASQNVYX₁X₂VX₃ (SEQ ID NO: 46), wherein X₁ is T or W; X₂ is A or E; X₃ is A, D or V;
v) LCDR2 of X₄X₅X₆NRYT (SEQ ID NO: 47), wherein X₄ is S, E or Q; X₅ is A or M; X₆ is S or V; and
vi) LCDR3 of QQX₇SX₈X₉PX₁₀T (SEQ ID NO: 48), wherein X₇ is Y, F or L; X₈ is S or W; X₉ is Y, F, Q or S; X₁₀ is Y, D or E.

In another preferred embodiment of the present invention, the PCSK9 antibody specifically binding to PCSK9 or antigen-binding fragment thereof according to the present invention, comprises HCDR1, HCDR2 and HCDR3 as shown in SEQ ID NO: 43, SEQ ID NO: 44 and SEQ ID NO: 45, respectively.

In another preferred embodiment of the present invention, the PCSK9 antibody specifically binding to PCSK9 or antigen-binding fragment thereof according to the present invention, comprises LCDR1, LCDR2 and LCDR3 as shown in SEQ ID NO: 46, SEQ ID NO: 47 and SEQ ID NO: 48, respectively.

Amino acid mutations can be made in the CDR regions of the above SEQ ID NOs: 43-48 according to the present invention by means of affinity maturation, so as to obtain higher activity.

In another preferred embodiment of the present invention, in the PCSK9 antibody specifically binding to PCSK9 or antigen-binding fragment thereof according to the present invention, the HCDR1 is selected from the sequences of SEQ ID NO: 14, SEQ ID NO: 20 or SEQ ID NO: 21, or the sequences having at least 95% identity to the above sequences.

HCDR2 is selected from the sequences of SEQ ID NO: 15, SEQ ID NO: 22, SEQ ID NO: 23, SEQ ID NO: 24, SEQ ID NO: 25, SEQ ID NO: 26 or SEQ ID NO: 27, or the sequences having at least 95% identity to the above sequences;

HCDR3 is selected from the sequences of SEQ ID NO: 16, SEQ ID NO: 28, SEQ ID NO: 29 or SEQ ID NO: 30, or the sequences having at least 95% identity to the above sequences.

In another preferred embodiment of the present invention, in the PCSK9 antibody specifically binding to PCSK9 or antigen-binding fragment thereof according to the present invention, the LCDR1 is selected from the sequences of SEQ ID NO: 17, SEQ ID NO: 31, SEQ ID NO: 32, SEQ ID NO: 33 or SEQ ID NO: 34, or the sequences having at least 95% identity to the above sequences;

LCDR2 is selected from the sequences of SEQ ID NO: 18, SEQ ID NO: 35, SEQ ID NO: 36 or SEQ ID NO: 37, or the sequences having at least 95% identity to the above sequences;

LCDR3 is selected from the sequences of SEQ ID NO: 19, SEQ ID NO: 38, SEQ ID NO: 39, SEQ ID NO: 40, SEQ ID NO: 41 or SEQ ID NO: 42, or the sequences having at least 95% identity to the above sequences.

In another preferred embodiment of the present invention, the PCSK9 antibody specifically binding to PCSK9 or antigen-binding fragment thereof according to the present invention, comprises six CDR regions selected from:
1) Six CDR regions as shown in SEQ ID NO: 14, SEQ ID NO: 15, SEQ ID NO: 16, SEQ ID NO: 17, SEQ ID NO: 18 and SEQ ID NO: 19, respectively;
2) Six CDR regions as shown in SEQ ID NO: 14, SEQ ID NO: 22, SEQ ID NO: 16, SEQ ID NO: 17, SEQ ID NO: 18 and SEQ ID NO: 19, respectively;
3) Six CDR regions as shown in SEQ ID NO: 14, SEQ ID NO: 23, SEQ ID NO: 16, SEQ ID NO: 17, SEQ ID NO: 18 and SEQ ID NO: 19, respectively;
4) Six CDR regions as shown in SEQ ID NO: 20, SEQ ID NO: 15, SEQ ID NO: 16, SEQ ID NO: 17, SEQ ID NO: 18 and SEQ ID NO: 19, respectively;
5) Six CDR regions as shown in SEQ ID NO: 14, SEQ ID NO: 15, SEQ ID NO: 28, SEQ ID NO: 17, SEQ ID NO: 18 and SEQ ID NO: 19, respectively;
6) Six CDR regions as shown in SEQ ID NO: 14, SEQ ID NO: 15, SEQ ID NO: 29, SEQ ID NO: 17, SEQ ID NO: 18 and SEQ ID NO: 19, respectively;
7) Six CDR regions as shown in SEQ ID NO: 14, SEQ ID NO: 15, SEQ ID NO: 16, SEQ ID NO: 17, SEQ ID NO: 35 and SEQ ID NO: 19, respectively;
8) Six CDR regions as shown in SEQ ID NO: 14, SEQ ID NO: 15, SEQ ID NO: 16, SEQ ID NO: 17, SEQ ID NO: 36 and SEQ ID NO: 19, respectively;
9) Six CDR regions as shown in SEQ ID NO: 14, SEQ ID NO: 15, SEQ ID NO: 16, SEQ ID NO: 31, SEQ ID NO: 18 and SEQ ID NO: 19, respectively;
10) Six CDR regions as shown in SEQ ID NO: 14, SEQ ID NO: 15, SEQ ID NO: 16, SEQ ID NO: 32, SEQ ID NO: 18 and SEQ ID NO: 19, respectively;
11) Six CDR regions as shown in SEQ ID NO: 14, SEQ ID NO: 15, SEQ ID NO: 16, SEQ ID NO: 17, SEQ ID NO: 18 and SEQ ID NO: 38, respectively;
12) Six CDR regions as shown in SEQ ID NO: 14, SEQ ID NO: 15, SEQ ID NO: 16, SEQ ID NO: 17, SEQ ID NO: 18 and SEQ ID NO: 39, respectively;
13) Six CDR regions as shown in SEQ ID NO: 21, SEQ ID NO: 24, SEQ ID NO: 16, SEQ ID NO: 17, SEQ ID NO: 18 and SEQ ID NO: 19, respectively;
14) Six CDR regions as shown in SEQ ID NO: 14, SEQ ID NO: 25, SEQ ID NO: 16, SEQ ID NO: 17, SEQ ID NO: 18 and SEQ ID NO: 19, respectively;
15) Six CDR regions as shown in SEQ ID NO: 14, SEQ ID NO: 15, SEQ ID NO: 30, SEQ ID NO: 17, SEQ ID NO: 37 and SEQ ID NO: 19, respectively;
16) Six CDR regions as shown in SEQ ID NO: 14, SEQ ID NO: 15, SEQ ID NO: 16, SEQ ID NO: 17, SEQ ID NO: 18 and SEQ ID NO: 40, respectively;
17) Six CDR regions as shown in SEQ ID NO: 14, SEQ ID NO: 15, SEQ ID NO: 16, SEQ ID NO: 17, SEQ ID NO: 18 and SEQ ID NO: 41, respectively;
18) Six CDR regions as shown in SEQ ID NO: 14, SEQ ID NO: 26, SEQ ID NO: 16, SEQ ID NO: 17, SEQ ID NO: 18 and SEQ ID NO: 19, respectively;
19) Six CDR regions as shown in SEQ ID NO: 14, SEQ ID NO: 27, SEQ ID NO: 16, SEQ ID NO: 17, SEQ ID NO: 18 and SEQ ID NO: 19, respectively;
20) Six CDR regions as shown in SEQ ID NO: 14, SEQ ID NO: 15, SEQ ID NO: 16, SEQ ID NO: 33, SEQ ID NO: 18 and SEQ ID NO: 19, respectively;
21) Six CDR regions as shown in SEQ ID NO: 14, SEQ ID NO: 15, SEQ ID NO: 16, SEQ ID NO: 34, SEQ ID NO: 18 and SEQ ID NO: 19, respectively;
22) Six CDR regions as shown in SEQ ID NO: 14, SEQ ID NO: 15, SEQ ID NO: 16, SEQ ID NO: 17, SEQ ID NO: 18 and SEQ ID NO: 42, respectively; and
23) Six CDR regions as shown in SEQ ID NO: 14, SEQ ID NO: 15, SEQ ID NO: 30, SEQ ID NO: 17, SEQ ID NO: 18 and SEQ ID NO: 19, respectively.

In another preferred embodiment of the present invention, the PCSK9 antibody specifically binding to PCSK9 or antigen-binding fragment thereof according to the present invention, is a murine-derived antibody or a fragment thereof.

In another preferred embodiment of the present invention, the PCSK9 antibody specifically binding to PCSK9 or antigen-binding fragment thereof according to the present invention, is a chimeric antibody or a fragment thereof.

In another preferred embodiment of the present invention, the PCSK9 antibody specifically binding to PCSK9 or antigen-binding fragment thereof according to the present invention, is humanized antibody or a fragment thereof.

In another preferred embodiment of the present invention, with respect to the PCSK9 antibody specifically binding to PCSK9 or antigen-binding fragment thereof according to the present invention, the heavy chain of the humanized antibody comprises heavy chain variable region, wherein the FR sequence is derived from a combination sequence of human germline heavy chains IGHV1-2*02 and hjh6.1, or mutant sequences thereof; wherein the FR sequence comprises FR1, FR2, FR3 from IGHV1-2*02 and FR4 from hjh6.1, or mutant sequence thereof, or comprises amino acid sequences having at least 95% identity to the above sequences.

In another preferred embodiment of the present invention, with respect to the PCSK9 antibody or antigen-binding fragment thereof according to the present invention, the heavy chain sequence of the humanized antibody is a variant of the sequence set forth in SEQ ID NO: 10; wherein the variant preferably has 0-10 amino acid change(s) in the heavy chain variable region. The amino acid change may be modification based on the technology available in the art for improving affinity or half-life of the antibody, for example, modifying the CDR amino acid sequences by using affinity maturation or modifying the FR amino acid sequences by using back-mutations.

In another preferred embodiment of the present invention, with respect to the PCSK9 antibody specifically binding to PCSK9 or antigen-binding fragment thereof according to the present invention, the humanized antibody comprises heavy chain FR region having 0-10 amino acid back-mutations, wherein the back-mutation is preferably selected from one or more back-mutations consisting of R72A, T74K, V68A, M70L, M48V, G49A, R67K and R38K.

In another preferred embodiment of the present invention, with respect to the PCSK9 antibody specifically binding to PCSK9 or antigen-binding fragment thereof according to the present invention, the PCSK9 antibody comprises VH, the amino acid sequence of which is selected from the group consisting of SEQ ID NO: 12, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57 and SEQ ID NO: 58, or selected from the sequences having at least 95% identity to the above sequences.

In another preferred embodiment of the present invention, with respect to the PCSK9 antibody or antigen-binding fragment thereof according to the present invention, the light chain FR sequence of the humanized antibody light chain variable region is derived from combination sequence of human germline light chains IGKV1-39*01 and hjk4.1, or the mutant sequences thereof. The light chain FR comprises FR1, FR2, FR3 from IGKV1-39*01 and FR4 from hjk4.1 or the mutant sequence thereof, or a sequence having at least 95% identity to the above sequences. In another preferred embodiment of the present invention, with respect to the PCSK9 antibody specifically binding to PCSK9 or antigen-binding fragment thereof according to the present invention, the humanized antibody comprises a light chain variable region of SEQ ID NO: 11 or a variant thereof; the variant means the presence of 1, 2, 3, 4, 5, 6, 7, 8, 9 or 10 amino acid changes in SEQ ID NO:11. The amino acid change may be modification based on the technology available in the art for improving affinity or half-life of the antibody, for example, modifying the CDR amino acid sequences by using affinity maturation or modifying the FR amino acid sequences by using back-mutations.

In another preferred embodiment of the present invention, with respect to the PCSK9 antibody specifically binding to PCSK9 or antigen-binding fragment thereof according to the present invention, the humanized antibody comprises light chain FR region having 0-10 amino acid back-mutations, wherein the back-mutation is preferably selected from one or more amino acid back-mutation consisting of Q3V, A43S and Y87F.

In another preferred embodiment of the present invention, the PCSK9 antibody or antigen-binding fragment thereof according to the present invention, comprises

VL, the amino acid sequence of which is selected from the group consisting of SEQ ID NO: 13, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69 and SEQ ID NO: 70, or selected from the sequences having at least 95% identity to the above sequences.

In another preferred embodiment of the present invention, the PCSK9 antibody specifically binding to PCSK9 or antigen-binding fragment thereof according to the present invention, comprises VH and VL selected from the following groups:
1) VH of SEQ ID NO: 12 and VL of SEQID NO: 13;
2) VH of SEQ ID NO: 49 and VL of SEQID NO: 13;
3) VH of SEQ ID NO: 50 and the VL of SEQID NO: 13;
4) VH of SEQ ID NO: 51 and VL of SEQID NO: 13;
5) VH of SEQ ID NO: 52 and VL of SEQID NO: 13;
6) VH of SEQ ID NO: 53 and VL of SEQID NO: 13;
7) VH of SEQ ID NO: 12 and VL of SEQID NO: 59;
8) VH of SEQ ID NO: 12 and VL of SEQID NO: 60;
9) VH of SEQ ID NO: 12 and VL of SEQID NO: 61;
10) VH of SEQ ID NO: 12 and VL of SEQID NO: 62;
11) VH of SEQ ID NO: 12 and VL of SEQID NO: 63;
12) VH of SEQ ID NO: 12 and VL of SEQID NO: 64;
13) VH of SEQ ID NO: 54 and VL of SEQID NO: 13;
14) VH of SEQ ID NO: 55 and VL of SEQID NO: 13;
15) VH of SEQ ID NO: 56 and VL of SEQID NO: 65;
16) VH of SEQ ID NO: 12 and VL of SEQID NO: 66;
17) VH of SEQ ID NO: 12 and VL of SEQID NO: 67;
18) VH of SEQ ID NO: 57 and VL of SEQID NO: 13;
19) VH of SEQ ID NO: 58 and VL of SEQID NO: 13;
20) VH of SEQ ID NO: 12 and VL of SEQID NO: 68;
21) VH of SEQ ID NO: 12 and VL of SEQID NO: 69;
22) VH of SEQ ID NO: 12 and VL of SEQID NO: 70; and
23) VH of SEQ ID NO: 56 and VL of SEQID NO: 13.

In another preferred embodiment of the present invention, with respect to the PCSK9 antibody or antigen-binding fragment thereof according to the present invention, the heavy chain of chimeric antibody or humanized antibody further comprises heavy chain constant region derived from human IgG1, IgG2, IgG3 or IgG4 or a variant thereof, or a sequence having at least 95% identity to sequences thereof; preferably comprises heavy chain constant region derived from human IgG1, IgG2 or IgG4 or heavy chain constant region of IgG1, IgG2 or IgG4 variants wherein the amino acid mutations prolong the half-life of the antibody in the serum, most preferably comprises IgG1, IgG2 or IgG4 heavy chain constant region introduced with YTE mutation.

The light chain of the chimeric antibody or humanized antibody further comprises constant region derived from human κ chain, human λ chain or a variant thereof, or a sequence having at least 95% identity to sequences thereof.

In another preferred embodiment of the present invention, the PCSK9 antibody specifically binding to PCSK9 or antigen-binding fragment thereof according to the present invention, comprises:
1) heavy chain sequence of SEQ ID NO: 71 and light chain sequence of SEQ ID NO: 73; or
2) heavy chain sequence of SEQ ID NO: 75 and light chain sequence of SEQ ID NO: 73.

On the other hand, the present invention provides a PCSK9 antibody or an antigen-binding fragment thereof, comprising one or more CDRs selected from HCDR as shown in SEQ ID NO: 14, SEQ ID NO: 15 or SEQ ID NO: 16, or HCDR as shown in sequence having at least 95% identity to SEQ ID NO: 14, SEQ ID NO: 15 or SEQ ID NO: 16; and LCDR as shown in SEQ ID NO: 17, SEQ ID NO:18 or SEQ ID NO: 19, or LCDR as shown in sequence having at least 95% identity to SEQ ID NO: 17, SEQ ID NO:18 or SEQ ID NO: 19. The sequence having at least 95% identity can be obtained by affinity maturation of CDR region, such as HCDR1 selected from SEQ ID NO: 20 and SEQ ID NO: 21, or HCDR2 selected from SEQ ID NOs: 22-27, or HCDR3 selected from SEQ ID NOs: 28-30; or LCDR1 selected from SEQ ID NOs: 31-34, or LCDR2 selected from SEQ ID NOs: 35-37, or LCDR3 selected from SEQ ID NOs: 38-42. The HCDRs of present invention preferably comprise SEQ ID NO: 21, SEQ ID NO: 24 and SEQ ID NO: 16, and preferably the LCDRs comprise SEQ ID NO: 17, SEQ ID NO: 18 and SEQ ID NO: 19.

The present invention further provides a DNA molecule encoding the PCSK9 antibody or the antigen-binding fragment thereof as described above.

The present invention further provides an expression vector comprising the DNA molecule as described above.

The present invention further provides a host cell transformed with the expression vector as described above, wherein the host cell is selected from the group consisting of prokaryotic cell and eukaryotic cell, preferably eukaryotic cell, more preferably mammalian cell.

The present invention further provides a method for preparing a PCSK9 antibody, comprising culturing the host cell as described above under the condition appropriate for expressing a nucleic acid encoding the PCSK9 antibody as described above, and/or recovering the PCSK9 antibody from the host cell.

The present invention further provides a pharmaceutical composition, comprising a therapeutically effective amount of the PCSK9 antibody or antigen-binding fragment thereof as described above, and one or more pharmaceutically acceptable carrier, diluent or excipient.

The present invention further provides use of the PCSK9 antibody or antigen-binding fragment thereof, or the pharmaceutical composition as described above, in preparation of a medicament for the treatment of a disease or a condition mediated by PCSK9, wherein the disease or the condition is preferably cholesterol related diseases (including "serum cholesterol related diseases"); the disease or the condition is more preferably selected from the group consisting of hypercholesterolemia, heart disease, metabolic syndrome, diabetes, coronary heart disease, stroke, cardiovascular disease, alzheimer disease and general dyslipidemia; most preferably selected from hypercholesterolemia, dyslipidemia, atherosclerosis, CVD or coronary heart disease.

The exemplary diseases which can be diagnosed with the antibody according to the present invention include cholesterol related diseases (including "serum cholesterol related diseases"), including any one or more disease selected from the group consisting of hypercholesterolemia, heart disease, metabolic syndrome, diabetes, coronary heart disease, stroke, cardiovascular disease, alzheimer disease and general dyslipidemia (characterized in an increased level of total serum cholesterol, LDL, triglyceride, very low density lipoprotein (VLDL) and/or a decreased level of HDL).

On one hand, the present invention provides a method of treating or preventing hypercholesterolemia and/or at least one symptom selected from dyslipidemia, atherosclerosis, cardiovascular disease (CVD) or coronary heart disease in an individual, wherein the method comprises administrating an effective amount of PCSK9 antibody to the individual. The present invention also provides use of an effective amount of PCSK9 antibody against extracellular or circulating PCSK9 in preparation of a medicament, wherein the medicament is for treating or preventing hypercholesterolemia and/or at least one symptom selected from dyslipidemia, atherosclerosis, CVD or coronary heart disease in an individual.

Any embodiment or combination thereof described in the present invention is suitable for any and all PCSK9 antibodies, methods and uses according to the present invention.

### BRIEF DESCRIPTION OF THE DRAWINGS

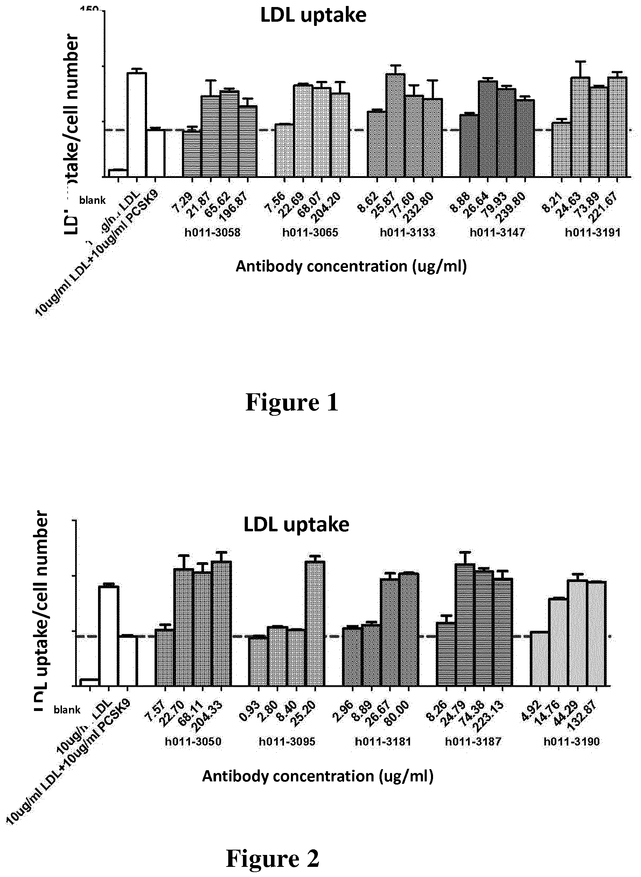

Figure 1: Changes in LDL uptake by HepG2 cells under various concentrations of PCSK9 antibodies (h011-3058, h011-3065, h011-3133, h011-3147, h011-3191). The results show that PCSK9 antibodies can promote LDL uptake by HepG2 cells.
Figure 2: Changes in LDL uptake by HepG2 cells under various concentrations of PCSK9 antibodies (h011-3050, h011-3095, h011-3181, h011-3187, h011-3190). The results show that PCSK9 antibodies can promote LDL uptake by HepG2 cells.

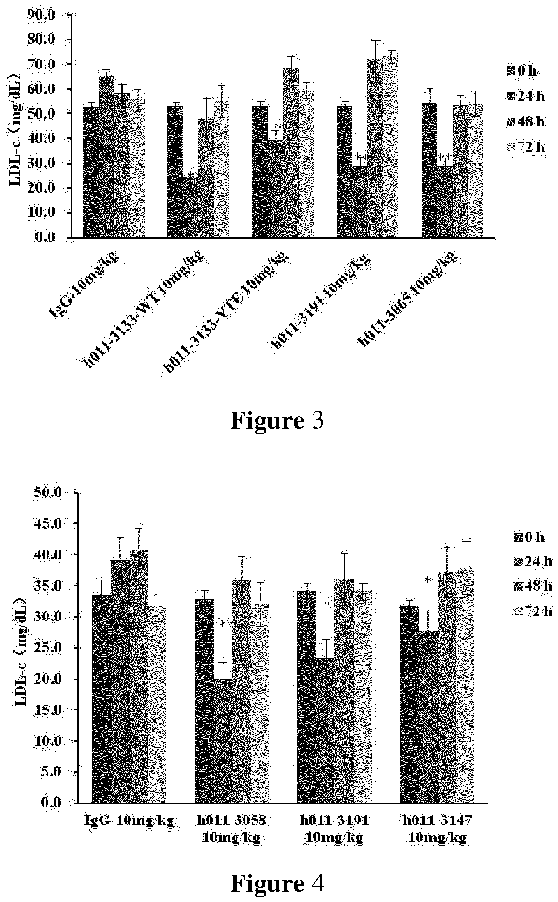

Figure 3: Changes in serum concentrations of LDL-c after injection with various PCSK9 antibodies (h011-3133-WT, h011-3133-YTE, h011-3191, h011-3065) in mice (*:p<0.05, vs IgG, **:p<0.01, vs IgG). The results show that PCSK9 antibodies can reduce serum concentration of LDL-c in mice overexpressing human PCSK9.
Figure 4: Changes in serum concentrations of LDL-c after injection with various PCSK9 antibodies (h011-3058, h011-3191, h011-3147) in mice (*:p<0.05, vs IgG, **:p<0.01, vs IgG). The results show that PCSK9 antibodies can reduce serum concentration of LDL-c in mice overexpressing human PCSK9.

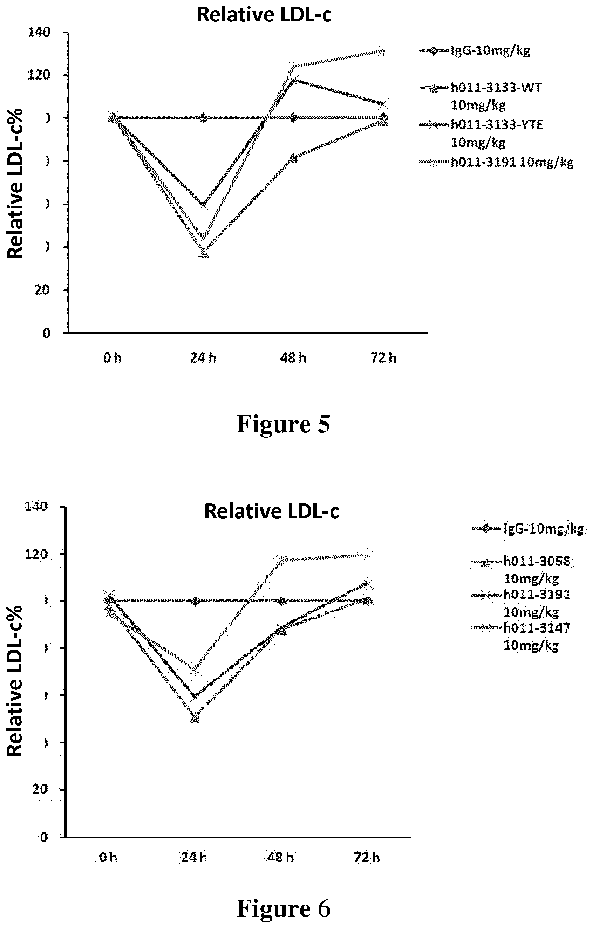

Figure 5: Changes in relative serum concentrations of LDL-c (vs that in IgG group) after injection with various PCSK9 antibodies (h011-3133-WT, h011-3133-YTE, h011-3191) in mice. The results show that compared to the IgG group, PCSK9 antibodies can reduce serum concentration of LDL-c in mice overexpressing human PCSK9.
Figure 6: Changes in relative serum concentrations of LDL-c (vs that in IgG group) after injection with various PCSK9 antibodies (h011-3058, h011-3191, h011-3147) in mice. The results show that compared to the IgG group, PCSK9 antibodies can reduce serum concentration of LDL-c in mice overexpressing human PCSK9.

### DETAILED DESCRIPTION OF THE INVENTION

The description or technology and method of the present invention are generally well understood and usually used by those skilled artisans in the art, such as those widely used methods described in the following literatures: Sambrook et al., Molecular Cloning: A Laboratory Manual version 3 (2001); Cold Spring Harbor Laboratory Press, Cold Spring Harbor, N.Y.CURRENT PROTOCOL SINMOLECULAR BIOLOGY (The latest experimental method in molecular biology) (F.M. Ausubel et al., Ed., (2003)); METHOD SINENZYMOLOGY (Academic Press, Inc.): PCR2: A PRACTICAL APPROACH (PCR2: practical method) (M. J. MacPherson, B. D. Hames and G.R.Taylor Ed. (1995)), Harlow and Lane Ed. (1988); ANTIBODIES, A LABORATORY MANUAL and ANIMAL CELL CULTURE (R.I. Freshney Ed. (1987)); Oligonucleotide Synthesis (M.J. Gait Ed. 1984); Methods in Molecular Biology, Humana Press; Cell Biology: A Laboratory Notebook (J.E.C ellis Ed. 1998) Academic Press; Animal Cell Culture (R.I. Freshney) Ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths and D.G. Newell Ed., 1993-8) J. Wiley and Sons; Handbook of Experimental Immunology (D.M. Weir and C.C. Blackwell Ed.); Gene Transfer Vectors for Mammalian Cells (J.M. Miller and M.P. Calos Ed., 1987); PCR: The Polymerase Chain Reaction (Mullis et al., Ed. 1994); Current Protocols in Immunology (J.E. Coligan er al., Ed., 1991); Short Protocols in Molecular Biology (Wiley and Sons, 1999); Immunobiology (C.A.Janeway and P.Travers, 1997); Antibodies (P. Finch, 1997); Antibodies: A Practical Approach (D. Catty Ed., IRL Press, 1988-1989); Monoclonal Antibodies: A Practical Approach (P. Shepherd and C.Dean Ed., Oxford University Press, 2000); Using Antibodies: A Laboratory Manual (E. Harlow and D. Lane(Cold Spring Harbor Laboratory Press, 1999); The Antibodies (M. Zanetti and J.D. Capra Ed., Harwood Academic Publishers, 1995); and Cancer: Principles and Practice of Oncology (V.T. DeVita et al., Ed., J.B. Lippincott Company, 1993).

### DEFINITION

Unless specifically defined elsewhere in this document, all the technical and scientific terms used herein have the same meaning as commonly understood by one of ordinary skilled artisan in the art to which this invention belongs. Singleton et al., Dictionary of Microbiology and Molecular Biology, version 2. J. Wiley&Sons (New York, N.Y.1994), and March, Advanced Organic Chemistry Reactions, Mechanisms and Structure, version 4, John Wiley&Sons (New York, N.Y.1992). Many terms used in the present invention provide some guidance to those skilled artisans in the art. All references cited herein (including patent applications and publications) are completely incorporated by reference.

For the purposes of interpreting this specification, the following definitions will be used, and where appropriate, terms used in the singular may also include the plural and vice versa. It should be understood that the terminology used herein is for the purpose of describing particular embodiments only and is not intended to be limiting. In the event that any of the definitions described below conflict with any of the documents incorporated by reference herein, the definitions described below shall prevail.

The term "Proprotein convertase subtilisin/kexin type 9 (PCSK9)", "PCSK9" or "NARC-1" as used herein, refers to any native PCSK9 from any vertebrate source, including mammals such as primates (e.g. humans) and rodents (e.g., mice and rats), unless otherwise indicated. The term encompasses "full-length," unprocessed PCSK9 as well as any form of PCSK9 produced or processed by the cells. The term also encompasses naturally occurring variants of PCSK9, e.g., splice variants or allelic variants.

In this specification and the claims, the numbering of residues in the heavy chain of an immunoglobulin is according to EU index as described in Kabat et al., Sequences of Proteins of Immunological Interest, 5th Ed. Public Health Service, National Institutes of Health, Bethesda, MD, 1991. The above literature is incorporated herein by reference. "The EU index as in Kabat" refers to the numbering of residues in the human IgG1 EU antibody.

The term "variable region" or "variable domain" refers to the antibody heavy or light chain domain that is involved in binding of the antibody to an antigen. The variable domains of the heavy chain and light chain (VH and VL, respectively) of a native antibody generally have similar structures, wherein each domain comprises four conserved framework regions (FRs) and three hypervariable regions (CDRs). (See, e.g., Kindt Kuby et al. Immunology, 6th ed., W.H. Freeman and Co., page 91 (2007).) Single VH or VL domain may be sufficient to confer antigen-binding specificity. Furthermore, antibodies binding to a particular antigen may be isolated by using a VH or VL domain from an antibody binding to the antigen to screen a library of complementary VL or VH domains, respectively. See, e.g., Portolano et al., J. Immunol. 150:880-887 (1993); Clarkson et al, Nature 352:624-628 (1991).

The term "hypervariable region" or "HVR," as used herein, refers to each region of an antibody variable domain which are hypervariable in sequence and/or form structurally defined loops ("hypervariable loops"). Generally, native four-chain antibodies comprise six CDRs; three within the VH (HI, H2, H3), and three within the VL (LI, L2, L3). CDRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (LI), 50-52 (L2), 91-96 (L3), 26-32 (HI), 53-55 (H2), and 96-101 (H3) (Chothia and Lesk, J. Mol. Biol. 196:901-917 (1987)). Exemplary CDRs (LCDR1, LCDR2, LCDR3, HCDR1, HCDR2, and HCDR3) occur at amino acid residues 24-34 of LI, 50-56 of L2, 89-97 of L3, 31-35B of H1, 50-65 of H2, and 95-102 of H3. (Kabat et al, Sequences of Proteins of Immunological Interest, 5th Ed. Public Health Service, National Institutes of Health, Bethesda, MD (1991)). With the exception of CDR1 in VH, CDRs generally comprise the amino acid residues that form the hypervariable loops. CDRs also comprise "specificity determining residues," or "SDRs," which are residues that contact with antigen. SDRs are contained within CDR region called as truncated-CDR, or a-CDR. Exemplary a-CDRs (a-LCDR1, a-LCDR2, a-LCDR3, a-HCDR1, a-HCDR2, and a-HCDR3) occur at amino acid residues 31- 34 of LI, 50-55 of L2, 89-96 of L3, 31-35B of H1, 50-58 of H2, and 95-102 of H3. (See Almagro and Fransson, Front. Biosci. 13: 1619-1633 (2008)). Unless otherwise indicated, CDR residues and other residues in the variable domain (e.g., FR residues) are numbered herein according to Kabat et al., supra.

The terms "anti-PCSK9 antibody", "anti-PCSK9", "PCSK9 antibody" or "antibody binding to PCSK9" are used interchangeably herein, and refer to an antibody capable of binding to PCSK9 with sufficient affinity and capable of being used as a diagnostic and/or therapeutic agent during targeting PCSK9.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

The terms "full length antibody," "intact antibody," and "whole antibody" are used herein interchangeably to refer to an antibody having a structure substantially similar to a native antibody structure or having heavy chains that contain an Fc region as defined herein.

The antigen-binding fragment of the present invention is selected from Fab, Fab'-SH, Fv, scFv or (Fab')₂ fragments.

An "antigen-binding fragment" refers to a molecule other than an intact antibody, and it comprises a portion of an intact antibody that binds to the antigen to which the intact antibody binds. Examples of antigen-binding fragment include but are not limited to Fv, Fab, Fab', Fab'-SH, F(ab')2; diabodies; linear antibodies; single-chain antibody molecules (e.g. scFv); and multispecific antibodies formed by antigen-binding fragments. Digesting antibodies with papain will produce two identical antigen-binding fragments, called as "Fab" fragments, each of which has a single antigen-binding site, and a residual "Fc" fragment, the name reflects its ability readily to be crystallized. Pepsin treatment yields an F(ab')2 fragment having two antigen-binding sites and is still capable of cross-linking with an antigen.

Fab fragments also contain constant domains of the light chain and the first constant domain (CH1) of the heavy chain, in addition to heavy chain variable domains and light chain variable domains. Fab' fragments differ from Fab fragments by the addition of a few residues, including one or more cysteine residues from the antibody hinge region, at the carboxyl terminus of the heavy chain CH1 domain. Fab'-SH is the designation herein for Fab' in which the cysteine residue(s) within the constant domains carry a free thiol group. F(ab')2 antibody fragment was originally produced as a pair of Fab' fragments, wherein hinge cysteine was located between the Fab' fragments. Other chemical couplings of antibody fragments are also known.

The term "Fc region" herein is used to define a C-terminal region of the immunoglobulin heavy chain, wherein at least a portion of the constant region is contained. The term includes native Fc region and variant Fc region sequence. In one embodiment, human IgG heavy chain Fc region extends from Cys226, or from Pro230, to the carboxyl-terminus of the heavy chain. However, the C-terminal lysine (Lys447) may or may not be present within the Fc region. Unless otherwise specified herein, numbering of amino acid residues in the Fc region or constant region is according to the EU numbering system, also called as the EU index, as described in Kabat et al., Sequences of Proteins of Immunological Interest, 5th Ed. Public Health Service, National Institutes of Health, Bethesda, MD, 1991. Fc region is essential for the effector functions of antibodies. The effector functions include initiating complement-dependent cytotoxicity (CDC), initiating phagocytosis and antibody-dependent cell-mediated cytotoxicity (ADCC), and transferring antibodies across cellular barriers via transcytosis. In addition, Fc region is critical for maintaining the serum half-life of an antibody of class IgG (Ward and Ghetie, Ther. Immunol. 2:77-94 (1995)). Studies have found that the serum half-life of an IgG antibody is mediated by binding of Fc to the neonatal Fc receptor (FcRn). FcRn is a heterodimer consisting of a transmembrane α chain and a soluble β chain (β2-microglobulin). A method of producing an antibody with a decreased biological half-life by introducing mutations into the DNA fragment encoding the antibody was disclosed in U.S. Patent No. 6,165,745. The mutations include amino acid substitutions at position 253, 310, 311, 433, or 434 within the Fc-hinge domain. A composition comprising a mutant IgG molecule was disclosed in U.S. Patent No. 6,277,375 B1, the molecule has an increased serum half-life relative to the wild-type IgG, wherein the mutant IgG molecule comprises the following amino acid substitutions: threonine to leucine at position 252, threonine to serine at position 254, or threonine to phenylalanine at position 256(M252Y, S254T and T256E). A mutant IgG with amino acid substitutions at position 433, 435, or 436 is also disclosed. An antibody variant comprising IgG Fc region was disclosed in U.S. Patent No. 6,528,624, the variant comprises amino acid substitutions at one or more of amino acid positions 270, 322, 326, 327, 329, 331, 333, and 334 within the human IgG Fc region. A modified IgG was disclosed in WO 02/060919 A2, the modified IgG comprises an IgG constant domain comprising one or more amino acid modifications relative to a wild-type IgG constant domain, wherein the modified IgG has an increased half-life compared to IgG having the wild-type IgG constant domain, and wherein the one or more amino acid modifications are located at one or more of positions 251, 253, 255, 285-290, 308-314, 385-389, and 428-435. Specifically, the "YTE" or "YET mutation" described herein refers to a mutation combination within the Fc region of IgG1, for promoting the binding of the Fc region to human FcRn, prolonging the half-life of the antibody in human serum. The YTE mutant contains a combination of three "YTE mutations": M252Y, S254T and T256E. Numbering of residues is according to EU Numbering System, which is also referred to as EU index (refer to U.S. Patent No. 7,658,921), such as numbering of IgG heavy chains in Kabat et al. Compared to wild-type antibodies, YTE mutant antibodies have greatly prolonged half-life in serum, e.g., Dall'Acqua et al, J. Biol. Chem. 281: 23514-24 (2006) and US Patent No. 7,083,784.

"Fv" is a minimum antigen-binding fragment comprising complete antigen-binding site. In one embodiment, a double-chain Fv consists of one heavy chain variable domain and one light chain variable domain tightly and non-covalently associated to form a dimer. In a single-chain Fv (scFv), one heavy chain variable domain and one light chain variable domain can be covalently linked via a flexible peptide linker such that the light chain and heavy chain can be associated in a "dimeric" structure similar to that of a double-chain Fv. It is in such configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind to antigen, the affinity is lower than that of the entire binding site.

"Single-chain Fv" or "scFv" antigen-binding fragment comprises VH and VL domains of an antibody, wherein these domains are present as a single polypeptide chain. Generally, the scFv polypeptide further comprises a polypeptide linker between VH and VL domains which enables the scFv to form the desired structure for antigen binding. For a review of scFv, see Pluckthun, in The Pharmacology of Monoclonal Antibodies, vol. 113, Rosenburg and Moore ed., Springer- Verlag, New York, pp. 269-315 (1994).

The term "chimeric antibody", is an antibody which is formed by fusing the variable region of a murine antibody with the constant region of human antibody, so as to alleviate the murine antibody-induced immune response. To establish a chimeric antibody, a hybridoma secreting specific murine monoclonal antibody is first established, variable region genes are then cloned from mouse hybridoma cells, and then constant region genes of human antibody are cloned as desired, the mouse variable region genes are ligated with human constant region genes to form a chimeric gene which can be inserted into human vector, and finally the chimeric antibody molecule is expressed in an eukaryotic or prokaryotic industrial system. In a preferred embodiment of the present invention, the light chain of PCSK9 chimeric antibody further comprises light chain Fc regions derived from human κ, λ chain or a variant thereof. The heavy chain of PCSK9 chimeric antibody further comprises heave chain Fc regions derived from human IgG1, IgG2, IgG3 or IgG4, or a variant thereof, preferably comprises heavy chain constant regions derived from human IgG1, IgG2, IgG3 or IgG4, or preferably comprises heavy chain constant region derived from human IgG1, IgG2 or IgG4 variants with prolonged half-life in serum via amino acid mutations (e.g., YTE mutations).

"Human antibody" is an antibody having amino acid sequences corresponding to those of an antibody produced from human or human cells or derived from non-human sources that utilize human antibody repertoires or other human antibody-encoding sequences. Such definition of human antibody specifically excludes humanized antibody comprising non-human antigen-binding residues.

"Humanized" antibody refers to a chimeric antibody comprising amino acid residues from non-human CDRs and amino acid residues from human FRs. In certain embodiments, humanized antibody will comprise substantially all of at least one, and typically two, variable domains, in which all or substantially all of the CDRs (e.g., CDRs) are corresponding to those of a non-human antibody, and all or substantially all of the FRs are corresponding to those of human antibody. Humanized antibody optionally may comprises at least a portion of an antibody constant region derived from human antibody. "Humanized form" of an antibody, e.g., a non-human antibody, refers to an antibody which has been humanized.

"Framework" or "FR" refers to variable domain residues other than hypervariable region (HRV) residues. The FRs within variable domains generally consist of four FR domains: FR1, FR2, FR3, and FR4. Accordingly, the HRV and FR sequences generally appear in the following order within VH (or VL): FR1-H1(L1)-FR2-H2(L2)-FR3-H3(L3)-FR4.

"Human consensus framework" is a framework which represents the most commonly occurring amino acid residues in a selection of human immunoglobulin VL or VH framework sequences. Generally, human immunoglobulin VL or VH sequences are selected from a subtype of variable domain sequences. Generally, the subtype of sequences is a subtype as described in Kabat et al., Sequences of Proteins of Immunological Interest, Fifth Edition, NIH Publication 91-3242, Bethesda MD (1991), vol. 1-3. In one embodiment, for the VL, the subtype is subtype kappa I as described in Kabat et al., supra. In one embodiment, for the VH, the subtype is subtype III as described in Kabat et al, supra.

"Acceptor human framework" for the purposes herein is a framework comprising the amino acid sequence of a light chain variable domain (VL) framework or a heavy chain variable domain (VH) framework derived from human immunoglobulin framework or human consensus framework, as defined below. An acceptor human framework "derived from" human immunoglobulin framework or human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework sequence is identical to the VL human immunoglobulin framework sequence or human consensus framework sequence.

"Affinity" refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described below.

"Affinity matured" antibody is an antibody with one or more alterations in one or more CDRs thereof which result in an improvement in the affinity of the antibody for antigen, compared to a parent antibody which does not possess those alteration(s).

The terms "host cell," "host cell line," and "host cell culture" are used interchangeably and refer to cells into which exogenous nucleic acid has been introduced, including the progeny of such cells. Host cells include "transformants" and "transformed cells," including primarily transformed cells and progeny derived therefrom, regardless of the number of passages. Progeny may not be completely identical in nucleic acid content to a parent cell, but may contain mutations. Mutant progenies that have the same function or biological activity as screened or selected for the originally transformed cells are included herein.

An "isolated" antibody is an antibody which has been separated from components in its natural environment. In some embodiments, an antibody is purified to greater than 95% or 99% purity as determined by, for example, electrophoretic (e.g., SDS-PAGE, isoelectric focusing (IEF), capillary electrophoresis) or chromatography (e.g., ion exchange or reverse phase HPLC). For review, methods for assessment of antibody purity can be found in, e.g., Flatman et al, J. Chromatogr. B 848:79-87 (2007).

"Isolated nucleic acid encoding anti-PCSK9 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "vector," as used herein, refers to a nucleic acid molecule capable of propagating another nucleic acid to which it is linked. The term includes a vector as a self-replicating nucleic acid structure as well as a vector incorporated into the genome of a host cell into which it has been introduced. Certain vectors are capable of directing the expression of nucleic acids to which they are operatively linked. Such vectors are referred to herein as "expression vectors."

The term "monoclonal antibody" as used herein refers to an antibody obtained from a population of substantially homogeneous antibodies, i.e., the individual antibodies comprising the population are identical and/or bind to the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be produced by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies are described herein.

"Naked antibody" refers to an antibody that is not conjugated to a heterologous moiety (e.g., a cytotoxic moiety) or radiolabel. A naked antibody may be present in a pharmaceutical formulation.

"Native antibody" refers to naturally occurring immunoglobulin molecule with varying structures. For example, native IgG antibody is a heterotetrameric glycoprotein having about 150,000 Daltons, it is composed of two identical light chains associated with two identical heavy chains via disulfide bond. Each heavy chain has variable regions (VH), also called as variable heavy domains or a heavy chain variable domains from N- to C-terminus, followed by three constant domains (CHI, CH2, and CH3). Similarly, each light chain has variable regions (VL), also called as variable light domains or a light chain variable domains from N- to C-terminus, followed by constant light (CL) domains. The light chain of an antibody may be assigned to one of two types, called as kappa (κ) and lambda (λ), based on the amino acid sequence of its constant domain.

"Percent (%) amino acid sequence identity" relative to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved by various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithm needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNLX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction of X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal to the % amino acid sequence identity of B to A. Unless specifically stated otherwise, all % amino acid sequence identity values used herein are obtained as described in the immediately preceding paragraph using the ALIGN-2 computer program.

An "effective amount" of an agent, e.g., a pharmaceutical formulation, refers to an amount effective, at dosages and for periods of time necessary, to achieve the desired therapeutic or prophylactic result.

The term "hypercholesterolemia," as used herein, refers to a condition in which cholesterol levels are elevated above a desired level. In certain embodiments, the LDL-cholesterol level is elevated above the desired level. In certain embodiments, the serum LDL-cholesterol levels are elevated above the desired level.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is human.

The term "pharmaceutical formulation" or "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient other than an active ingredient in a pharmaceutical formulation, which is nontoxic to a subject. A pharmaceutically acceptable carrier includes, but is not limited to, buffer, excipient, stabilizer, or preservative.

The term "PCSK9 activity" or "biological activity" of PCSK9, as used herein, includes any biological effect of PCSK9. In certain embodiments, PCSK9 activity includes the ability of PCSK9 to interact with or bind to a substrate or receptor. In certain embodiments, the biological activity of PCSK9 is the ability of PCSK9 to bind to a LDL-receptor (LDLR). In certain embodiments, PCSK9 activity includes the ability of PCSK9 to decrease or reduce the availability of LDLR. In certain embodiments, the biological activity of PCSK9 includes the ability of PCSK9 to increase the amount of LDL in a subject. In certain embodiments, the biological activity of PCSK9 includes the ability of PCSK9 to decrease the amount of LDLR that is available to bind to LDL in a subject. In certain embodiments, the biological activity of PCSK9 includes the ability of PCSK9 to decrease the amount of LDLR that is available to bind to LDL. In certain embodiments, biological activity of PCSK9 includes any biological activity resulting from PCSK9 signaling.

As used herein, "treatment" refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishing any direct or indirect pathological consequences of the disease, decreasing the rate of disease progression, amelioration or palliation of the disease state, and improving prognosis. In some embodiments, antibodies of the invention are used to delay development of a disease or to slow down the progression of a disease.

### COMPOSITIONS AND METHODS

In one aspect, the present invention is based, in part, on the experimental results obtained by using PCSK9 antibody. Results obtained indicate that blocking biological activity of PCSK9 with anti-PCSK9 antibodies leads to a prevention of reduction in LDLR. In addition, the results demonstrate that administration of anti-PCSK9 antibody reduces total LDL-cholesterol level in a subject. Accordingly, PCSK9 antibodies of the invention, as described herein, provide important therapeutic and diagnostic agents for use in targeting pathological conditions associated with PCSK9, e.g., cholesterol related disorders.

In certain embodiments, "cholesterol related disorder" includes any one or more selected from: hypercholesterolemia, heart disease, metabolic syndrome, diabetes, coronary heart disease, stroke, cardiovascular diseases, Alzheimers disease and general dyslipidemia, characterized in, for example, an elevated total serum cholesterol level, elevated LDL level, elevated triglycerides level, elevated VLDL level, and/or lowered HDL level. Some non-limiting examples of primary and secondary dyslipidemias which can be treated with an anti-PCSK9 antibody, either alone, or in combination with one or more other agents, include the metabolic syndrome, diabetes mellitus, familial combined hyperlipidemia, familial hypertriglyceridemia, familial hypercholesterolemia, including heterozygous hypercholesterolemia, homozygous hypercholesterolemia, familial defective apoplipoprotein B-100; polygenic hypercholesterolemia; remnant removal disease, hepatic lipase deficiency; dyslipidemia secondary to any of the following: dietary indiscretion, hypothyroidism, drugs including estrogen and progestin therapy, beta-blockers, and thiazide diuretics; nephrotic syndrome, chronic renal failure, Cushing's syndrome, primary biliary cirrhosis, glycogen storage diseases, hepatoma, cholestasis, acromegaly, insulinoma, isolated growth hormone deficiency, and alcohol-induced hypertriglyceridemia. Anti-PCSK9 antibodies described herein can also be useful in preventing or treating atherosclerotic diseases, such as, for example, coronary heart disease, coronary artery disease, peripheral arterial disease, stroke (ischemic and hemorrhagic), angina pectoris, or cerebrovascular disease and acute coronary syndrome, myocardial infarction. In certain embodiments, the anti-PCSK9 antibodies described herein are useful in reducing the risk of: nonfatal heart attacks, fatal and non-fatal stroke, certain types of heart surgery, hospitalization for heart failure, chest pain in patients with heart disease, and/or cardiovascular events due to established heart disease such as precedent heart attack, precedent heart surgery, and/or chest pain with evidence of clogged arteries. In certain embodiments, the anti-PCSK9 antibodies and methods described herein can be used to reduce the risk of recurrent cardiovascular events.

### Exemplary Anti-PCSK9 Antibodies

In one aspect, the invention provides an isolated antibody specifically binding to PCSK9. In certain embodiments, the anti-PCSK9 antibody activates the activity of PCSK9.

In some embodiments, the anti-PCSK9 antibody may be humanized. In one embodiment, the anti-PCSK9 antibody comprises CDRs as defined in any of the above embodiments, and further comprises acceptor human frameworks, e.g. human immunoglobulin frameworks or human consensus frameworks.

In another aspect, the anti-PCSK9 antibody comprises a heavy chain variable domain(VH) sequence having at least 90%, 91%, 92%, 93%, 94%, 95%, 96%, 97%, 98%, 99% or 100% sequence identity to the amino acid sequence selected from SEQ ID NOs: 12 and 49-58. In certain embodiments, the VH sequence having at least 90%, 91%, 92%, 93%, 94%, 95%, 96%o, 97%, 98% or 99% identity contains substitutions (e.g., conservative substitutions), insertions, or deletions relative to the reference sequence, but the anti-PCSK9 antibody comprising said sequence retains the ability to bind to PCSK9. In certain embodiments, a total of 1 to 10 amino acids have been substituted, inserted and/or deleted in SEQ ID NO: 12, 49-57 or 58. In certain embodiments, substitutions, insertions, or deletions occur in regions outside the CDRs (i.e., in the FRs). Optionally, the anti-PCSK9 antibody comprises VH sequence as shown in SEQ ID NO: 12, 49-57 or 58, including post-translational modifications of said sequence. In a particular embodiment, VH comprises at least one, two, or three CDRs selected from: (a) HCDR1 comprising amino acid sequence of SEQ ID NO: 14, 20 or 21; (b) HCDR2 comprising amino acid sequence of SEQ ID NO: 15, 22-26 or 27; and (c) HCDR3 comprising amino acid sequence of SEQ ID NO: 16, 28-29 or 30.

In another aspect, an anti-PCSK9 antibody is provided, wherein the antibody comprises a light chain variable domain (VL) having at least 90%, 91%, 92%, 93%, 94%, 95%, 96%, 97%, 98%, 99% or 100% sequence identity to the amino acid sequence of SEQ ID NO: 13, 59-69 or 70. In certain embodiments, VL sequence having at least 90%, 91%, 92%, 93%, 94%, 95%, 96%, 97%, 98% or 99% identity contains substitutions (e.g., conservative substitutions), insertions, or deletions relative to the reference sequence, but an anti-PCSK9 antibody comprising said sequence retains the ability to bind to PCSK9. In certain embodiments, a total of 1 to 10 amino acids have been substituted, inserted and/or deleted in SEQ ID NO: 13, 59-69 or 70. In certain embodiments, the substitutions, insertions, or deletions occur in regions outside the CDRs (i.e., in the FRs). Optionally, the anti-PCSK9 antibody comprises VL sequence as shown in SEQ ID NO: 13, 59-69 or 70, including post-translational modifications of said sequence. In a particular embodiment, VL comprises at least one, two, or three CDRs selected from: (a) LCDR1 comprising amino acid sequence of SEQ ID NO: 17, 31-33 or 34; (b) LCDR2 comprising amino acid sequence of SEQ ID NO: 18, 35-36 or 37; and (c) LCDR3 comprising amino acid sequence of SEQ ID NO: 19, 38-41 or 42.

In another aspect, an anti-PCSK9 antibody is provided, wherein the antibody comprises VH as described in any of the embodiments provided above, and VL as described in any of the embodiments provided above. In one embodiment, the antibody comprises VH and VL sequences as shown in SEQ ID NO: 12 and SEQ ID NO: 13, respectively, including post-translational modifications of those sequences. In one embodiment, the antibody comprises VH and VL sequences as shown in SEQ ID NO: 12 and SEQ ID NO: 59, respectively, including post-translational modifications of those sequences. In one embodiment, the antibody comprises VH and VL sequences as shown in SEQ ID NO: 52 and SEQ ID NO: 13, respectively, including post-translational modifications of those sequences. In one embodiment, the antibody comprises VH and VL sequences as shown in SEQ ID NO: 54 and SEQ ID NO: 13, respectively, including post-translational modifications of those sequences. In one embodiment, the antibody comprises VH and VL sequences as shown in SEQ ID NO: 56 and SEQ ID NO: 65, respectively, including post-translational modifications of those sequences. In one embodiment, the antibody comprises VH and VL sequences as shown in SEQ ID NO: 56 and SEQ ID NO: 13, respectively, including post-translational modifications of those sequences.

In a further aspect of the invention, the anti-PCSK9 antibody according to any of the above embodiments is a monoclonal antibody, including a chimeric, humanized or human antibody. In one embodiment, the anti-PCSK9 antibody is antigen-binding fragment, e.g., Fv, Fab, Fab', scFv, diabody, or F(ab')2 fragment. In another embodiment, the antibody is a full length antibody, e.g., an intact IgG1 antibody or other antibody class or isotype as defined herein.

In a further aspect, the anti-PCSK9 antibody according to any of the above embodiments may have any of the features, alone or in combination, as described in Sections below:

### 1. Antibody affinity

In certain embodiments, the antibody provided herein has dissociation constant (Kd) of ≤ 1µM, ≤ 100 nM, ≤ 10 nM, ≤ 1 nM, ≤0.1 nM, ≤ 0.01 nM, or ≤0.001 nM (e.g. 10E-8M or less, e.g. from 10E-8M to 10E-13M, e.g., from 10E-9M to 10E-13M).

### 2. Antigen-binding fragment

In certain embodiments, the antibody provided herein is an antigen-binding fragment. Antigen-binding fragments include, but are not limited to, Fab, Fab', Fab'-SH, F(ab')2, Fv, and scFv fragments, and other fragments described below. For review of certain antigen-binding fragments, see Hudson et al. Nat. Med. 9: 129-134 (2003). For review of scFv fragments, see, e.g., Pluckthun, The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half- life, see U.S. Patent No. 5,869,046.

Single-domain antibodies are antigen-binding fragments comprising all or a portion of the heavy chain variable domain or all or a portion of the light chain variable domain of an antibody. In certain embodiments, a single-domain antibody is human single-domain antibody (Domantis, Inc., Waltham, MA; see, e.g., U.S. Patent No. 6,248,516 B1).

Antigen-binding fragments can be made by various techniques, including but not limited to proteolytic digestion of an intact antibody as well as production by recombinant host cells (e.g. E. coli or phage), as described herein.

### 3. Chimeric and humanized antibodies

In certain embodiments, the antibody provided herein is a chimeric antibody. Certain chimeric antibodies are described, e.g., in U.S. Patent No. 4,816,567; and Morrison et al, Proc. Natl. Acad. Sci. USA, 81 :6851-6855 (1984)). In one example, a chimeric antibody comprises non-human variable region (e.g., a variable region derived from mouse, rat, hamster, rabbit, or non-human primate, such as a monkey) and human constant region. In a further example, a chimeric antibody is a "class switching" antibody in which the class or subclass has been changed from that of the parent antibody. Chimeric antibodies include antigen-binding fragments thereof.

In certain embodiments, a chimeric antibody is humanized antibody. Typically, non-human antibody is humanized to reduce immunogenicity to humans, while retaining the specificity and affinity of the parental non-human antibody. Generally, humanized antibody comprises one or more variable domains in which HVRs, e.g., CDRs, (or portions thereof) are derived from non-human antibody, and FRs (or portions thereof) are derived from human antibody sequences. Humanized antibody optionally will also comprise at least a portion of human constant region. In some embodiments, some FR residues in humanized antibody are substituted with corresponding residues from a non-human antibody (e.g., an antibody from which the CDR residues are derived), e.g., to restore or improve antibody specificity or affinity.

Humanized antibodies and methods of making the same are reviewed, e.g., in Almagro and Fransson, Front. Biosci. 13: 1619-1633 (2008), and are further described, e.g., in Riechmann et al, Nature 332:323-329 (1988); Queen et al, Proc. Nat '1 Acad. Sci. USA 86: 10029-10033 (1989); US Patent Nos. 5, 821,337, 7,527,791, 6,982,321, and 7,087,409; Kashmiri et al, Methods 36:25-34 (2005) (describing SDR (a-CDR) grafting); Padlan, Mol. Immunol. 28:489-498 (1991) (describing "resurfacing"); DaU'Acqua et al, Methods 36:43-60 (2005) (describing "FR shuffling"); and Osbourn et al, Methods 36:61-68 (2005) and Klimka et al, Br. J. Cancer, 83: 252-260 (2000) (describing the "guided selection" approach for FR shuffling).

Human framework regions that may be used for humanization include but are not limited to: framework regions selected with the "best-fit" method (see, e.g., Sims et al. J. Immunol. 151 :2296 (1993)); framework regions derived from the consensus sequence of human antibodies of a particular subtype of light or heavy chain variable regions (see, e.g., Carter et al. Proc. Natl. Acad. Sci. USA, 89:4285 (1992); and Presta et al. J. Immunol, 151 :2623 (1993)); human mature (somatically mutated) framework regions or human germline framework regions (see, e.g., Almagro and Fransson, Front. Biosci. 13: 1619-1633 (2008)); and framework regions derived from screening FR libraries (see, e.g., Baca et al, J. Biol. Chem. 272: 10678-10684 (1997) and Rosok et al, J. Biol. Chem. 271: 22611-22618 (1996)).

### 4. Library-derived antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. Methods in Molecular Biology 178: 1-37 (O'Brien et al, ed., Human Press, Totowa, NJ, 2001) and further described, e.g., in McCafferty et al, Nature 348:552- 554; Clackson et al, Nature 352: 624-628 (1991); Marks et al, J. Mol. Biol. 222: 581-597 (1992); Marks and Bradbury, Methods in Molecular Biology 248: 161-175 (Lo, ed., Human Press, Totowa, NJ, 2003); Sidhu et al, J. Mol. Biol. 338(2): 299-310 (2004); Lee et al, J. Mol. Biol. 340(5): 1073-1093 (2004); Fellouse, Proc. Natl. Acad. Sci. USA 101(34): 12467-12472 (2004); and Lee et al, J. Immunol. Methods 284(1-2): 119-132 (2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antigen-binding fragments, either as single-chain Fv (scFv) fragments or as Fab fragments. Libraries from immunized sources provide high-affinity antibodies against the immunogen without the requirement of constructing hybridomas. Alternatively, the naive repertoire can be cloned (e.g., from human) to provide a single source of antibodies against a wide range of non-self and also self-antigens without any immunization as described by Griffiths et al, EMBO J 12: 725-734 (1993). Finally, naive libraries can also be made synthetically by cloning un-rearranged V-gene segments from stem cells, and using PCR primers containing random sequence to encode the highly variable CDR3 regions and to accomplish rearrangement in vitro, as described by Hoogenboom and Winter, J. Mol. Biol, 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360. Antibodies or antibody fragments isolated from human antibody libraries are considered as human antibodies or human antibody fragments herein.

### 5. Antibody variants

In certain embodiments, amino acid sequence variants of the antibodies provided herein are contemplated. For example, it may be desirable to improve the binding affinity, prolong serum half-life and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### Substitution, Insertion, and Deletion Variants

In certain embodiments, antibody variants having one or more amino acid substitutions are provided. Sites of interest for substitution mutagenesis include the CDRs and FRs. Conservative substitutions are shown in Table 1 under the heading of "conservative substitutions." More substantial changes are provided in Table 1 under the heading of "exemplary substitutions" and as further described below in reference to amino acid side chain classes. Amino acid substitutions may be introduced into an antibody of interest and the products are screened for a desired activity, e.g., retained/improved antigen binding, decreased immunogenicity, or prolonged half-life products.

**TABLE 1**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Glv (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Try |
| Pro (p) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) Hydrophobic: Norleucine, Met, Ala, Val, Leu, Ile;
(2) Neutral hydrophilic: Cys, Ser, Thr, Asn, Gln;
(3) Acidic: Asp, Glu;
(4) Basic: His, Lys, Arg;
(5) Residues that influence chain orientation: Gly, Pro;
(6) Aromatic: Trp, Tyr, Phe;

Non-conservative substitutions will entail exchanging a member of one of these classes for another class.

One type of substitution variant involves substituting one or more hypervariable region residues of a parent antibody (e.g. humanized or human antibody). Generally, the resulting variant(s) selected for further study will have modifications (e.g., improvements) in certain biological properties (e.g., increased affinity, prolonged half-life or reduced immunogenicity) relative to the parent antibody and/or will have substantially retained certain biological properties of the parent antibody. An exemplary substitution variant is an affinity matured antibody, which may be conveniently generated, e.g., using phage display-based affinity maturation techniques such as those described herein. Briefly, one or more CDR residues are mutated and the variant antibodies will be displayed on phage and screened for a particular biological activity (e.g. binding affinity).

Alterations (e.g., substitutions) may be made in CDRs, e.g., to improve antibody affinity. Such alterations may be made in CDR "hotspots," i.e., residues encoded by codons that undergo mutations at high frequency during the somatic maturation process (see, e.g., Chowdhury, Methods Mol. Biol. 207: 179-196 (2008)), and/or SDRs (a-CDRs), with the resulting variant VH or VL being tested for binding affinity. Affinity maturation obtained by constructing and reselecting from secondary libraries has been described, e.g., in Hoogenboom et al. Methods in Molecular Biology 178: 1-37 (O'Brien et al, ed., Human Press, Totowa, NJ, (2001)). In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variant with the desired affinity. Another method to introduce diversity involves CDR-directed approaches, in which several CDR residues (e.g., 4-6 residues at a time) are randomized. CDR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more CDRs so long as such alterations do not substantially reduce the ability of the antibody to bind to an antigen. For example, conservative alterations (e.g., conservative substitutions as provided herein) that do not substantially reduce binding affinity may be made in CDRs. Such alterations may be outside of CDR "hotspots" or SDRs. In certain embodiments of the variant VH and VL sequences provided above, each CDR either is unaltered, or contains no more than one, two or three amino acid substitutions.

### Fc region variants

In certain embodiments, one or more amino acid modifications may be introduced into the Fc region of an antibody provided herein, thereby generating an Fc region variant and promoting the binding of the Fc region to human FcRn, and the half-life of the antibody in human serum is prolonged. The Fc region variant may comprise human Fc region sequence {e.g., human IgG1, IgG2, IgG3 or IgG4 Fc region) comprising amino acid modifications {e.g. substitutions) at one or more amino acid positions.

### Assays

Anti-PCSK9 antibodies provided herein may be identified, screened for, or characterized for their physical/chemical properties and/or biological activities by various assays known in the art.

### 1. Binding assays and other assays

In one aspect, anti-PCSK9 antibodies of the invention are tested for their antigen binding activities, e.g., by known methods such as ELISA, Western Blot, etc.

### 2. Activity assays

In one aspect, assays are provided for identifying anti-PCSK9 antibodies having biological activities. For example, the biological activities of PCSK9 antibodies may include the ability to block, antagonize, inhibit, interfere with, modulate and/or reduce one or more of the biological activities of PCSK9. Antibodies having such biological activity in vivo and/or in vitro are also provided.

In certain embodiments, anti-PCSK9 antibodies bind to human PCSK9 and prevent interaction with the LDLR. In certain embodiments, the invention provides isolated anti-PCSK9 antibodies which specifically bind to PCSK9 and which antagonize the effect on LDLR levels mediated by PCSK9 when measuring the LDLR down regulation in vitro in HepG2 cells disclosed herein.

Exemplary diseases that may be diagnosed with an antibody of the invention include cholesterol related diseases (which include "serum cholesterol related diseases"), including any one or more selected from: hypercholesterolemia, heart disease, metabolic syndrome, diabetes, coronary heart disease, stroke, cardiovascular diseases, Alzheimers disease and general dyslipidemia, characterized in, for example, an elevated total serum cholesterol level, elevated LDL level, elevated triglycerides level, elevated very low density lipoprotein (VLDL) level, and/or lowered HDL level. In one aspect, the invention provides a method for treating or preventing hypercholesterolemia, and/or at least one symptom selected from dyslipidemia, atherosclerosis, cardiovascular disease (CVD) or coronary heart disease in an individual, comprising administering to the individual an effective amount of anti-PCSK9 antibody. In certain embodiments, the invention provides an effective amount of an anti-PCSK9 antibody for use in treating or preventing hypercholesterolemia, and/or at least one symptom selected from dyslipidemia, atherosclerosis, CVD or coronary heart disease in a subject. The invention further provides the use of an effective amount of an anti-PCSK9 antibody that antagonizes extracellular or circulating PCSK9 in manufacture of a medicament for treating or preventing hypercholesterolemia, and/or at least one symptom selected from dyslipidemia, atherosclerosis, CVD or coronary heart disease in an individual.

### EXAMPLES

The examples of method and composition in the present invention are as follows. It will be understood that many other examples can be performed according to general descriptions indicated above. In the examples of the present invention, where specific conditions are not described, the experiments are generally conducted under conventional conditions as described in for example, Antibody Technology Laboratory Manual and Mecular Cloning Manual of Cold Spring Harbor, or under conditions proposed by the material or product manufacturers. Where the source of the reagents is not specifically given, the reagents are commercially available conventional reagents.

### EXAMPLE 1. Preparation of PCSK9 Antigen and Test Protein

UniProt Proprotein convertase subtilisin/kexin type 9 (human PCSK9, Uniprot: Q8MBP7) was used as the template of PCSK9 in the present invention. Optionally, different labels such as his-tag or peptide promoting immunization such as PADRE peptide were fused to PCSK9 protein, then the fusion protein was cloned into pTT5 vector (Biovector, Cat#: 102762) or pTargeT vector (promega, A1410), and transiently expressed in 293 cells or stably expressed in CHO-S. Purification steps were performed with conventional methods and the antigen and test protein of the present invention were obtained. The particular sequences were shown in SEQ ID NOs:1-9. The obtained proteins or mutant proteins thereof (such as PCSK9 D374Y mutation, PCSK9-Y) were used as antigens for preparing anti-human PCSK9 monoclonal antibody and for selecting library.
PCSK9 with his-tag: PCSK9-His6, used as an immunogen for immunizing mice or used as detection reagent. 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
 
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVLV
TAAGNFRDDACLYSPASAPEVITVGATNAQDQPVTLGTLGTNFGRCVDLFAP
GEDIIGASSDCSTCFVSQSGTSQAAAHVAGIAAMMLSAEPELTLAELRQRLIHF
SAKDVINEAWFPEDQRVLTPNLVAALPPSTHGAGWQLFCRTVWSAHSGPTR
MATAVARCAPDEELLSCSSFSRSGKRRGERMEAQGGKLVCRAHNAFGGEGV
YAIARCCLLPQANCSVHTAPPAEASMGTRVHCHQQGHVLTGCSSHWEVEDL
GTHKPPVLRPRGQPNQCVGHREASIHASCCHAPGLECKVKEHGIPAPQEQVT
VACEEGWTLTGCSALPGTSHVLGAYAVDNTCVVRSRDVSTTGSTSEGAVTA
VAICCRSRHLAQASQELQHHHHHH
SEQ ID NO: 1
 NOTE: Underlined sequence is a signal peptide, and italic part is His6-tag sequence.
PCSK9 with PADRE peptide and his-tag: PCSK9-PADRE-His6, used as an immunogen, wherein the contained PADRE peptide can promote immunization; 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVLV
TAAGNFRDDACLYSPASAPEVITVGATNAQDQPVTLGTLGTNFGRCVDLFAP
GEDIIGASSDCSTCFVSQSGTSQAAAHVAGIAAMMLSAEPELTLAELRQRLIHF
SAKDVINEAWFPEDQRVLTPNLVAALPPSTHGAGWQLFCRTVWSAHSGPTR
MATAVARCAPDEELLSCSSFSRSGKRRGERMEAQGGKLVCRAHNAFGGEGV
YAIARCCLLPQANCSVHTAPPAEASMGTRVHCHQQGHVLTGCSSHWEVEDL
GTHKPPVLRPRGQPNQCVGHREASIHASCCHAPGLECKVKEHGIPAPQEQVT
VACEEGWTLTGCSALPGTSHVLGAYAVDNTCVVRSRDVSTTGSTSEGAVTA
VAICCRSRHLAQASQELQGSGAKFVAAWTLKAAAHHHHHH
SEQ ID NO: 2
 NOTE: Underlined sequence is a signal peptide, double underlined sequence is a linker, the dashed line sequence is PADRE peptide, and italic part is the His6-tag.
Fusion protein of PCSK9 containing TEV cleavage site and his tag: PCSK9-TEV-His6. N-PCSK9 (N terminal PCSK9 domain) will be obtained as an immunogen after TEV enzyme digestion; 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVLV
TAAGNFRDDACLYSPASAPEVITVGATNAQDQPVTLGTLGTNFGRCVDLFAP
GEDIIGASSDCSTCFVSQSGTSQAAAHVAGIAAMMLSAEPELTLAELRQRLIHF
SAKDVINEAWFPEDQRVLTPNLVAALPPSTHENLYFQGAGWQLFCRTVWSA
HSGPTRMATAVARCAPDEELLSCSSFSRSGKRRGERMEAQGGKLVCRAHNA
FGGEGVYAIARCCLLPQANCSVHTAPPAEASMGTRVHCHQQGHVLTGCSSH
WEVEDLGTHKPPVLRPRGQPNQCVGHREASIHASCCHAPGLECKVKEHGIPA
PQEQVTVACEEGWTLTGCSALPGTSHVLGAYAVDNTCVVRSRDVSTTGSTSE
GAVTAVAICCRSRHLAQASQELQHHHHHH
SEQ ID NO: 3
 NOTE: Underlined sequence is a signal peptide, double underlined sequence is TEV cleavage site, and italic part is the His6-tag.
PCSK9-D374Y mutant protein with his-tag: PCSK9-D374Y-His6, as a detection reagent; 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVLV
TAAGNFRDDACLYSPASAPEVITVGATNAQDQPVTLGTLGTNFGRCVDLFAP
GEDIIGASSYCSTCFVSQSGTSQAAAHVAGIAAMMLSAEPELTLAELRQRLIHF
SAKDVINEAWFPEDQRVLTPNLVAALPPSTHGAGWQLFCRTVWSAHSGPTR
MATAVARCAPDEELLSCSSFSRSGKRRGERMEAQGGKLVCRAHNAFGGEGV
YAIARCCLLPQANCSVHTAPPAEASMGTRVHCHQQGHVLTGCSSHWEVEDL
GTHKPPVLRPRGQPNQCVGHREASIHASCCHAPGLECKVKEHGIPAPQEQVT
 
VACEEGWTLTGCSALPGTSHVLGAYAVDNTCVVRSRDVSTTGSTSEGAVTA
VAICCRSRHLAQASQELQHHHHHH
SEQ ID NO: 4
 NOTE: Underlined sequence is a signal peptide, and italic part is the His6-tag.
PCSK9 protein inserted with biotin receiving peptide BP15 and his tag: PCSK9-BP15-His6. As a detection reagent, biotin will be labeled to BP15 peptide position during the expression, avoiding the biotin labeling in vitro and consequently avoiding possible conformational changes. 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVLV
TAAGNFRDDACLYSPASAPEVITVGATNAQDQPVTLGTLGTNFGRCVDLFAP
GEDIIGASSDCSTCFVSQSGTSQAAAHVAGIAAMMLSAEPELTLAELRQRLIHF
SAKDVINEAWFPEDQRVLTPNLVAALPPSTHGAGWQLFCRTVWSAHSGPTR
MATAVARCAPDEELLSCSSFSRSGKRRGERMEAQGGKLVCRAHNAFGGEGV
YAIARCCLLPQANCSVHTAPPAEASMGTRVHCHQQGHVLTGCSSHWEVEDL
GTHKPPVLRPRGQPNQCVGHREASIHASCCHAPGLECKVKEHGIPAPQEQVT
VACEEGWTLTGCSALPGTSHVLGAYAVDNTCVVRSRDVSTTGSTSEGAVTA
VAICCRSRHLAQASQELQGSTSGSGLNDIFEAQKIEWHEHHHHHH
SEQ ID NO: 5
 NOTE: Underlined sequence is a signal peptide, double underlined sequence is the biotin receiving peptide, and italic part is the His6-tag.
PCSK9 D374Y mutant protein inserted with biotin receiving peptide BP15 and his tag: PCSK9-D374Y-BP15-His6, as a detection protein: 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
 
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVLV
TAAGNFRDDACLYSPASAPEVITVGATNAQDQPVTLGTLGTNFGRCVDLFAP
GEDIIGASSYCSTCFVSQSGTSQAAAHVAGIAAMMLSAEPELTLAELRQRLIHF
SAKDVINEAWFPEDQRVLTPNLVAALPPSTHGAGWQLFCRTVWSAHSGPTR
MATAVARCAPDEELLSCSSFSRSGKRRGERMEAQGGKLVCRAHNAFGGEGV
YAIARCCLLPQANCSVHTAPPAEASMGTRVHCHQQGHVLTGCSSHWEVEDL
GTHKPPVLRPRGQPNQCVGHREASIHASCCHAPGLECKVKEHGIPAPQEQVT
VACEEGWTLTGCSALPGTSHVLGAYAVDNTCVVRSRDVSTTGSTSEGAVTA
VAICCRSRHLAQASQELQGSTSGSGLNDIFEAQKIEWHEHHHHHH
SEQ ID NO: 6
 NOTE: Underlined sequence is a signal peptide, double underlined sequence is the biotin receiving peptide, and italic part is the His6-tag.
PCSK9 receptor protein LDLR extracellular domain with Flag tag and His tag: LDLR-ECD-Flag-His6 as a detection reagent; 
MGPWGWKLRWTVALLLAAAGTAVGDRCERNEFQCQDGKCISYKWVCDGS
AECQDGSDESQETCLSVTCKSGDFSCGGRVNRCIPQFWRCDGQVDCDNGSDE
QGCPPKTCSQDEFRCHDGKCISRQFVCDSDRDCLDGSDEASCPVLTCGPASFQ
CNSSTCIPQLWACDNDPDCEDGSDEWPQRCRGLYVFQGDSSPCSAFEFHCLS
GECIHSSWRCDGGPDCKDKSDEENCAVATCRPDEFQCSDGNCIHGSRQCDRE
YDCKDMSDEVGCVNVTLCEGPNKFKCHSGECITLDKVCNMARDCRDWSDEP
IKECGTNECLDNNGGCSHVCNDLKIGYECLCPDGFQLVAQRRCEDIDECQDP
DTCSQLCVNLEGGYKCQCEEGFQLDPHTKACKAVGSIAYLFFTNRHEVRKMT
LDRSEYTSLIPNLRNVVALDTEVASNRIYWSDLSQRMICSTQLDRAHGVSSYD
TVISRDIQAPDGLAVDWIHSNIYWTDSVLGTVSVADTKGVKRKTLFRENGSK
PRAIVVDPVHGFMYWTDWGTPAKIKKGGLNGVDIYSLVTENIQWPNGITLDL
LSGRLYWVDSKLHSISSIDVNGGNRKTILEDEKRLAHPFSLAVFEDKVFWTDII
NEAIFSANRLTGSDVNLLAENLLSPEDMVLFHNLTQPRGVNWCERTTLSNGG
CQYLCLPAPQINPHSPKFTCACPDGMLLARDMRSCLTEAEAAVATQETSTVR
LKVSSTAVRTQHTTTRPVPDTSRLPGATPGLTTVEIVTMSHQALGDVAGRGN
EKKPSSVRDYKDDDDKHHHHHH
SEQ ID NO: 7
 NOTE: Underlined sequence is a signal peptide, double underlined sequence is the Flag tag, and italic part is the His6-tag.
Fusion protein of truncated LDLR extracellular domain and hIgG1 Fc (with PCSK9 binding activity): LDLR-sECD -Fc (hIgG1) as a detection reagent; 
MEFGLSWLFLVAILKGVQCGTNECLDNNGGCSHVCNDLKIGYECLCPDGFQL
VAQRRCEDIDECQDPDTCSQLCVNLEGGYKCQCEEGFQLDPHTKACKEPKSS
DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQKSL
SLSPGK
SEQ ID NO: 8
 NOTE: Underlined sequence is a signal peptide, double underlined sequence is the truncated LDLR extracellular domain with PCSK9 binding activity (LDLR-sECD), and italic part is the hIgG1-Fc.
Fusion protein of further truncated LDLR extracellular domain and hIgG1 Fc (with PCSK9 binding activity): LDLR-ssECD -Fc (hIgG1) as a detection reagent; 
MEFGLSWLFLVAILKGVQCGTNECLDNNGGCSHVCNDLKIGYECLCPDGFQL
VAQRRCEDIDEPKSSDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSV
MHEALHNHYTQKSLSLSPGK
SEQ ID NO: 9
 NOTE: Underlined sequence is a signal peptide, double underlined sequence is the further truncated LDLR extracellular domain with PCSK9 binding activity (LDLR-ssECD), and italic part is the hIgG1-Fc.

### EXAMPLE 2. Preparation of Anti-human PCSK9 Monoclonal Antibody

The anti-human PCSK9 monoclonal antibody was produced by immunizing mice. Experimental SJL white mice, female, 6 weeks old (Beijing Weitong Lihua Experimental Animal Technology Co., Ltd., animal production license number: SCXK (Beijing) 2012-0001).

Feeding environment: SPF level. After the mice were purchased, the animals were kept in the laboratory for 1 week, 12/12 hours light/dark cycle, temperature 20-25 °C, humidity 40-60%. The mice that had been adapted to the environment were immunized according to the following two schemes (A/B), each group of 6-10 mice. Immunized antigen was human PCSK9 with His tag: PCSK9-His6 (SEQ ID NO: 1), PCSK9-PADRE-His6 (SEQ ID NO: 2) and N-PCSK9 (SEQ ID NO: 3).

Scheme A: emulsifying with Freund's adjuvant (sigma Lot Num: F5881/F5506): first immunization with Complete Freund's adjuvant (CFA), booster immunization with Incomplete Freund's adjuvant (IFA). The ratio of antigen and adjuvant was 1:1, 100µg/mouse (first immunization), 50µg/mouse (booster immunization). On day 0, mice were intraperitoneally (IP) injected with 100µg/mouse of emulsified antigens, after first immunization, once every two weeks, total 6-8 weeks.

Scheme B: Cross immunization with Titermax (sigma Lot Num: T2684) and Alum (Thremo Lot Num: 77161). The ratio of antigen and adjuvant (titermax) was 1:1, and the ratio of antigen and adjuvant (Alum) was 3:1, 10-20µg/mouse (first immunization), 5µg/mouse (booster immunization). On day 0, mice were intraperitoneally (IP) injected with 20/10 µg/mouse emulsified antigen, once a week after first immunization, Titermax and Alum were alternately used, total 6-11weeks. Four weeks after immunization, back injection or intraperitoneal injection with antigen was selected according to the swelling conditions on back and abdomen.

### EXAMPLE 3. Library Construction

Spleens of the immunized mice were sieved via filter, washed with PBS. Then the RNA was extracted and cDNA was obtained by reverse transcription. The antibody residues were numbered according to Kabat (Kabat et al., Sequences of proteins of immunological interest, 5th Ed., Public Health Service, National Institutes of Health, Bethesda, MD (1991)). The heavy and light chains were amplified separately using upstream primer mixture and downstream primer mixture. Primers were designed based on Brocks et al. 2001. SfiI restriction site and protective bases were incorporated into the heavy chain upstream primer, and partial sequence of the linker was incorporated into the downstream primer; partial sequence of the linker, which was complementary to the heavy chain downstream primer, was incorporated into the light chain upstream primer, another sfiI restriction site and protected bases were incorporated into the downstream primer. The amplified VH and VL fragments were recovered via gel recovery, and spliced into scFv (comprising VH-(G4S) 3-VL) by over-lap PCR. The phagemid vector and scFv were both digested with sfiI, and then connected with each other. E. coli strain SS320 was electro-transformed and the capacity of the library was approximately 1E9.

### EXAMPLE 4. Screening

Panning was performed by liquid-phase method after the E. coli library was packaged into phage particles with helper phage (NEB, N0315S): Phages were bound to biotinylated PCSK9 in liquid phase and were separated by streptavidin beads. After two rounds of panning, monoclonal antibodies were picked from the phage for phage ELISA assay. The assay was divided into two parts: binding activity and blocking activity. Binding activity: ELISA plate was coated with 2 ng/µl streptavidin, and incubated with the 1 ng/µl biotinylated PCSK9 (SEQ ID NO: 5), then phage supernatant diluted with 1: 1 blocking buffer (1×PBS÷2% skim milk) was added and finally detected with anti-M13 HRP (GE, 27-9421-01); blocking activity: similar to binding activity, except that 50ng/µl final concentration of LDLR-Fc (SEQ ID NO: 8) was added during the incubation of phage supernatant. The ELISA OD45 value obtained from the binding activity test was divided by the ELISA OD45 value obtained from the blocking activity test, and then clones with resulting value greater than 2.0 were screened, including murine clone mAb-011, for sequencing, and further screening.

### EXAMPLE 5. Expression and Identification of Chimeric Antibodies

The selected clones (including mAb-011) were constructed into an IgG1/κ chimeric antibody expression vector and transiently expressed in mammalian cells. After Protein A affinity purification, the binding activity of PCSK9 (WT PCSK9, SEQ ID NO: 5) and PCSK9-Y (mutant PCSK9, SEQ ID NO: 6) were tested (test examples 1 and 2), then the EC50 values were calculated; and blocking activity (test examples 3 and 4) of wild-type PCSK9 and PCSK9-Y were also tested, and their IC50 values were calculated. The binding activity was tested by using streptavidin-coated plates and incubating biotinylated PCSK9 (or PCSK9-Y) and then incubating serially diluted chimeric antibodies. The blocking activity was tested by using LDLR-Fc-coated plates, blocking the plates, and incubating serially diluted chimeric antibodies and biotinylated PCSK9 (or biotinylated PCSK9-Y) at the same time, and then incubating streptavidin HRP for detection. The chimeric antibody ch-011 with better activity (see Table 2) was screened as a key molecule for subsequent humanization.

**Table 2 Binding and blocking activity of the chimeric antibody:**

| | Ch-0 11 | |
|---|---|---|
| Receptor | Binding activity EC50(ug/ml) | Blocking activity IC50(ug/ml) |
| PCSK9 WT | 0.005 | 0.263 |
| PCSK9Y mutant | 0.050 | 3.113 |

The results of the binding test, in which the PCSK9 chimeric antibody ch-011 screened in the present invention was tested for binding activity to the PCSK9/PCSK9-Y protein, showed that: ch-011 antibody has an effective binding activity to PCSK9/PCSK9-Y, and has a higher binding activity to PCSK9.

The results of the blocking test, in which the PCSK9 chimeric antibody ch-011 screened in the present invention was tested for activity of blocking the binding of LDLR with PCSK9/PCSK9-Y, showed that: ch-011 antibody has an effective activity of blocking the binding of LDLR with PCSK9/PCSK9-Y, and has a higher blocking effect on the binding of LDLR with PCSK9.

### EXAMPLE6. Humanization and Identification of mAb-011

We chose the murine-derived clone mAb-011 for humanization based on the results of the chimeric antibody experiment. Humanization strategy was the CDR-graft strategy. After aligning with the human germline gene database of heavy light chain variable regions, the germline gene with the highest homology to murine mAb-011 sequence was selected as a template. The humanized light chain templates for the murine antibody mAb-011 are IGKV1-39*01 and hjk4.1, and the humanized heavy chain templates are IGHV1-2*02 and hjh6.1, and the humanized variable region sequences are as follows:
> h011-1 VH (CDR graft) 
QVQLVQSGAEVKKPGASVKVSCKASGYTFTGYTIHWVRQAPGQGLEW
MGYINPSSTYTKFNQKFKDRVTMTRDTSISTAYMELSRLRSDDTAVYYCARE
RIYSNYWFFDVWGQGTTVTVSS
 SEQ ID NO: 10
> h011-1 VL (CDR graft)

DIQMTQSPSSLSASVGDRVTITCKASQNVYTAVAWYQQKPGKAPKLLIY
SASNRYTGVPSRFSGSGSGTDFTLTISSLQPEDFATYYCQQYSSYPYTFGGGTK
VEIK
SEQ ID NO: 11

Different back mutations were selected for combination (Table 3) after CDR grafting. The designed humanized sequences were fully-gene synthesized and heavy and light chains were combined with each other for mammalian expression (Table 4). After purification of the protein, the blocking activity test was performed similarly to that for chimeric antibodies (see test 3 and 4 for the method and the results are shown in Table 5). Some cloned proteins were selected and were determined for dissociation constants by Surface Plasmon resonance (SPR) (Biacore X100, GE, see test 5). The sample to be tested was captured by the Amine-Coupling Anti-Fc pAb (GE) on the CM5 chip (GE). PCSK9(SEQ ID NO: 1) or PCSK9-Y(SEQ ID NO: 4) was the mobile phase, working buffer was IX HBS-EP⁺, pH 7.4, regeneration bu0ffer was 3 M MgCl₂. The results were shown in Table 6.

**Table 3 Template selection and back mutation design for mAb-011**

| mAb-011_VH | | mAb-011_VL | |
|---|---|---|---|
| h011_VH.1 | Graft | h011_VL.1 | Graft |
| h011_VH.1A | R72A,T74K | h011_VL.1A | Q3V |
| h011_VH.1B | R72A,T74K,M48V,V68A | h011_VL.1B | Q3V,A43S,Y87F |
| h011_VH.1C | R72A,T74K,V68A,M70L | | |
| h011_VH.1D | R72A,T74K,V68A,M70L,M48V | | |
| h011_VH.1E | R72A,T74K,V68A,M70L,M48V,G49A | | |
| h011_VH.1F | R72A,T74K,V68A,M70L,M48V,G49A,R67K,R38K | | |

**Table 4 Combination of heavy and light chains**

| | h011_VL.1 | h011_VL.1A | h011_VL.1B |
|---|---|---|---|
| h011_VH.1 | h011-1 | h011-2 | h011-3 |
| h011_VH.1A | h011-4 | h011-5 | h0117-6 |
| h011_VH.1B | h011-7 | h011-8 | h011-9 |
| h011_VH.1C | h011-10 | h011-11 | h011-12 |
| h011_VH.1D | h011-13 | h011-14 | h011-15 |
| h011_VH.1E | h011-16 | h011-17 | h011-18 |
| h011_VH.1F | h011-19 | h011-20 | h011-21 |

Note: The table indicated the sequence obtained by combining various mutations. As indicated by h011-5, there were two mutations (heavy chain h011_VH.1A and light chain h011_VL.1A) on the humanized antibody h011-5. And so on.

ELISA test was performed to detect the binding to PCSK9 or PCSK9-Y (see Test 1 and 2). The positive cells detected in above ELISA test were further used in ELISA test to detect blocking of the binding of PCSK9/ PCSK9-Y to LDLR (see test 3 and 4). The light and heavy chain combination was finally determined as h011-VH.1 and h011-VL.1B. The heavy and light chain variable region sequences of the humanized h011-3 were as follows:
>h011-3 VH 
QVQLVQSGAEVKKPGASVKVSCKASGYTFTGYTIHWVRQAPGQGLEWMGYI
NPSSTYTKFNQKFKDRVTMTRDTSISTAYMELSRLRSDDTAVYYCARERIYSN
YWFFDVWGQGTTVTVSS
SEQ ID NO: 12 >h011-3 VL

DIVMTQSPSSLSASVGDRVTITCKASQNVYTAVAWYQQKPGKSPKLLIYSASN
RYTGVPSRFSGSGSGTDFTLTISSLQPEDFATYFCQQYSSYPYTFGGGTKVEIK
SEQ ID NO: 13

**Table 5 Blocking activities of chimeric and humanized antibodies on the binding of LDLR-FC to PCSK9 or PCSK9-Y**

| Clone No. | IC50 | |
|---|---|---|
| | PCSK9 (ug/ml) | PCSK9-Y(ug/ml) |
| ch-011 | 0.2028 | 3.552 |
| h011-3 | 0.2055 | 5.127 |

The results of the binding test of PCSK9 chimeric or humanized antibodies screened by the present invention to wild type/mutant PCSK9 protein showed that: h011-3 and ch-011 antibodies have high binding activity to wild type/mutant PCSK9, and the binding activity of h011-3 and ch-011 antibodies to wild type PCSK9 is higher.

**Table 6 Dissociation constants of some samples after humanization**

| Analytical substrate | sample | ka (1/Ms) | kd (1/s) | KD (M) |
|---|---|---|---|---|
| PCSK9 | ch-011 | 1.12E+05 | 4.32E-05 | 3.85E-10 |
| | h011-3 | 7.13E+04 | 1.88E-05 | 2.63E-10 |
| PCSK9-Y | ch-011 | 2.43E+05 | 3.27E-02 | 1.35E-07 |
| | h011-3 | 1.68E+05 | 5.78E-03 | 3.45E-08 |

| | | | | |
|---|---|---|---|---|
| Note: ch011: mAb-011chimeric antibody | | | | |

Biacore test results of the PCSK9 chimeric or humanized antibodies screened by the present invention to wild type/mutant PCSK9 showed that: h011-3 and ch-011 have lower equilibrium dissociation constants, and high affinity. h011-3 and ch-011 have higher affinity to wild-type PCSK9.

### EXAMPLE 7. Affinity Maturation of h011-3

We decided to carry out the affinity maturation against PCSK9-Y with h011-3, as the affinity of murine mAb-011 and its humanized antibody h011-3 to PCSK9-Y is low. M13 phage display technology was used in the affinity maturation. Codon-based primers (during the synthesis of primers, single codon consists of wild-type codon and NNK) were adopted to introduce mutations in each CDR, and a separate phage display library was constructed for each CDR. Based on the length of the CDRs, the ratio of NNK and the library size required for the library were adjusted (Table 7).

**Table 7 Library size and NNK incorporation ratio**

| Lib | CDR length | NNK ratio | Lib size |
|---|---|---|---|
| H1 | 5 | 50% | >2E7 |
| H2 | 17 | 20% | >1E8 |
| H3 | 12 | 30% | >1E8 |
| L1 | 11 | 30% | >1E8 |
| L2 | 7 | 50% | >2E7 |
| L3 | 9 | 40% | >1E8 |

The constructed 6 libraries were packaged into phages for panning: associated with biotinylated PCSK9-Y (SEQ ID NO: 6) in liquid phase, captured by streptavidin, elutriated, eluted, then re-infected with E.coli for the next round of panning. The concentration of biotinylated PCSK9-Y was reduced by 10-fold in each round of panning. After 3-4 rounds of panning, a single clone was picked from each library for sequencing verification. According to the enrichment degree of amino acid residues in CDR regions, some clones were selected to construct full-length Ig for expression in mammalian cells; meanwhile the mutations with different enrichment degree of CDRs were combined artificially and constructed as a full-length Ig for mammalian cell expression (as shown in Table 8-11).

After purification, the cloned protein was used in ELISA to detect the binding to PCSK9 (test 1) and to PCSK9-Y (test 2); then the positive cells detected in the above ELISA were used in ELISA test to detect blocking of PCSK9-Y/LDLR binding (test 3), and to detect blocking of PCSK9/LDLR binding (test 4). The results are shown in Tables 12-15.

The results indicated that the PCSK9 antibodies obtained by the present invention have high binding activity to PCSK9 and PCSK9-Y, and can effectively block the binding of PCSK9/PCSK9-Y to LDLR.

**Table 8 CDR sequences of enriched clones and artificial combinatorial clones**

| Clone No. | HCDR1 | HCDR2 | HCDR3 | LCDR1 | LCDR2 | LCDR3 |
|---|---|---|---|---|---|---|
| h011-3 | GYTIH | YINPSSTYTKFNQKFKD | ARERIYSNYWFFDV | KASQNVYTAVA | SASNRYT | QQYSSYPYT |
| SEQ ID N0 | 14 | 15 | 16 | 17 | 18 | 19 |
| h011-3050 | ----- | e-1-------------- | -------------- | ----------- | ------- | --------- |
| SEQ ID NO | 14 | 22 | 16 | 17 | 18 | 19 |
| h011-3058 | ----- | c---- g-------------- | -------------- | ----------- | ------- | --------- |
| SEQ ID N0 | 14 | 23 | 16 | 17 | 18 | 19 |
| h011-3065 | --d-- | ----------------- | -------------- | ----------- | ------- | --------- |
| SEQ ID NO | 20 | 15 | 16 | 17 | 18 | 19 |
| h011-3070 | ----- | ----------------- | ---n--f------r | ----------- | ------- | --------- |
| SEQ ID NO | 14 | 15 | 28 | 17 | 18 | 19 |
| h011-3073 | ----- | ----------------- | ----n-f-------r | ----------- | ------- | --------- |
| SEQ ID NO | 14 | 15 | 29 | 17 | 18 | 19 |
| h011-3093 | ----- | ----------------- | -------------- | ----------- | emv---- | --------- |
| SEQ ID NO | 14 | 15 | 16 | 17 | 35 | 19 |
| h011-3095 | ----- | ----------------- | -------------- | ----------- | e------ | --------- |
| SEQ ID NO | 14 | 15 | 16 | 17 | 36 | 19 |
| h011-3111 | ----- | ----------------- | -------------- | -------we-d | ------- | --------- |
| SEQ ID NO | 14 | 15 | 16 | 31 | 18 | 19 |
| h011-3118 | ----- | ----------------- | -------------- | -------we-v | ------- | --------- |
| SEQ ID NO | 14 | 15 | 16 | 32 | 18 | 19 |
| h011-3120 | ----- | ----------------- | -------------- | ----------- | ------- | --f-wf--- |
| SEQ ID NO | 14 | 15 | 16 | 17 | 18 | 38 |
| h011-3121 | ----- | ----------------- | -------------- | ----------- | ------- | --l--q-e- |
| SEQ ID NO | 14 | 15 | 16 | 17 | 18 | 39 |
| h011-3133 | --e-- | -----a----------- | -------------- | ----------- | ------- | --------- |
| SEQ ID NO | 21 | 24 | 16 | 17 | 18 | 19 |
| h011-3147 | ----- | e-i-------------- | -------------- | ----------- | ------- | --------- |
| SEQ ID NO | 14 | 25 | 16 | 17 | 18 | 19 |
| h011-3174 | ----- | ----------------- | -----f-------- | ----------- | q------ | --------- |
| SEQ ID NO | 14 | 15 | 30 | 17 | 37 | 19 |
| h011-3181 | ----- | ----------------- | -------------- | ----------- | ------- | --l----d- |
| SEQ ID NO | 14 | 15 | 16 | 17 | 18 | 40 |
| h011-3187 | ----- | ----------------- | -------------- | ----------- | ------- | --1--s-e- |
| SEQ ID NO | 14 | 15 | 16 | 17 | 18 | 41 |
| h011-3190 | ----- | e---------------- | -------------- | ----------- | ------- | --------- |
| SEQ ID NO | 14 | 26 | 16 | 17 | 18 | 19 |
| h011-3191 | ----- | --v-------------- | -------------- | ----------- | ------- | --------- |
| SEQ ID NO | 14 | 27 | 16 | 17 | 18 | 19 |
| h011-3192 | ----- | ----------------- | -------------- | -------e-- | ------- | --------- |
| SEQ ID NO | 14 | 15 | 16 | 33 | 18 | 19 |
| h011-3193 | ----- | ----------------- | -------------- | d | ------- | --------- |
| SEQ ID NO | 14 | 15 | 16 | 34 | 18 | 19 |
| h011-3194 | ----- | ----------------- | -------------- | ----------- | ------- | --1------ |
| SEQ ID N0 | 14 | 15 | 16 | 17 | 18 | 42 |
| h011-3195 | ----- | ----------------- | -----f-------- | ----------- | ------- | --------- |
| SEQ ID NO | 14 | 15 | 30 | 17 | 18 | 19 |

The CDR sequences of the artificial combination clones in Table 8 above could be defined and summarized in Table 9:

**Table 9 CDR sequences of the antibodies in the present invention**

| Clone No. | HCDR1 | HCDR2 | HCDR3 | LCDR1 | LCDR2 | LCDR3 |
|---|---|---|---|---|---|---|
| h011 3 | GYX¹IH | X²IX³PSX⁴TYTKFNQKFKD | AREX⁵IX⁶X⁷NYWFFDX⁸ | KASQNVYX₁X₂VX₃ | X₄X₅X₆NRYT | QQX₇SX₈X₉PX₁₀T |
| SEQ ID NO | 43 | 44 | 45 | 46 | 47 | 48 |

X¹ is selected from T, D or E;
X² is selected from Y or E; X³is selected from N, L, I or V; X⁴ is selected from S, G or A;
X⁵ is selected from R or N; X⁶ is selected from Y or F; X⁷ is selected from S or F; X⁸ is selected from V or R;
X₁ is selected from T or W; X₂ is selected from A or E; X₃ is selected from A, D or V;
X₄ is selected from S, E or Q; X₅ is selected from A or M; X₆ is selected from S or V;
X₇ is selected from Y, F or L; X₈ is selected from S or W; X₉ is selected from Y, F, Q or S; X₁₀ is selected from Y, D or E.
The artificial combinations of the light and heavy chain variable region sequences of monoclonal antibodies were shown in Table 10 below:

**Table 10 Combinations of variable region sequences of monoclonal antibody**

| Clone No. | VH | VL |
|---|---|---|
| | SEQ ID NO | SEQ ID NO |
| h011-3 | 12 | 13 |
| h011-3050 | 49 | 13 |
| h011-3058 | 50 | 13 |
| h011-3065 | 51 | 13 |
| h011-3070 | 52 | 13 |
| h011-3073 | 53 | 13 |
| h011-3093 | 12 | 59 |
| h011-3095 | 12 | 60 |
| h011-3111 | 12 | 61 |
| h011-3118 | 12 | 62 |
| h011-3120 | 12 | 63 |
| h011-3121 | 12 | 64 |
| h011-3133 | 54 | 13 |
| h011-3147 | 55 | 13 |
| h011-3174 | 56 | 65 |
| h011-3181 | 12 | 66 |
| h011-3187 | 12 | 67 |
| h011-3190 | 57 | 13 |
| h011-3191 | 58 | 13 |
| h011-3192 | 12 | 68 |
| h011-3193 | 12 | 69 |
| h011-3194 | 12 | 70 |
| h011-3195 | 56 | 13 |

The variable region sequences of the light and heavy chains were shown in Table 11:

| SEQ ID NO | sequence |
|---|---|
| 50 | QVQLVQSGAEVKKPGASVKVSCKASGYTFTGYTIHWVRQAPGQGLEWMGEINPSGTYTKFNQKFKDRVTMTRDTSISTAYM ELSRLRSDDTAVYYCARERIYSNYWFFDVWGQGTTVTVSS |
| 51 | QVQLVQSGAEVKKPGASVKVSCKASGYTFTGYDIHWVRQAPGQGLEWMGYINPSSTYTKFNQKFKDRVTMTRDTSISTAYM ELSRLRSDDTAVYYCARERIYSNYWFFDVWGQGTTVTVSS |
| 52 | QVQLVQSGAEVKKPGASVKVSCKASGYTFTGYTIHWVRQAPGQGLEWMGYINPSSTYTKFNQKFKDRVTMTRDTSISTAYM ELSRLRSDDTAVYYCARENIYFNYWFFDRWGQGTTVTVSS |
| 53 | QVQLVQSGAEVKKPGASVKVSCKASGYTFTGYTIHWVRQAPGQGLEWMGYINPSSTYTKFNQKFKDRVTMTRDTSISTAYM ELSRLRSDDTAVYYCARENIFSNYWFFDRWGQGTTVTVSS |
| 54 | QVQLVQSGAEVKKPGASVKVSCKASGYTFTGYEIHWVRQAPGQGLEWMGYINPSATYTKFNQKFKDRVTMTRDTSISTAYM ELSRLRSDDTAVYYCARERIYSNYWFFDVWGQGTTVTVSS |
| 55 | QVQLVQSGAEVKKPGASVKVSCKASGYTFTGYTIHWVRQAPGQGLEWMGEIIPSSTYTKFNQKFKDRVTMTRDTSISTAYM ELSRLRSDDTAVYYCARERIYSNYWFFDVWGQGTTVTVSS |
| 56 | QVQLVQSGAEVKKPGASVKVSCKASGYTFTGYTIHWVRQAPGQGLEWMGYINPSSTYTKFNQKFKDRVTMTRDTSISTAYM ELSRLRSDDTAVYYCARERIFSNYWFFDVWGQGTTVTVSS |
| 57 | QVQLVQSGAEVKKPGASVKVSCKASGYTFTGYTIHWVRQAPGQGLEWMGEINPSSTYTKFNQKFKDRVTMTRDTSISTAYM ELSRLRSDDTAVYYCARERIYSNYWFFDVWGQGTTVTVSS |
| 58 | QVQLVQSGAEVKKPGASVKVSCKASGYTFTGYTIHWVRQAPGQGLEWMGYIVPSSTYTKFNQKFKDRVTMTRDTSISTAYM ELSRLRSDDTAVYYCARERIYSNYWFFDVWGQGTTVTVSS |
| 59 | DIVMTQSPSSLSASVGDRVTITCKASQNVYTAVAWYQQKPGKSPKLLIYEMVNRYTGVPSRFSGSGSGTDFTLTISSLQPE DFATYFCQQYSSYPYTFGGGTKVEIK |
| 60 | DIVMTQSPSSLSASVGDRVTITCKASQNVYTAVAWYQQKPGKSPKLLIYEASNRYTGVPSRFSGSGSGTDFTLTISSLQPE DFATYFCQQYSSYPYTFGGGTKVEIK |
| 61 | DIVMTQSPSSLSASVGDRVTITCKASQNVYWEVDWYQQKPGKSPKLLIYSASNRYTGVPSRFSGSGSGTDFTLTISSLQPE DFATYFCQQYSSYPYTFGGGTKVEIK |
| 62 | DIVMTQSPSSLSASVGDRVTITCKASQNVYWEVVWYQQKPGKSPKLLIYSASNRYTGVPSRFSGSGSGTDFTLTISSLQPE DFATYFCQQYSSYPYTFGGGTKVEIK |
| 63 | DIVMTQSPSSLSASVGDRVTITCKASQNVYTAVAWYQQKPGKSPKLLIYSASNRYTGVPSRFSGSGSGTDFTLTISSLQPE DFATYFCQQFSWFPYTFGGGTKVEIK |
| 64 | DIVMTQSPSSLSASVGDRVTITCKASQNVYTAVAWYQQKPGKSPKLLIYSASNRYTGVPSRFSGSGSGTDFTLTISSLQPE DFATYFCQQLSSQPETFGGGTKVEIK |
| 65 | DIVMTQSPSSLSASVGDRVTITCKASQNVYTAVAWYQQKPGKSPKLLIYQASNRYTGVPSRFSGSGSGTDFTLTISSLQPE DFATYFCQQYSSYPYTFGGGTKVEIK |
| 66 | DIVMTQSPSSLSASVGDRVTITCKASQNVYTAVAWYQQKPGKSPKLLIYSASNRYTGVPSRFSGSGSGTDFTLTISSLQPE DFATYFCQQLSSYPDTFGGGTKVEIK |
| 67 | DIVMTQSPSSLSASVGDRVTITCKASQNVYTAVAWYQQKPGKSPKLLIYSASNRYTGVPSRFSGSGSGTDFTLTISSLQPE DFATYFCQQLSSSPETFGGGTKVEIK |
| 68 | DIVMTQSPSSLSASVGDRVTITCKASQNVYTEVAWYQQKPGKSPKLLIYSASNRYTGVPSRFSGSGSGTDFTLTISSLQPE DFATYFCQQYSSYPYTFGGGTKVEIK |
| 69 | DIVMTQSPSSLSASVGDRVTITCKASQNVYTAVDWYQQKPGKSPKLLIYSASNRYTGVPSRFSGSGSGTDFTLTISSLQPE DFATYFCQQYSSYPYTFGGGTKVEIK |
| 70 | DIVMTQSPSSLSASVGDRVTITCKASQNVYTAVAWYQQKPGKSPKLLIYSASNRYTGVPSRFSGSGSGTDFTLTISSLQPE DFATYFCQQLSSYPYTFGGGTKVEIK |

### EXAMPLE 8. Construction and Expression of IgG1 and IgG1-YTE Formats of Anti-human PCSK9 Humanized Antibodies

PCR primers were designed to construct VH/VK gene fragments of humanized antibodies, the VH/VK gene fragments were then homologously recombinated with expression vector pHr (with signal peptide and constant region gene (CH1-FC/CL) fragment) to construct full-length antibody expression vector VH-CH1-FC-pHr/VK-CL-pHr. The IgGl-YTE antibody format can be obtained via point mutation of the IgG1 antibody format. Several antibody formats were designed and constructed, 1) h011-3133-WT: h011-3133-IgG1 format, i.e., humanized sequence combination h011-3133, in combination with the heavy chain constant regions from human IgG1 and the light chain constant regions from human kappa chains; 2) h011-3133-YTE: h011-3133-IgG1-YTE format, i.e., humanized sequence combination h011-3133, in combination with the heavy chain constant regions from mutant human IgG1 (YTE mutant) and the light chain constant regions from human kappa chains. The mutant human IgG1 can also be another format of mutation.

The mutated antibody was tested for its affinity with BIAcore (test 6). The results were shown in Table 16.

### h011-3133-IgG1

### The amino acid sequence of heavy chain: IgG1

QVQLVQSGAEVKKPGASVKVSCKASGYTFTGYEIHWVRQAPGQGLEWM
GYINPSATYTKFNQKFKDRVTMTRDTSISTAYMELSRLRSDDTAVYYCAR
ERIYSNYWFFDVWGQGTTVTVSSASTKGPSVFPLAPSSKSTSGGTAALGC
LVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPP
KPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLS
LSPGK
SEQ ID NO: 71

### Heavy chain DNA sequence:

ATGGAGTTTGGGCTGAGCTGGCTTTTTCTTGTCGCGATTCTTAAGGGT
GTCCAGTGCCAGGTGCAGCTGGTGCAGTCTGGCGCCGAGGTGAAGAA
GCCCGGAGCATCTGTGAAGGTGTCTTGTAAGGCCTCTGGCTATACCT
TTACCGGCTACGAGATCCACTGGGTGCGGCAGGCACCCGGGCAGGGC
CTGGAGTGGATGGGCTACATCAACCCCTCTGCTACCTACACCAAGTTT
AACCAGAAGTTCAAGGACCGGGTGACCATGACCCGGGACACCTCTAT
CTCTACCGCCTACATGGAGCTGTCTCGGCTGCGGTCTGACGACACCG
CCGTGTACTACTGCGCACGCGAACGGATCTACTCTAACTACTGGTTCT
TCGACGTGTGGGGCCAGGGCACCACCGTGACCGTGTCTTCTGCTTCG
ACCAAGGGCCCATCGGTCTTCCCCCTGGCACCCTCCTCCAAGAGCAC
CTCTGGGGGCACAGCGGCCCTGGGCTGCCTGGTCAAGGACTACTTCC
CCGAACCGGTGACGGTGTCGTGGAACTCAGGCGCCCTGACCAGCGGC
GTGCACACCTTCCCGGCTGTCCTACAGTCCTCAGGACTCTACTCCCTC
AGCAGCGTGGTGACCGTGCCCTCCAGCAGCTTGGGCACCCAGACCTA
CATCTGCAACGTGAATCACAAGCCCAGCAACACCAAGGTGGACAAGA
AAGTTGAGCCCAAATCTTGTGACAAAACTCACACATGCCCACCGTGCC
CAGCACCTGAACTCCTGGGGGGACCGTCAGTCTTCCTCTTCCCCCCA
AAACCCAAGGACACCCTCATGATCTCCCGGACCCCTGAGGTCACATG
CGTGGTGGTGGACGTGAGCCACGAAGACCCTGAGGTCAAGTTCAACT

GGTACGTGGACGGCGTGGAGGTGCATAATGCCAAGACAAAGCCGCG
GGAGGAGCAGTACAACAGCACGTACCGTGTGGTCAGCGTCCTCACCG
TCCTGCACCAGGACTGGCTGAATGGCAAGGAGTACAAGTGCAAGGTC
TCCAACAAAGCCCTCCCAGCCCCCATCGAGAAAACCATCTCCAAAGC
CAAAGGGCAGCCCCGAGAACCACAGGTGTACACCCTGCCCCCATCCC
GGGATGAGCTGACCAAGAACCAGGTCAGCCTGACCTGCCTGGTCAAA
GGCTTCTATCCCAGCGACATCGCCGTGGAGTGGGAGAGCAATGGGCA
GCCGGAGAACAACTACAAGACCACGCCTCCCGTGCTGGACTCCGACG
GCTCCTTCTTCCTCTACAGCAAGCTCACCGTGGACAAGAGCAGGTGG
CAGCAGGGGAACGTCTTCTCATGCTCCGTGATGCATGAGGCTCTGCA
CAACCACTACACGCAGAAGAGCCTCTCCCTGTCTCCGGGTAAATGA
SEQ ID NO: 72

### h011-3133-kappa

### The amino acid sequence of light chain: kappa light chain

DIVMTQSPSSLSASVGDRVTITCKASQNVYTAVAWYQQKPGKSPKLLIYS
ASNRYTGVPSRFSGSGSGTDFTLTISSLQPEDFATYFCQQYSSYPYTFGGG
TKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGL
SSPVTKSFNRGEC
SEQ ID NO: 73

### Light chain DNA sequence:

ATGGACATGCGCGTGCCCGCCCAGCTGCTGGGCCTGCTGCTGCTGTG
GTTCCCCGGCTCGCGATGCGACATCGTGATGACCCAGTCTCCCTCAT
CTCTGAGTGCCTCTGTTGGCGACCGGGTGACCATCACCTGCAAAGCC
TCTCAGAACGTATACACAGCCGTGGCCTGGTATCAACAGAAGCCCGG
CAAGTCCCCCAAGCTGCTGATTTACTCTGCCTCTAACCGGTACACCGG
CGTGCCCTCTCGGTTCTCTGGCTCTGGTTCTGGCACCGACTTCACCCT
GACTATCTCTTCTCTGCAGCCCGAGGACTTCGCCACCTACTTCTGCCA
GCAGTACTCTTCTTACCCCTACACCTTCGGCGGAGGCACCAAGGTGG
AGATCAAGCGTACGGTGGCTGCACCATCTGTCTTCATCTTCCCGCCAT
CTGATGAGCAGTTGAAATCTGGAACTGCCTCTGTTGTGTGCCTGCTGA
ATAACTTCTATCCCAGAGAGGCCAAAGTACAGTGGAAGGTGGATAAC
GCCCTCCAATCGGGTAACTCCCAGGAGAGTGTCACAGAGCAGGACAG
CAAGGACAGCACCTACAGCCTCAGCAGCACCCTGACGCTGAGCAAAG
CAGACTACGAGAAACACAAAGTCTACGCCTGCGAAGTCACCCATCAG

GGCCTGAGCTCGCCCGTCACAAAGAGCTTCAACAGGGGAGAGTGTTG
A
SEQ ID NO: 74

### h011-3133-IgG1-YTE (light chain: h011-3133-kappa SEQ ID NO: 73)

### The amino acid sequence of heavy chain: IgG1-YTE

QVQLVQSGAEVKKPGASVKVSCKASGYTFTGYEIHWVRQAPGQGLEWM
GYINPSATYTKFNQKFKDRVTMTRDTSISTAYMELSRLRSDDTAVYYCAR
ERIYSNYWFFDVWGQGTTVTVSSASTKGPSVFPLAPSSKSTSGGTAALGC
LVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPP
KPKDTLYITREPEVTCVVVDVSHEDPEVKFNWYVDGVEVHNAKTKPREE
QYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLS
LSPGK
SEQ ID NO: 75

### Heavy chain DNA sequence:

ATGGAGTTTGGGCTGAGCTGGCTTTTTCTTGTCGCGATTCTTAAGGGT
GTCCAGTGCCAGGTGCAGCTGGTGCAGTCTGGCGCCGAGGTGAAGAA
GCCCGGAGCATCTGTGAAGGTGTCTTGTAAGGCCTCTGGCTATACCT
TTACCGGCTACGAGATCCACTGGGTGCGGCAGGCACCCGGGCAGGGC
CTGGAGTGGATGGGCTACATCAACCCCTCTGCTACCTACACCAAGTTT
AACCAGAAGTTCAAGGACCGGGTGACCATGACCCGGGACACCTCTAT
CTCTACCGCCTACATGGAGCTGTCTCGGCTGCGGTCTGACGACACCG
CCGTGTACTACTGCGCACGCGAACGGATCTACTCTAACTACTGGTTCT
TCGACGTGTGGGGCCAGGGCACCACCGTGACCGTGTCTTCTGCTTCG
ACCAAGGGCCCATCGGTCTTCCCCCTGGCACCCTCCTCCAAGAGCAC
CTCTGGGGGCACAGCGGCCCTGGGCTGCCTGGTCAAGGACTACTTCC
CCGAACCGGTGACGGTGTCGTGGAACTCAGGCGCCCTGACCAGCGGC
GTGCACACCTTCCCGGCTGTCCTACAGTCCTCAGGACTCTACTCCCTC
AGCAGCGTGGTGACCGTGCCCTCCAGCAGCTTGGGCACCCAGACCTA
CATCTGCAACGTGAATCACAAGCCCAGCAACACCAAGGTGGACAAGA
AAGTTGAGCCCAAATCTTGTGACAAAACTCACACATGCCCACCGTGCC
CAGCACCTGAACTCCTGGGGGGACCGTCAGTCTTCCTCTTCCCCCCA
AAACCCAAGGACACCCTCTACATCACCCGGGAGCCTGAGGTCACATG

CGTGGTGGTGGACGTGAGCCACGAAGACCCTGAGGTCAAGTTCAACT
GGTACGTGGACGGCGTGGAGGTGCATAATGCCAAGACAAAGCCGCG
GGAGGAGCAGTACAACAGCACGTACCGTGTGGTCAGCGTCCTCACCG
TCCTGCACCAGGACTGGCTGAATGGCAAGGAGTACAAGTGCAAGGTC
TCCAACAAAGCCCTCCCAGCCCCCATCGAGAAAACCATCTCCAAAGC
CAAAGGGCAGCCCCGAGAACCACAGGTGTACACCCTGCCCCCATCCC
GGGATGAGCTGACCAAGAACCAGGTCAGCCTGACCTGCCTGGTCAAA
GGCTTCTATCCCAGCGACATCGCCGTGGAGTGGGAGAGCAATGGGCA
GCCGGAGAACAACTACAAGACCACGCCTCCCGTGCTGGACTCCGACG
GCTCCTTCTTCCTCTACAGCAAGCTCACCGTGGACAAGAGCAGGTGG
CAGCAGGGGAACGTCTTCTCATGCTCCGTGATGCATGAGGCTCTGCA
CAACCACTACACGCAGAAGAGCCTCTCCCTGTCTCCGGGTAAATGA
SEQ ID NO:76

### Test 1 ELISA Experiment for PCSK9 Antibody Binding to PCSK9

The binding ability of anti-PCSK9 antibodies of the present invention to PCSK9 protein (WT PCSK9, SEQ ID NO: 5) was detected by measuring the amounts of antibodies binding to PCSK9 immobilized on the ELISA plate.

Streptavidin (sigma, CAT#S4762) was diulted to 2µg/ml with PBS and was added into 96-well ELISA plate, at 4 °C for overnight. The plate was washed and then was blocked with Tris buffer (including 0.9mM CaCl₂, 0.05% Tween 20 and 5% skim milk) at 37°C for 2 hours. Then the plate was washed again and was added with 100µl/well of biotin-labeled PCSK9 (produced in-house, diulted with Tris buffer containing 0.9mM CaCl₂, 0.05% Tween 20 and 1% skim milk), and incubated at 37°C for 1 hour. After washed, the plate was added with various concentrations of diluted PCSK9 antibody samples and incubated at 37 °C for 1 hour. Then the plate was washed again and added with HRP-goat-anti-human (H+L) antibody (jackson, CAT#109-035-088) and incubated at 37 °C for 1 hour. Then the plate was washed and added with tetramethyl brenzidine solution for development. Finally, the stop buffer was added and the OD450 value was measured on the Microplate reader, and then EC50 was calculated.

The results of ELISA test for detecting the binding ability of humanized and affinity maturation PCSK9 antibodies according to the present invention to human PCSK9 were shown in table 12.

**Table 12 Binding Assay of PCSK9 antibodies according to the present invention to PCSK9**

| Clone No. | EC50 (µg/ml) |
|---|---|
| h011-3050 | 0.00312 |
| h011-3058 | 0.00369 |
| h011-3065 | 0.00539 |
| h011-3070 | 0.00511 |
| h011-3073 | 0.00389 |
| h011-3093 | 0.00381 |
| h011-3095 | 0.00441 |
| h011-3111 | 0.00498 |
| h011-3118 | 0.00351 |
| h011-3120 | 0.00538 |
| h011-3121 | 0.00378 |
| h011-3133 | 0.00435 |
| h011-3147 | 0.00483 |
| h011-3174 | 0.00529 |
| h011-3181 | 0.00221 |
| h011-3187 | 0.00450 |
| h011-3190 | 0.00500 |
| h011-3191 | 0.00631 |
| h011-3192 | 0.00664 |
| h011-3193 | 0.00470 |
| h011-3194 | 0.00469 |
| h011-3195 | 0.00679 |
| h011-3 | 0.00719 |

The data showed that the humanized antibodies according to the present invention have excellent binding activity to PCSK9.

### Test 2 ELISA for Binding of PCSK9 Antibodies to PCSK9-Y

The binding ability of anti-PCSK9 antibodies of the present invention to PCSK9-Y protein (mutant PCSK9, SEQ ID NO: 6) was detected by measuring the amounts of antibodies binding to PCSK9-Y immobilized on the ELISA plate.

Streptavidin (sigma, CAT#S4762) was diulted to 2µg/ml with PBS and was added into 96-well ELISA plate, at 4 °C for overnight. The plate was washed and then was blocked with Tris buffer (including 0.9mM CaCl₂, 0.05% Tween 20 and 5% skim milk) at 37°C for 2 hours. The plate was washed and was added with 100µl/well of biotin-labeled PCSK9-Y (produced in-house, diulted with Tris buffer containing 0.9mM CaCl₂, 0.05% Tween 20 and 1% skim milk), and incubated at 37°C for 1 hour. After washed, the plate was added with various concentrations of diluted PCSK9 antibody samples and incubated at 37 °C for 1 hour. Then the plate was washed again and added with HRP-goat-anti-human (H+L) antibody (jackson, CAT#109-035-088) and incubated at 37°C for 1 hour. Then the plate was washed and added with tetramethyl brenzidine solution for development. Finally, the stop buffer was added and the OD450 value was measured on the Microplate reader, and then EC50 was calculated.

The results of ELISA test for detecting the binding ability of humanized and affinity maturation PCSK9 antibodies according to the present invention to human PCSK9-Y were shown in table 13.

**Table 13 Binding Assay of PCSK9 antibodies according to the present invention to PCSK9-Y**

| Clone No | EC50 (µg/ml) |
|---|---|
| h011-3050 | 0.00525 |
| h011-3058 | 0.00606 |
| h011-3065 | 0.01092 |
| h011-3070 | 0.01444 |
| h011-3073 | 0.01227 |
| h011-3093 | 0.00989 |
| h011-3095 | 0.01323 |
| h011-3111 | 0.00681 |
| h011-3118 | 0.00851 |
| h011-3120 | 0.00711 |
| h011-3121 | 0.00695 |
| h011-3133 | 0.00492 |
| h011-3147 | 0.00668 |
| h011-3174 | 0.01123 |
| h011-3181 | 0.00678 |
| h011-3187 | 0.00704 |
| h011-3190 | 0.00478 |
| h011-3191 | 0.00660 |
| h011-3192 | 0.12670 |
| h011-3193 | 0.02210 |
| h011-3194 | 0.01685 |
| h011-3195 | 0.02178 |
| h011-3 | 1.77950 |

The data showed that the humanized antibodies according to the present invention have excellent binding activity to PCSK9-Y.

### Test 3 Anti-PCSK9 Antibodies Block the Binding of LDLR-FC/PCSK9-Y

The blocking ability of PCSK9 antibodies according to the present invention for the binding of LDLR-FC and PCSK9-Y (mutant PCSK9, SEQ ID NO: 6) was tested by measuring the amount of PCSK9-Y binding to LDLR in the presence of the antibodies.

LDLR-FC (produced in-house, with sequence of SEQ ID NO: 8) was diluted to 2µg/ml with phosphate buffer and was added into the 96-well ELISA plate, at 4 °C overnight. The plate was washed and then blocked with Tris buffer (including 0.9mM CaCl₂, 0.05% Tween 20 and 5% skim milk) at 37 °C for 2 hours. Then the plate was washed and added with 100µl/well of mixture of biotin-labeled mutant PCSK9 (diulted to 1µg/ml with Tris buffer containing 0.9mM CaCl₂, 0.05% Tween 20 and 1% skim milk) and antibody samples (diulted with Tris buffer containing 0.9mM CaCl₂, 0.05% Tween 20 and 1% skim milk), and incubated at 37 °C for 1 hour. Then the plate was washed again and added with horseradish peroxidase-streptavidin (sigma, CAT#S2438) and incubated at 37 °C for 1 hour. Then the plate was washed and added with tetramethyl brenzidine solution for development. Finally, the stop buffer was added and OD450 value was measuredon the Microplate reader, then IC50 value was calculated.

The results of blocking test of the humanized and affinity maturation antibodies according to the present invention to the binding of LDLR-FC/PCSK9-Y, were shown in table 14.

**Table 14 Blocking test of PCSK9 antibodies to the binding of PCSK9-Y and LDLR**

| Clone No. | IC50 (µg/ml) |
|---|---|
| h011-3050 | 0.123 |
| h011-3058 | 0.162 |
| h011-3065 | 0.312 |
| h011-3070 | 0.228 |
| h011-3073 | 0.129 |
| h011-3093 | 0.287 |
| h011-3095 | 0.391 |
| h011-3111 | 0.226 |
| h011-3118 | 0.230 |
| h011-3120 | 0.213 |
| h011-3121 | 0.174 |
| h011-3133 | 0.175 |
| h011-3147 | 0.173 |
| h011-3174 | 0.230 |
| h011-3181 | 0.233 |
| h011-3187 | 0.156 |
| h011-3190 | 0.222 |
| h011-3191 | 0.226 |
| h011-3192 | 2.360 |
| h011-3193 | 0.871 |
| h011-3194 | 0.579 |
| h011-3195 | 0.689 |
| h011-3 | 5.489 |

The data showed that the PCSK9 antibodies according to the present invention can effectively block the binding of PCSK9-Y to LDLR.

The method above was also used to test the blocking effect of PCSK9 antibodies according to the present invention to the binding of other formats of LDLR-FC (produced in-house, with sequence of SEQ ID NO: 7 or SEQ ID NO: 9) to PCSK9 (SEQ ID NO: 5). The results showed that the PCSK9 antibodies according to the present invention can effectively block the binding of PCSK9 to truncated LDLR.

### Test 4 Anti-PCSK9 Antibodies Block the Binding of LDLR-FC/PCSK9

The blocking ability of PCSK9 antibodies according to the present invention for the binding of LDLR-FC (produced in-house, with sequence of SEQ ID NO: 8) and PCSK9 (SEQ ID NO: 5) was tested by measuring the amount of PCSK9 binding to LDLR in the presence of the antibodies.

LDLR-FC was diluted to 5µg/ml with phosphate buffer and was added into the 96-well ELISA plate, kept at 4 °C overnight. The plate was washed and then blocked with Tris buffer (including 0.9mM CaCl₂, 0.05% Tween 20 and 5% skim milk) at 37°C for 2 hours. Then the plate was washed and added with 100µl/well of mixture of biotin-labeled PCSK9 (diulted to 2µg/ml with Tris buffer containing 0.9mM CaCl₂, 0.05% Tween 20 and 1% skim milk) and antibody samples (diulted with Tris buffer containing 0.9mM CaCl₂, 0.05% Tween 20 and 1% skim milk), and incubated at 37°C for 1 hour. Then the plate was washed again and added with horseradish peroxidase-streptavidin (sigma, CAT#S2438) and incubated at 37°C for 1 hour. Then the plate was washed and added with tetramethyl brenzidine solution for development. Finally, the stop buffer was added and OD450 value was measuredon the Microplate reader, then IC50 value was calculated.

The results of blocking test of the humanized and affinity maturation antibodies according to the present invention to the binding of LDLR-FC/PCSK9, were shown in table 15.

**Table 15 Blocking test of PCSK9 antibodies to the binding of PCSK9 to LDLR**

| Clone No. | IC50 (µg/ml) |
|---|---|
| h011-3050 | 0.598 |
| h011-3058 | 0.542 |
| h011-3065 | 0.730 |
| h011-3070 | 0.629 |
| h011-3073 | 0.604 |
| h011-3093 | 0.706 |
| h011-3095 | 0.582 |
| h011-3111 | 1.224 |
| h011-3118 | 1.042 |
| h011-3120 | 0.911 |
| h011-3121 | 0.662 |
| h011-3133 | 0.495 |
| h011-3147 | 0.567 |
| h011-3174 | 1.671 |
| h011-3181 | 0.857 |
| h011-3187 | 0.666 |
| h011-3190 | 0.837 |
| h011-3191 | 0.740 |
| h011-3192 | 0.698 |
| h011-3193 | 0.621 |
| h011-3194 | 0.628 |
| h011-3195 | 2.252 |
| h011-3 | 0.681 |

The data showed that the PCSK9 antibodies according to the present invention can effectively block the binding of PCSK9 to LDLR.

The method above was also used to test the blocking effect of PCSK9 antibodies according to the present invention on the binding of other formats of LDLR-FC (produced in-house, with sequence of SEQ ID NO: 7 or SEQ ID NO: 9) to PCSK9 (SEQ ID NO: 5). The results showed that the PCSK9 antibodies according to the present invention can effectively block the binding of PCSK9 to truncated LDLR formats.

### Test 5 LDL uptake of PCSK9 Antibodies

HepG2 cells (Chinese Academy of Sciences cell bank, #CAT , TCHu72) were cultured in DMEM medium (Hyclone, #CAT SH30243.01B) (containing 10% FBS, Gibco, #CAT 10099-141). When the cells covered 80-90% of the plate, the cells were digested with blowing away and counted, 1.5*10⁴cells/well were plated in a 96-well plate. 24 h later, the medium was replaced for DMEM and 10% serum without lipoprotein (Millipore, CAT#LP4). 48 h later, the plate was washed twice with PBS buffer, then added with a mixture pre-incubated at 4°C for 1 hour of PCSK9 (SEQ ID NO: 1, final concentration was 10µg/ml) and antibody samples (diluted to various concentrations with medium), and BODIPY-®LDL with final concentration of 10µg/ml (Invitrogen, CAT#L3483), incubated at 37°C 6 h later, the plate was washed twice with PBS buffer. The fluorescence value was readout on Microplate reader (EX485nm/ EM535nm), then added with 50µl/well of CellTiter-Glo® Cell Activity Luminescence Detection Reagent (Promega, G7571), and the chemiluminescence value was readout. LDL uptake results were shown in figure 1 and 2, which indicated that PCSK9 antibodies according to the present invention can promote the LDL uptake by HepG2 cells.

### Test 6 BIAcore Assay for PCSK9 Antibody Affinity

According to the method described in the human Fab capture kit (Cat. # 28-9583-25, GE), the human Fab capture molecule was covalently linked to the CM5 biochip (Cat. # BR-1000-12, GE), so that the PCSK9 antibodies of the present invention were affinity captured. Then, human PCSK9 antigen (PCSK9 with His tag: PCSK9-His6, SEQ ID NO: 1) was flowed through the surface of the biochip, and the reaction signal was detected in real time using Biacore instrument to obtain the association and dissociation curves. Finally, the affinity values were obtained by fitting and shown at table 16 below. After each cycle of dissociation was finished, the biochip was washed and regenerated with regeneration solution in the human Fab capture kit (GE).

**Table 16 Affinity of PCSK9 Monoclonal Antibodies**

| Stationary phase | Mobile phase | Affinity(M) |
|---|---|---|
| h011-3133-WT | huPCSK9 | < 5.26E-11 |
| h011-3133-YTE | | < 5.18E-11 |

The result demonstrated that the PCSK9 antibodies of present invention have strong affinity to human PCSK9 antigen.

The same method was used to detect the affinity of PCSK9 antibodies to PCSK9-Y (SEQ ID NO: 4), and the results demonstrated that the PCSK9 antibodies of present invention have strong affinity to PCSK9-Y antigen.

### Test 7 Pharmacodynamic Test: PCSK9 Antibody Reduced the LDL-c in vivo

PCSK9-overexpressing mouse model was built up and the mice were injected with PCSK9 antibody via tail vein. The effect of the PCSK9 antibodies according to the present invention on reducing LDL-c level in vivo in PCSK9-overexpressing mice was evaluated. Human IgG (human immunoglobulin purified from the mixed normal human serum by traditional affinity chromatography, such as Protein A) was used as blank control.

C57Bl/6 mice (purchased from Shanghai Sippr-BK Laboratory Animal Co., Ltd.) were adapted for 5 days in the laboratory environment, and injected with 4×10¹¹ v.g. of AAV-PCSK9 virus (Benyuan Zhengyang Gene Technology Co., Ltd.) via tail vein. 10 days after the virus injection, the mice were fasted overnight. Then blood was taken from the eyelid and LDL-c was measured by HDL and LDL/VLDL Cholesterol Quantification Kit (purchased from BioVision, catalog number #K613-100). Mice were randomly divided into groups (6 mice/group (n=6) ) according to the concentration of LDL-c and were administered with antibodies via tail vein injection. Human IgG and PCSK9 antibody, produced in-house, were administered at a dose of 10 mg/kg (human IgG and PCSK9 antibody were both prepared in PBS at a concentration of 1 mg/ml). The mice were fasted overnight before blood sampling. 24 h, 48 h, and 72 h after administration, blood was taken from the eyelids, kept at 37°C for 1 hour, centrifuged at 3500 rpm for 10 minutes, and the serum was stored at -80°C.

After the last serum collection, all the frozen serum were tested on the same day. The concentration of LDL-c in the serum were measured by HDL and LDL/VLDL Cholesterol Quantification Kit in accordance with kit instructions.

### Discussion:

### 1. Pharmacodynamic Test: PCSK9 antibodies of Group h011-3133 reduced LDL-c level in vivo

10 day after the injection of the AAV8-PCSK9 virus, the concentrations of LDL-c in the serum were averaged as 53 mg/dl. 24 h after administration of antibodies to the divided groups, the concentrations of LDL-c of PCSK9 antibody groups of h011-3133-WT, h011-3133-YTE, h011-3191, h011-3065 were decreased by 62%, 40%, 56%, and 56%, respectively, compared to that of the IgG group. Each antibody-dosing group significantly reduced the concentration of LDL-c in the serum at a dose of 10 mg/kg and there was no significant difference between the antibody-dosing groups. 48 h after administration of antibodies, the concentration of LDL-c in the h011-3133-WT group was decreased by 18% compared to the IgG group, and there was no significant difference from the IgG group. The other groups were comparable to the IgG group. 72 h after administration of antibodies, the concentration of LDL-c in each group was comparable to that of the IgG group. The results were shown in Figure 3, Table 17 and Figure 5. Figure 3 shows the absolute value of serum LDL-c at different time points after administration of each sample. Figure 5 shows the percentage of LDL-c serum content of antibody-dosing group relative to IgG group at the same time point, and the value in IgG group was used as control of 100%.

In Figure 4, the concentration of LDL-c in the serum of normal mouse is about 6 mg/dl. 34 days after the injection of AAV8-PCSK9 virus, the concentrations of LDL-c in the serum were averaged as 33 mg/dl. Mice were divided into groups and administered immediately, 24 h after administration, the concentrations of LDL-c in h011-3058, h011-3191, h011-3147 groups were decreased by 49%, 40% and 29%, respectively, compared to the IgG group. h011-3058, h011-3191, h011-3147 significantly reduced the concentration of LDL-c in the serum at a dose of 10 mg/kg and there was no significant difference between the antibody-dosing groups. 48 h after administration, the concentrations of LDL-c in the h011-3058, h011-3191, h011-3147 groups were decreased by 12%, 11% and 9%, respectively, compared to the IgG group, and there was no significant difference from the IgG group. 72 h after administration, the concentration of LDL-c in each group was comparable to that of the IgG group. The results were shown in Figure 4, Table 18 and Figure 6. Figure 4 shows the absolute value of serum LDL-c at different time points after administration of each sample. Figure 6 shows the percentage of LDL-c serum content of antibody-dosing group relative to IgG group at the same time point, and the value in IgG group was used as control of 100%.

In summary, h011-3133-WT, h011-3133-YTE, h011-3191, h011-3065, h011-3058 and h011-3147 all were able to reduce the concentration of LDL-c in the serum of human PCSK9 overexpressed mice in this experiment.

**Table17 Changes in the concentrations of LDL-c in the serum of mice**

| **LDL-c (mg/dl)** | | | | | **%IgG** | | | |
|---|---|---|---|---|---|---|---|---|
| **Unit(10mg/kg)** | 0h | 24 h | 48 h | 72 h | 0h | 24h | 48h | 72h |
| **IgG** | 52.4±2.47 | 65.4±2.66 | 58.3±3.71 | 55.7±4.51 | 100 | 100 | 100 | 100 |
| **h011-3133-WT** | 52.8±2.12 | 24.6±1.09 | 47.7±8.38 | 55.0±6.35 | 101 | 38 | 82 | 99 |
| **h011-3133- YTE** | 52.9±2.08 | 39.0±4.40 | 68.5±4.85 | 59.4±3.36 | 101 | 60 | 118 | 107 |
| **h011-3191** | 52.9±2.15 | 28.7±4.04 | 72.2±7.39 | 73.2±2.66 | 101 | 44 | 124 | 131 |
| **h011-3065** | 54.3±6.30 | 28.6±3.78 | 53.4±4.09 | 54.1±5.21 | 104 | 44 | 92 | 97 |

**Table 18 Changes in the concentration of LDL-c in the serum of mice**

| **LDL-c (mg/dl)** | | | | | **%IgG** | | | |
|---|---|---|---|---|---|---|---|---|
| **Unit (10mg/kg)** | 0h | 24 h | 48 h | 72 h | 0h | 24h | 48h | 72h |
| **IgG** | 33.3±2.57 | 39.1±3.81 | 40.8±3.56 | 31.7±2.47 | 100 | 100 | 100 | 100 |
| **h011-3058** | 32.8±1.59 | 20.0±2.59 | 35.9±3.83 | 32.0±3.49 | 98 | 51 | 88 | 101 |
| **h011-3191** | 34.2±1.18 | 23.3±3.13 | 36.1±4.19 | 34.1±1.36 | 103 | 60 | 89 | 108 |
| **h011-3147** | 31.7±1.03 | 27.8±3.32 | 37.2±4.13 | 37.9±4.31 | 95 | 71 | 91 | 119 |

### Test 8 Competitive Experiment

In the competitive ELISA experiment, the plate was coated with one antibody overnight. Then biotin-PCSK9-his and a competitive antibody at concentration 50 times higher than the coating antibody were added. The coating antibody will compete with the competitive antibody to bind to an antigen. The antigen signal at the plate was then tested. The results show that, h011-3133-YTE and 21B12 (US8030457B2) can compete to bind to antigen, however, there is not obviously competition binding between the two antibodies, suggesting antigen epitopes of the two antibodies are different.

| IR (%) | h011-3133-YTE | 21B12 |
|---|---|---|
| h011-3133-YTE | 96.17 | -0.28 |
| 21B12 | 3.01 | 97.78 |

Although the present invention has been described in detail with the aid of the drawings and examples for the sake of clarity, the description and examples should not be construed as limiting the scope of the invention. The disclosures of all patents and scientific literature cited herein are hereby incorporated by reference in their entirety.

## Claims

1. A PCSK9 antibody specifically binding to PCSK9 or antigen-binding fragment thereof, comprising variable region comprising at least one or more CDR regions selected from:
i) HCDR1, wherein the sequence of said HCDR1 is GYX¹IH (SEQ ID NO: 43), wherein X¹ is T, D or E;
ii) HCDR2, wherein the sequence of said HCDR2 is X²IX³PSX⁴TYTKFNQKFKD (SEQ ID NO: 44), wherein X² is Y or E; X³ is N, L, I or V; X⁴ is S, G or A;
iii) HCDR3, wherein the sequence of said HCDR3 is AREX⁵IX⁶X⁷NYWFFDX⁸ (SEQ ID NO: 45), wherein X⁵ is R or N; X⁶ is Y or F; X⁷ is S or F; X⁸ is V or R;
iv) LCDR1, wherein the sequence of said LCDR1 is KASQNVYX₁X₂VX₃ (SEQ ID NO: 46), wherein X₁ is T or W; X₂ is A or E; X₃ is A, D or V;
v) LCDR2, wherein the sequence of said LCDR2 is X₄X₅X₆NRYT (SEQ ID NO: 47), wherein X₄ is S, E or Q; X₅ is A or M; X₆ is S or V; and
vi) LCDR3, wherein the sequence of said LCDR3 is QQX₇SX₈X₉PX₁₀T (SEQ ID NO: 48), wherein X₇ is Y, F or L; X₈ is S or W; X₉ is Y, F, Q or S; X₁₀ is Y, D or E.

2. The PCSK9 antibody specifically binding to PCSK9 or antigen-binding fragment thereof according to claim 1, wherein the PCSK9 antibody or antigen-binding fragment thereof comprises HCDR1, HCDR2 and HCDR3 as shown in SEQ ID NO:43, SEQ ID NO:44 and SEQ ID NO: 45, respectively.

3. The PCSK9 antibody specifically binding to PCSK9 or antigen-binding fragment thereof according to claim 1, wherein the PCSK9 antibody or antigen-binding fragment thereof comprises LCDR1, LCDR2 and LCDR3 as shown in SEQ ID NO:46, SEQ ID NO:47 and SEQ ID NO: 48, respectively.

4. The PCSK9 antibody specifically binding to PCSK9 or antigen-binding fragment thereof according to any one of claims 1-3, wherein the PCSK9 antibody or antigen-binding fragment thereof comprises HCDR1, HCDR2 and HCDR3 as shown in SEQ ID NO: 43, SEQ ID NO: 44 and SEQ ID NO: 45, respectively and LCDR1, LCDR2 and LCDR3 as shown in SEQ ID NO: 46, SEQ ID NO: 47 and SEQ ID NO: 48, respectively.

5. The PCSK9 antibody specifically binding to PCSK9 or antigen-binding fragment thereof according to claim 1 or claim 2, wherein said HCDR1 is selected from amino acid sequence SEQ ID NO:14, SEQ ID NO:20 or SEQ ID NO:21, or amino acid sequence having at least 95% identity to the above sequences;
HCDR2 is selected from amino acid sequence SEQ ID NO: 15, SEQ ID NO: 22, SEQ ID NO: 23, SEQ ID NO: 24, SEQ ID NO: 25, SEQ ID NO: 26 or SEQ ID NO: 27, or amino acid sequence having at least 95% identity to the above sequences;
HCDR3 is selected from amino acid sequence SEQ ID NO:16, SEQ ID NO:28, SEQ ID NO:29 or SEQ ID NO:30, or amino acid sequence having at least 95% identity to the above sequences.

6. The PCSK9 antibody specifically binding to PCSK9 or antigen-binding fragment thereof according to claim 1 or claim 3, wherein said LCDR1 is selected from amino acid sequence SEQ ID NO: 17, SEQ ID NO: 31, SEQ ID NO: 32, SEQ ID NO: 33 or SEQ ID NO: 34, or amino acid sequence having at least 95% identity to the above sequences;
LCDR2 is selected from amino acid sequence SEQ ID NO: 18, SEQ ID NO: 35, SEQ ID NO: 36 or SEQ ID NO: 37, or amino acid sequence having at least 95% identity to the above sequences;
LCDR3 is selected from amino acid sequence SEQ ID NO: 19, SEQ ID NO: 38, SEQ ID NO: 39, SEQ ID NO: 40, SEQ ID NO: 41 or SEQ ID NO: 42, or amino acid sequence having at least 95% identity to the above sequences.

7. The PCSK9 antibody specifically binding to PCSK9 or antigen-binding fragment thereof according to any one of claims 1-6, wherein said HCDR1 is selected from amino acid sequence SEQ ID NO:14, SEQ ID NO:20 or SEQ ID NO:21, or amino acid sequence having at least 95% identity to the above sequences;
HCDR2 is selected from amino acid sequence SEQ ID NO: 15, SEQ ID NO: 22, SEQ ID NO: 23, SEQ ID NO: 24, SEQ ID NO: 25, SEQ ID NO: 26 or SEQ ID NO: 27, or amino acid sequence having at least 95% identity to the above sequences;
HCDR3 is selected from amino acid sequence SEQ ID NO: 16, SEQ ID NO: 28, SEQ ID NO: 29 or SEQ ID NO: 30, or amino acid sequence having at least 95% identity to the above sequences; and
LCDR1 is selected from amino acid sequence SEQ ID NO: 17, SEQ ID NO: 31, SEQ ID NO: 32, SEQ ID NO: 33 or SEQ ID NO: 34, or amino acid sequence having at least 95% identity to the above sequences;
LCDR2 is selected from amino acid sequence SEQ ID NO: 18, SEQ ID NO: 35, SEQ ID NO: 36 or SEQ ID NO: 37, or amino acid sequence having at least 95% identity to the above sequences;
LCDR3 is selected from amino acid sequence SEQ ID NO: 19, SEQ ID NO: 38, SEQ ID NO: 39, SEQ ID NO: 40, SEQ ID NO: 41 or SEQ ID NO: 42, or amino acid sequence having at least 95% identity to the above sequences.

8. The PCSK9 antibody specifically binding to PCSK9 or antigen-binding fragment thereof according to any one of claims 1-7, wherein the PCSK9 antibody comprising six CDRs selected from the group consisting of:
1) Six CDRs as shown in SEQ ID NO: 14, SEQ ID NO: 15, SEQ ID NO: 16, SEQ ID NO: 17, SEQ ID NO: 18 and SEQ ID NO: 19, respectively;
2) Six CDRs as shown in SEQ ID NO: 14, SEQ ID NO: 22, SEQ ID NO: 16, SEQ ID NO: 17, SEQ ID NO: 18 and SEQ ID NO: 19, respectively;
3) Six CDRs as shown in SEQ ID NO: 14, SEQ ID NO: 23, SEQ ID NO: 16, SEQ ID NO: 17, SEQ ID NO: 18 and SEQ ID NO: 19, respectively;
4) Six CDRs as shown in SEQ ID NO: 20, SEQ ID NO: 15, SEQ ID NO: 16, SEQ ID NO: 17, SEQ ID NO: 18 and SEQ ID NO: 19, respectively;
5) Six CDRs as shown in SEQ ID NO: 14, SEQ ID NO: 15, SEQ ID NO: 28, SEQ ID NO: 17, SEQ ID NO: 18 and SEQ ID NO: 19, respectively;
6) Six CDRs as shown in SEQ ID NO: 14, SEQ ID NO: 15, SEQ ID NO: 29, SEQ ID NO: 17, SEQ ID NO: 18 and SEQ ID NO: 19, respectively;
7) Six CDRs as shown in SEQ ID NO: 14, SEQ ID NO: 15, SEQ ID NO: 16, SEQ ID NO: 17, SEQ ID NO: 35 and SEQ ID NO: 19, respectively;
8) Six CDRs as shown in SEQ ID NO: 14, SEQ ID NO: 15, SEQ ID NO: 16, SEQ ID NO: 17, SEQ ID NO: 36 and SEQ ID NO: 19, respectively;
9) Six CDRs as shown in SEQ ID NO: 14, SEQ ID NO: 15, SEQ ID NO: 16, SEQ ID NO: 31, SEQ ID NO: 18 and SEQ ID NO: 19, respectively;
10) Six CDRs as shown in SEQ ID NO: 14, SEQ ID NO: 15, SEQ ID NO: 16, SEQ ID NO: 32, SEQ ID NO: 18 and SEQ ID NO: 19, respectively;
11) Six CDRs as shown in SEQ ID NO: 14, SEQ ID NO: 15, SEQ ID NO: 16, SEQ ID NO: 17, SEQ ID NO: 18 and SEQ ID NO: 38, respectively;
12) Six CDRs as shown in SEQ ID NO: 14, SEQ ID NO: 15, SEQ ID NO: 16, SEQ ID NO: 17, SEQ ID NO: 18 and SEQ ID NO: 39, respectively;
13) Six CDRs as shown in SEQ ID NO: 21, SEQ ID NO: 24, SEQ ID NO: 16, SEQ ID NO: 17, SEQ ID NO: 18 and SEQ ID NO: 15, respectively;
14) Six CDRs as shown in SEQ ID NO: 14, SEQ ID NO: 25, SEQ ID NO: 16, SEQ ID NO: 17, SEQ ID NO: 18 and SEQ ID NO: 19, respectively;
15) Six CDRs as shown in SEQ ID NO: 14, SEQ ID NO: 15, SEQ ID NO: 30, SEQ ID NO: 17, SEQ ID NO: 37 and SEQ ID NO: 19, respectively;
16) Six CDRs as shown in SEQ ID NO: 14, SEQ ID NO: 15, SEQ ID NO: 16, SEQ ID NO: 17, SEQ ID NO: 18 and SEQ ID NO: 40, respectively;
17) Six CDRs as shown in SEQ ID NO: 14, SEQ ID NO: 15, SEQ ID NO: 16, SEQ ID NO: 17, SEQ ID NO: 18 and SEQ ID NO: 41, respectively;
18) Six CDRs as shown in SEQ ID NO: 14, SEQ ID NO: 26, SEQ ID NO: 16, SEQ ID NO: 17, SEQ ID NO: 18 and SEQ ID NO: 19, respectively;
19) Six CDRs as shown in SEQ ID NO: 14, SEQ ID NO: 27, SEQ ID NO: 16, SEQ ID NO: 17, SEQ ID NO: 18 and SEQ ID NO: 19, respectively;
20) Six CDRs as shown in SEQ ID NO: 14, SEQ ID NO: 15, SEQ ID NO: 16, SEQ ID NO: 33, SEQ ID NO: 18 and SEQ ID NO: 19, respectively;
21) Six CDRs as shown in SEQ ID NO: 14, SEQ ID NO: 15, SEQ ID NO: 16, SEQ ID NO: 34, SEQ ID NO: 18 and SEQ ID NO: 19, respectively;
22) Six CDRs as shown in SEQ ID NO: 14, SEQ ID NO: 15, SEQ ID NO: 16, SEQ ID NO: 17, SEQ ID NO: 18 and SEQ ID NO: 42, respectively; and
23) Six CDRs as shown in SEQ ID NO: 14, SEQ ID NO: 15, SEQ ID NO: 30, SEQ ID NO: 17, SEQ ID NO: 18 and SEQ ID NO: 19, respectively.

9. The PCSK9 antibody specifically binding to PCSK9 or antigen-binding fragment thereof according to any one of claims 1-8, wherein the PCSK9 antibody or the antigen-binding fragment thereof is a murine antibody or fragment thereof.

10. The PCSK9 antibody specifically binding to PCSK9 or antigen-binding fragment thereof according to any one of claims 1-8, wherein the PCSK9 antibody or antigen-binding fragment thereof is a chimeric antibody or fragment thereof.

11. The PCSK9 antibody specifically binding to PCSK9 or antigen-binding fragment thereof according to any one of claims 1-8, wherein the PCSK9 antibody or antigen-binding fragment thereof is a humanized antibody or fragment thereof.

12. The PCSK9 antibody specifically binding to PCSK9 or antigen-binding fragment thereof according to claim 11, wherein the humanized antibody comprises heavy chain variable FR region, wherein the heavy chain variable FR region is derived from a combination sequence of human germline heavy chains IGHV1-2*02 and hjh6.1 or mutant sequence thereof; wherein the heavy chain variable FR region comprises FR1, FR2, FR3 region from IGHV1-2*02 and FR4 region from hjh6.1 or mutant sequence thereof.

13. The PCSK9 antibody specifically binding to PCSK9 or antigen-binding fragment thereof according to claim 12, wherein the humanized antibody comprises heavy chain variable region as shown in SEQ ID NO: 10 or a variant thereof; wherein the variant means the present of 1, 2, 3, 4, 5, 6, 7, 8, 9 or 10 amino acid changes in SEQ ID NO: 10.

14. The PCSK9 antibody specifically binding to PCSK9 or antigen-binding fragment thereof according to claim 14, wherein the humanized antibody comprises heavy chain FR region with 1-10 amino acid back-mutation, wherein the back-mutation is preferably selected from one or more back-mutation consisting of R72A, T74K, V68A, M70L, M48V, G49A, R67K and R38K.

15. The PCSK9 antibody specifically binding to PCSK9 or antigen-binding fragment thereof according to any one of claims 1-11, wherein the PCSK9 antibody comprises VH, wherein the amino acid sequence of said VH is selected from amino acid sequence SEQ ID NO: 12, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57 or SEQ ID NO: 58, or amino acid sequence having at least 95% identity to the above sequences.

16. The PCSK9 antibody specifically binding to PCSK9 or antigen-binding fragment thereof according to claim 11, wherein the humanized antibody comprises light chain FR region on light chain variable region, wherein the sequence of the light chain FR region is derived from a combination sequence of human germline light chains IGKV1-39*01 and hjk4.1 or mutant sequence thereof; wherein the light chain FR region comprises FR1, FR2, FR3 from human germline light chain IGKV1-39*01 and FR4 from hjk4.1, or the mutant sequence thereof.

17. The PCSK9 antibody specifically binding to PCSK9 or antigen-binding fragment thereof according to claim 16, wherein the humanized antibody comprises light chain variable region as shown in SEQ ID NO: 11 or a variant thereof; wherein the variant means the presence of 1, 2, 3, 4, 5, 6, 7, 8, 9 or 10 amino acid changes in SEQ ID NO: 10.

18. The PCSK9 antibody specifically binding to PCSK9 or antigen-binding fragment thereof according to claim 16, wherein the humanized antibody comprises light chain FR region with 1-10 amino acid back-mutations, wherein the back-mutation is preferably selected from one or more back-mutations consisting of Q3V, A43S and Y87F.

19. The PCSK9 antibody specifically binding to PCSK9 or antigen-binding fragment thereof according to any one of claims 1-11, wherein the PCSK9 antibody comprises VL, wherein the amino acid sequence of said VL is selected from amino acid sequence SEQ ID NO: 13, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69 or SEQ ID NO: 70, or amino acid sequence having at least 95% identity to the above sequences.

20. The PCSK9 antibody specifically binding to PCSK9 or antigen-binding fragment thereof according to claim 11, wherein the PCSK9 antibody comprises VH and VL selected from the group consisting of:
1) VH of SEQ ID NO: 12 and VL of SEQID NO: 13;
2) VH of SEQ ID NO: 49 and VL of SEQID NO: 13;
3) VH of SEQ ID NO: 50 and VL of SEQID NO: 13;
4) VH of SEQ ID NO: 51 and VL of SEQID NO: 13;
5) VH of SEQ ID NO: 52 and VL of SEQID NO: 13;
6) VH of SEQ ID NO: 53 and VL of SEQID NO: 13;
7) VH of SEQ ID NO: 12 and VL of SEQID NO: 59;
8) VH of SEQ ID NO: 12 and VL of SEQID NO: 60;
9) VH of SEQ ID NO: 12 and VL of SEQID NO: 61;
10) VH of SEQ ID NO: 12 and VL of SEQID NO: 62;
11) VH of SEQ ID NO: 12 and VL of SEQID NO: 63;
12) VH of SEQ ID NO: 12 and VL of SEQID NO: 64;
13) VH of SEQ ID NO: 54 and VL of SEQID NO: 13;
14) VH of SEQ ID NO: 55 and VL of SEQID NO: 13;
15) VH of SEQ ID NO: 56 and VL of SEQID NO: 65;
16) VH of SEQ ID NO: 12 and VL of SEQID NO: 66;
17) VH of SEQ ID NO: 12 and VL of SEQID NO: 67;
18) VH of SEQ ID NO: 57 and VL of SEQID NO: 13;
19) VH of SEQ ID NO: 58 and VL of SEQID NO: 13;
20) VH of SEQ ID NO: 12 and VL of SEQID NO: 68;
21) VH of SEQ ID NO: 12 and VL of SEQID NO: 69;
22) VH of SEQ ID NO: 12 and VL of SEQID NO: 70; and
23) VH of SEQ ID NO: 56 and VL of SEQID NO: 13.

21. The PCSK9 antibody specifically binding to PCSK9 or antigen-binding fragment thereof according to any one of claims 10-20, wherein the PCSK9 antibody or antigen-binding fragment thereof further comprises a heavy chain constant region derived from human IgG1, IgG2, IgG3 or IgG4 or a variant thereof, or amino acid sequence having at least 95% identity to sequences thereof; preferably comprises a heavy chain constant region derived from human IgG1, IgG2 or IgG4 or from human IgG1, IgG2 or IgG4 variant wherein the amino acid mutations prolong the half-life of an antibody in serum, more preferably comprises IgG1, IgG2 or IgG4 heavy chain constant region introduced with YTE mutation;
wherein the PCSK9 antibody or antigen-binding fragment thereof further comprises a light chain constant region derived from human κ chain, human λ chain or a variant thereof, or amino acid sequence having at least 95% identity to the sequence thereof.

22. The PCSK9 antibody specifically binding to PCSK9 or antigen-binding fragment thereof according to claim 21, wherein the PCSK9 antibody or antigen-binding fragment thereof comprises:
1) heavy chain sequence of SEQ ID NO: 71 and light chain sequence of SEQ ID NO: 73; or
2) heavy chain sequence of SEQ ID NO: 75 and light chain sequence of SEQ ID NO: 73.

23. A DNA molecule encoding the PCSK9 antibody or antigen-binding fragment thereof according to any one of claims 1-22.

24. An expression vector comprising the DNA molecule according to claim 23.

25. A host cell transformed with the expression vector according to claim 24, wherein the host cell is selected from the group consisting of prokaryotic cells and eukaryotic cells, preferably eukaryotic cells, more preferably mammalian cells.

26. A method for preparing a PCSK9 antibody, wherein the method comprises culturing the host cells of claim 25 under conditions suitable for the expression of a nucleic acid encoding the PCSK9 antibody of any one of claims 1 to 22, and/or recovering the PCSK9 antibody from the host cells.

27. A pharmaceutical composition, comprising therapeutically effective amount of the PCSK9 antibody or the antigen-binging fragment thereof according to any one of claims 1-22, and one or more pharmaceutically acceptable carriers, diluents or excipients.

28. Use of the PCSK9 antibody or antigen-binding fragment thereof according to any one of claims 1-22, or the pharmaceutical composition according to claim 27, in the preparation of a medicament for treatment of PCSK9-mediated disease or condition, wherein the disease or condition is preferably cholesterol related disease; more preferably hypercholesterolemia, heart disease, metabolic syndrome, diabetes, coronary heart disease, stroke, cardiovascular disease, Alzheimer's disease and general dyslipidemia; most preferably hypercholesterolemia, dyslipidemia, atherosclerosis, CVD or coronary heart disease.


SEQUENCE LISTING

<110>  JIANGSU HENGRUI MEDICINE CO.,LTD  SHANGHAI HENGRUI PHARMACEUTICAL
CO.,LTD

<120>  PCSK9 ANTIBODY,ANTIGEN-BINDING FRAGMENT THEREOF,AND MEDICAL USES
THEREOF

<130>  760087CPCT

<160>  76

<170>  PatentIn version 3.3

<210>  1
<211>  698
<212>  PRT
<213>  Artificial

<220>
<223>  PCSK9 with his tag: PCSK9-His6, used as an immunogen for immunizing
mice or used as a detection reagent

<400>  1

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile

                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln His His His His His His
    690                 695

<210>  2
<211>  714
<212>  PRT
<213>  ARTIFICIAL

<220>
<223>  PCSK9 with PADRE peptide and his tag: PCSK9-PADRE-His6, used as an
immunogen wherein the contained PADRE peptide can promote immunization

<400>  2

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln Gly Ser Gly Ala Lys Phe Val Ala Ala Trp Thr Leu
    690                 695                 700

Lys Ala Ala Ala His His His His His His
705                 710

<210>  3
<211>  704
<212>  PRT
<213>  ARTIFICIAL

<220>
<223>  Fusion protein of PCSK9 containing TEV cleavage site and his tag:
PCSK9-TEV-His6, as an immunogen, N-PCSK9 (N-terminal PCSK9 domain) can be
obtained via TEV enzyme digestion

<400>  3

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg

145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Glu Asn Leu Tyr Phe Gln Gly Ala Gly Trp Gln Leu Phe Cys Arg
    450                 455                 460

Thr Val Trp Ser Ala His Ser Gly Pro Thr Arg Met Ala Thr Ala Val
465                 470                 475                 480

Ala Arg Cys Ala Pro Asp Glu Glu Leu Leu Ser Cys Ser Ser Phe Ser
                485                 490                 495

Arg Ser Gly Lys Arg Arg Gly Glu Arg Met Glu Ala Gln Gly Gly Lys
            500                 505                 510

Leu Val Cys Arg Ala His Asn Ala Phe Gly Gly Glu Gly Val Tyr Ala
        515                 520                 525

Ile Ala Arg Cys Cys Leu Leu Pro Gln Ala Asn Cys Ser Val His Thr
    530                 535                 540

Ala Pro Pro Ala Glu Ala Ser Met Gly Thr Arg Val His Cys His Gln
545                 550                 555                 560

Gln Gly His Val Leu Thr Gly Cys Ser Ser His Trp Glu Val Glu Asp
                565                 570                 575

Leu Gly Thr His Lys Pro Pro Val Leu Arg Pro Arg Gly Gln Pro Asn
            580                 585                 590

Gln Cys Val Gly His Arg Glu Ala Ser Ile His Ala Ser Cys Cys His
        595                 600                 605

Ala Pro Gly Leu Glu Cys Lys Val Lys Glu His Gly Ile Pro Ala Pro
    610                 615                 620

Gln Glu Gln Val Thr Val Ala Cys Glu Glu Gly Trp Thr Leu Thr Gly
625                 630                 635                 640

Cys Ser Ala Leu Pro Gly Thr Ser His Val Leu Gly Ala Tyr Ala Val
                645                 650                 655

Asp Asn Thr Cys Val Val Arg Ser Arg Asp Val Ser Thr Thr Gly Ser
            660                 665                 670

Thr Ser Glu Gly Ala Val Thr Ala Val Ala Ile Cys Cys Arg Ser Arg
        675                 680                 685

His Leu Ala Gln Ala Ser Gln Glu Leu Gln His His His His His His
    690                 695                 700

<210>  4
<211>  698
<212>  PRT
<213>  ARTIFICIAL

<220>
<223>  PCSK9-D374Y mutant protein with his tag: PCSK9-D374Y-His6, as a
detection reagent

<400>  4

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Tyr Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
        420                 425                 430

Gln Arg Val Leu Thr Pro Asn Leu Val Ala Ala Leu Pro Pro Ser Thr
    435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val

660                 665                 670

Thr Ala Val Ala Ile Cys Cys Arg Ser Arg His Leu Ala Gln Ala Ser
        675                 680                 685

Gln Glu Leu Gln His His His His His His
    690                 695

<210>  5
<211>  719
<212>  PRT
<213>  ARTIFICIAL

<220>
<223>  PCSK9 protein inserted with biotin receiving peptide and his tag:
PCSK9-BP15-His6. As a detection reagent, biotin will be labeled to the
position of BP15 peptide during the expression, avoiding the biotin labeling
in vitro and consequently avoiding possible conformational changes

<400>  5

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
        420                 425                 430

Gln Arg Val Leu Thr Pro Asn Leu Val Ala Ala Leu Pro Pro Ser Thr
    435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val

660                 665                 670

Thr Ala Val Ala Ile Cys Cys Arg Ser Arg His Leu Ala Gln Ala Ser
        675                 680                 685

Gln Glu Leu Gln Gly Ser Thr Ser Gly Ser Gly Leu Asn Asp Ile Phe
    690                 695                 700

Glu Ala Gln Lys Ile Glu Trp His Glu His His His His His His
705                 710                 715

<210>  6
<211>  719
<212>  PRT
<213>  ARTIFICIAL

<220>
<223>  PCSK9 D374Y mutant protein inserted with biotin receiving peptide and
his tag: PCSK9-D374Y-BP15-His6, as a detection protein

<400>  6

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg

145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Tyr Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln Gly Ser Thr Ser Gly Ser Gly Leu Asn Asp Ile Phe
    690                 695                 700

Glu Ala Gln Lys Ile Glu Trp His Glu His His His His His His
705                 710                 715

<210>  7
<211>  802
<212>  PRT
<213>  ARTIFICIAL

<220>
<223>  PCSK9 receptor protein LDLR extracellular domain with Flag tag and
his tag: LDLR-ECD-Flag-His6 as a detection reagent

<400>  7

Met Gly Pro Trp Gly Trp Lys Leu Arg Trp Thr Val Ala Leu Leu Leu
1               5                   10                  15

Ala Ala Ala Gly Thr Ala Val Gly Asp Arg Cys Glu Arg Asn Glu Phe
            20                  25                  30

Gln Cys Gln Asp Gly Lys Cys Ile Ser Tyr Lys Trp Val Cys Asp Gly
        35                  40                  45

Ser Ala Glu Cys Gln Asp Gly Ser Asp Glu Ser Gln Glu Thr Cys Leu
    50                  55                  60

Ser Val Thr Cys Lys Ser Gly Asp Phe Ser Cys Gly Gly Arg Val Asn
65                  70                  75                  80

Arg Cys Ile Pro Gln Phe Trp Arg Cys Asp Gly Gln Val Asp Cys Asp
                85                  90                  95

Asn Gly Ser Asp Glu Gln Gly Cys Pro Pro Lys Thr Cys Ser Gln Asp
            100                 105                 110

Glu Phe Arg Cys His Asp Gly Lys Cys Ile Ser Arg Gln Phe Val Cys
        115                 120                 125

Asp Ser Asp Arg Asp Cys Leu Asp Gly Ser Asp Glu Ala Ser Cys Pro
    130                 135                 140

Val Leu Thr Cys Gly Pro Ala Ser Phe Gln Cys Asn Ser Ser Thr Cys
145                 150                 155                 160

Ile Pro Gln Leu Trp Ala Cys Asp Asn Asp Pro Asp Cys Glu Asp Gly
                165                 170                 175

Ser Asp Glu Trp Pro Gln Arg Cys Arg Gly Leu Tyr Val Phe Gln Gly
            180                 185                 190

Asp Ser Ser Pro Cys Ser Ala Phe Glu Phe His Cys Leu Ser Gly Glu
        195                 200                 205

Cys Ile His Ser Ser Trp Arg Cys Asp Gly Gly Pro Asp Cys Lys Asp
    210                 215                 220

Lys Ser Asp Glu Glu Asn Cys Ala Val Ala Thr Cys Arg Pro Asp Glu
225                 230                 235                 240

Phe Gln Cys Ser Asp Gly Asn Cys Ile His Gly Ser Arg Gln Cys Asp
                245                 250                 255

Arg Glu Tyr Asp Cys Lys Asp Met Ser Asp Glu Val Gly Cys Val Asn
            260                 265                 270

Val Thr Leu Cys Glu Gly Pro Asn Lys Phe Lys Cys His Ser Gly Glu
        275                 280                 285

Cys Ile Thr Leu Asp Lys Val Cys Asn Met Ala Arg Asp Cys Arg Asp
    290                 295                 300

Trp Ser Asp Glu Pro Ile Lys Glu Cys Gly Thr Asn Glu Cys Leu Asp
305                 310                 315                 320

Asn Asn Gly Gly Cys Ser His Val Cys Asn Asp Leu Lys Ile Gly Tyr
                325                 330                 335

Glu Cys Leu Cys Pro Asp Gly Phe Gln Leu Val Ala Gln Arg Arg Cys
            340                 345                 350

Glu Asp Ile Asp Glu Cys Gln Asp Pro Asp Thr Cys Ser Gln Leu Cys
        355                 360                 365

Val Asn Leu Glu Gly Gly Tyr Lys Cys Gln Cys Glu Glu Gly Phe Gln
    370                 375                 380

Leu Asp Pro His Thr Lys Ala Cys Lys Ala Val Gly Ser Ile Ala Tyr
385                 390                 395                 400

Leu Phe Phe Thr Asn Arg His Glu Val Arg Lys Met Thr Leu Asp Arg
                405                 410                 415

Ser Glu Tyr Thr Ser Leu Ile Pro Asn Leu Arg Asn Val Val Ala Leu
            420                 425                 430

Asp Thr Glu Val Ala Ser Asn Arg Ile Tyr Trp Ser Asp Leu Ser Gln
        435                 440                 445

Arg Met Ile Cys Ser Thr Gln Leu Asp Arg Ala His Gly Val Ser Ser
    450                 455                 460

Tyr Asp Thr Val Ile Ser Arg Asp Ile Gln Ala Pro Asp Gly Leu Ala
465                 470                 475                 480

Val Asp Trp Ile His Ser Asn Ile Tyr Trp Thr Asp Ser Val Leu Gly
                485                 490                 495

Thr Val Ser Val Ala Asp Thr Lys Gly Val Lys Arg Lys Thr Leu Phe
            500                 505                 510

Arg Glu Asn Gly Ser Lys Pro Arg Ala Ile Val Val Asp Pro Val His
        515                 520                 525

Gly Phe Met Tyr Trp Thr Asp Trp Gly Thr Pro Ala Lys Ile Lys Lys
    530                 535                 540

Gly Gly Leu Asn Gly Val Asp Ile Tyr Ser Leu Val Thr Glu Asn Ile
545                 550                 555                 560

Gln Trp Pro Asn Gly Ile Thr Leu Asp Leu Leu Ser Gly Arg Leu Tyr
                565                 570                 575

Trp Val Asp Ser Lys Leu His Ser Ile Ser Ser Ile Asp Val Asn Gly
            580                 585                 590

Gly Asn Arg Lys Thr Ile Leu Glu Asp Glu Lys Arg Leu Ala His Pro
        595                 600                 605

Phe Ser Leu Ala Val Phe Glu Asp Lys Val Phe Trp Thr Asp Ile Ile
    610                 615                 620

Asn Glu Ala Ile Phe Ser Ala Asn Arg Leu Thr Gly Ser Asp Val Asn
625                 630                 635                 640

Leu Leu Ala Glu Asn Leu Leu Ser Pro Glu Asp Met Val Leu Phe His

645                 650                 655

Asn Leu Thr Gln Pro Arg Gly Val Asn Trp Cys Glu Arg Thr Thr Leu
            660                 665                 670

Ser Asn Gly Gly Cys Gln Tyr Leu Cys Leu Pro Ala Pro Gln Ile Asn
        675                 680                 685

Pro His Ser Pro Lys Phe Thr Cys Ala Cys Pro Asp Gly Met Leu Leu
    690                 695                 700

Ala Arg Asp Met Arg Ser Cys Leu Thr Glu Ala Glu Ala Ala Val Ala
705                 710                 715                 720

Thr Gln Glu Thr Ser Thr Val Arg Leu Lys Val Ser Ser Thr Ala Val
                725                 730                 735

Arg Thr Gln His Thr Thr Thr Arg Pro Val Pro Asp Thr Ser Arg Leu
            740                 745                 750

Pro Gly Ala Thr Pro Gly Leu Thr Thr Val Glu Ile Val Thr Met Ser
        755                 760                 765

His Gln Ala Leu Gly Asp Val Ala Gly Arg Gly Asn Glu Lys Lys Pro
    770                 775                 780

Ser Ser Val Arg Asp Tyr Lys Asp Asp Asp Asp Lys His His His His
785                 790                 795                 800

His His

<210>  8
<211>  331
<212>  PRT
<213>  ARTIFICIAL

<220>
<223>  Fusion protein of truncated LDLR extracellular domain and hIgG1 Fc
(with PCSK9 binding activity): LDLR-sECD-Fc (hIgG1) as a detection reagent

<400>  8

Met Glu Phe Gly Leu Ser Trp Leu Phe Leu Val Ala Ile Leu Lys Gly
1               5                   10                  15

Val Gln Cys Gly Thr Asn Glu Cys Leu Asp Asn Asn Gly Gly Cys Ser
            20                  25                  30

His Val Cys Asn Asp Leu Lys Ile Gly Tyr Glu Cys Leu Cys Pro Asp

35                  40                  45

Gly Phe Gln Leu Val Ala Gln Arg Arg Cys Glu Asp Ile Asp Glu Cys
    50                  55                  60

Gln Asp Pro Asp Thr Cys Ser Gln Leu Cys Val Asn Leu Glu Gly Gly
65                  70                  75                  80

Tyr Lys Cys Gln Cys Glu Glu Gly Phe Gln Leu Asp Pro His Thr Lys
                85                  90                  95

Ala Cys Lys Glu Pro Lys Ser Ser Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Asp
225                 230                 235                 240

Glu Leu Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210>  9
<211>  294
<212>  PRT
<213>  ARTIFICIAL

<220>
<223>  Fusion protein of further truncated LDLR extracellular domain and
hIgG1 Fc (with PCSK9 binding activity): LDLR-ssECD-Fc (hIgG1) as a detection
reagent

<400>  9

Met Glu Phe Gly Leu Ser Trp Leu Phe Leu Val Ala Ile Leu Lys Gly
1               5                   10                  15

Val Gln Cys Gly Thr Asn Glu Cys Leu Asp Asn Asn Gly Gly Cys Ser
            20                  25                  30

His Val Cys Asn Asp Leu Lys Ile Gly Tyr Glu Cys Leu Cys Pro Asp
        35                  40                  45

Gly Phe Gln Leu Val Ala Gln Arg Arg Cys Glu Asp Ile Asp Glu Pro
    50                  55                  60

Lys Ser Ser Asp Lys Thr His Thr Cys Pro Pro Cys Pro Ala Pro Glu
65                  70                  75                  80

Leu Leu Gly Gly Pro Ser Val Phe Leu Phe Pro Pro Lys Pro Lys Asp
                85                  90                  95

Thr Leu Met Ile Ser Arg Thr Pro Glu Val Thr Cys Val Val Val Asp
            100                 105                 110

Val Ser His Glu Asp Pro Glu Val Lys Phe Asn Trp Tyr Val Asp Gly
        115                 120                 125

Val Glu Val His Asn Ala Lys Thr Lys Pro Arg Glu Glu Gln Tyr Asn
    130                 135                 140

Ser Thr Tyr Arg Val Val Ser Val Leu Thr Val Leu His Gln Asp Trp
145                 150                 155                 160

Leu Asn Gly Lys Glu Tyr Lys Cys Lys Val Ser Asn Lys Ala Leu Pro
                165                 170                 175

Ala Pro Ile Glu Lys Thr Ile Ser Lys Ala Lys Gly Gln Pro Arg Glu
            180                 185                 190

Pro Gln Val Tyr Thr Leu Pro Pro Ser Arg Asp Glu Leu Thr Lys Asn
        195                 200                 205

Gln Val Ser Leu Thr Cys Leu Val Lys Gly Phe Tyr Pro Ser Asp Ile
    210                 215                 220

Ala Val Glu Trp Glu Ser Asn Gly Gln Pro Glu Asn Asn Tyr Lys Thr
225                 230                 235                 240

Thr Pro Pro Val Leu Asp Ser Asp Gly Ser Phe Phe Leu Tyr Ser Lys
                245                 250                 255

Leu Thr Val Asp Lys Ser Arg Trp Gln Gln Gly Asn Val Phe Ser Cys
            260                 265                 270

Ser Val Met His Glu Ala Leu His Asn His Tyr Thr Gln Lys Ser Leu
        275                 280                 285

Ser Leu Ser Pro Gly Lys
    290

<210>  10
<211>  121
<212>  PRT
<213>  ARTIFICIAL

<220>
<223>  h011-1 VH

<400>  10

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Tyr Ile Asn Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120

<210>  11
<211>  107
<212>  PRT
<213>  ARTIFICIAL

<220>
<223>  h011-1 VL

<400>  11

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ala Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Tyr
                85                  90                  95

Thr Phe Gly Gly Gly Thr Lys Val Glu Ile Lys
            100                 105

<210>  12
<211>  121
<212>  PRT
<213>  ARTIFICIAL

<220>
<223>  h011-3 VH

<400>  12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Tyr Ile Asn Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120

<210>  13
<211>  107
<212>  PRT
<213>  ARTIFICIAL

<220>
<223>  h011-3 VL

<400>  13

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105

<210>  14
<211>  5
<212>  PRT
<213>  Murine

<400>  14

Gly Tyr Thr Ile His
1               5

<210>  15
<211>  17
<212>  PRT
<213>  Murine

<400>  15

Tyr Ile Asn Pro Ser Ser Thr Tyr Thr Lys Phe Asn Gln Lys Phe Lys
1               5                   10                  15

Asp

<210>  16
<211>  14
<212>  PRT
<213>  Murine

<400>  16

Ala Arg Glu Arg Ile Tyr Ser Asn Tyr Trp Phe Phe Asp Val
1               5                   10

<210>  17
<211>  11
<212>  PRT
<213>  Murine

<400>  17

Lys Ala Ser Gln Asn Val Tyr Thr Ala Val Ala
1               5                   10

<210>  18
<211>  7
<212>  PRT
<213>  Murine

<400>  18

Ser Ala Ser Asn Arg Tyr Thr
1               5

<210>  19
<211>  9
<212>  PRT
<213>  Murine

<400>  19

Gln Gln Tyr Ser Ser Tyr Pro Tyr Thr
1               5

<210>  20
<211>  5
<212>  PRT
<213>  Artificial

<220>
<223>  HCDR1-1

<400>  20

Gly Tyr Asp Ile His
1               5

<210>  21
<211>  5
<212>  PRT
<213>  Artificial

<220>
<223>  HCDR1-2

<400>  21

Gly Tyr Glu Ile His
1               5

<210>  22
<211>  17
<212>  PRT
<213>  Artificial

<220>
<223>  HCDR2-1

<400>  22

Glu Ile Leu Pro Ser Ser Thr Tyr Thr Lys Phe Asn Gln Lys Phe Lys
1               5                   10                  15

Asp

<210>  23
<211>  17

<212>  PRT
<213>  Artificial

<220>
<223>  HCDR2-2

<400>  23

Glu Ile Asn Pro Ser Gly Thr Tyr Thr Lys Phe Asn Gln Lys Phe Lys
1               5                   10                  15

Asp

<210>  24
<211>  17
<212>  PRT
<213>  Artificial

<220>
<223>  HCDR2-3

<400>  24

Tyr Ile Asn Pro Ser Ala Thr Tyr Thr Lys Phe Asn Gln Lys Phe Lys
1               5                   10                  15

Asp

<210>  25
<211>  17
<212>  PRT
<213>  Artificial

<220>
<223>  HCDR2-4

<400>  25

Glu Ile Ile Pro Ser Ser Thr Tyr Thr Lys Phe Asn Gln Lys Phe Lys
1               5                   10                  15

Asp

<210>  26
<211>  17
<212>  PRT
<213>  Artificial

<220>
<223>  HCDR2-5

<400>  26

Glu Ile Asn Pro Ser Ser Thr Tyr Thr Lys Phe Asn Gln Lys Phe Lys

1               5                   10                  15

Asp

<210>  27
<211>  17
<212>  PRT
<213>  Artificial

<220>
<223>  HCDR2-6

<400>  27

Tyr Ile Val Pro Ser Ser Thr Tyr Thr Lys Phe Asn Gln Lys Phe Lys
1               5                   10                  15

Asp

<210>  28
<211>  14
<212>  PRT
<213>  Artificial

<220>
<223>  HCDR3-1

<400>  28

Ala Arg Glu Asn Ile Tyr Phe Asn Tyr Trp Phe Phe Asp Arg
1               5                   10

<210>  29
<211>  14
<212>  PRT
<213>  Artificial

<220>
<223>  HCDR3-2

<400>  29

Ala Arg Glu Asn Ile Phe Ser Asn Tyr Trp Phe Phe Asp Arg
1               5                   10

<210>  30
<211>  14
<212>  PRT
<213>  Artificial

<220>
<223>  HCDR3-3

<400>  30

Ala Arg Glu Arg Ile Phe Ser Asn Tyr Trp Phe Phe Asp Val
1               5                   10

<210>  31
<211>  11
<212>  PRT
<213>  Artificial

<220>
<223>  LCDR1-1

<400>  31

Lys Ala Ser Gln Asn Val Tyr Trp Glu Val Asp
1               5                   10

<210>  32
<211>  11
<212>  PRT
<213>  Artificial

<220>
<223>  LCDR1-2

<400>  32

Lys Ala Ser Gln Asn Val Tyr Trp Glu Val Val
1               5                   10

<210>  33
<211>  11
<212>  PRT
<213>  Artificial

<220>
<223>  LCDR1-3

<400>  33

Lys Ala Ser Gln Asn Val Tyr Thr Glu Val Ala
1               5                   10

<210>  34
<211>  11
<212>  PRT
<213>  Artificial

<220>
<223>  LCDR1-4

<400>  34

Lys Ala Ser Gln Asn Val Tyr Thr Ala Val Asp
1               5                   10

<210>  35
<211>  7
<212>  PRT

<213>  Artificial

<220>
<223>  LCDR2-1

<400>  35

Glu Met Val Asn Arg Tyr Thr
1               5


<210>  36
<211>  7
<212>  PRT
<213>  Artificial

<220>
<223>  LCDR2-2

<400>  36

Glu Ala Ser Asn Arg Tyr Thr
1               5


<210>  37
<211>  7
<212>  PRT
<213>  Artificial

<220>
<223>  LCDR2-3

<400>  37

Gln Ala Ser Asn Arg Tyr Thr
1               5


<210>  38
<211>  9
<212>  PRT
<213>  Artificial

<220>
<223>  LCDR3-1

<400>  38

Gln Gln Phe Ser Trp Phe Pro Tyr Thr
1               5


<210>  39
<211>  9
<212>  PRT
<213>  Artificial

<220>
<223>  LCDR3-2

<400>  39

Gln Gln Leu Ser Ser Gln Pro Glu Thr
1               5

<210>  40
<211>  9
<212>  PRT
<213>  Artificial

<220>
<223>  LCDR3-3

<400>  40

Gln Gln Leu Ser Ser Tyr Pro Asp Thr
1               5

<210>  41
<211>  9
<212>  PRT
<213>  Artificial

<220>
<223>  LCDR3-4

<400>  41

Gln Gln Leu Ser Ser Ser Pro Glu Thr
1               5

<210>  42
<211>  9
<212>  PRT
<213>  Artificial

<220>
<223>  LCDR3-5

<400>  42

Gln Gln Leu Ser Ser Tyr Pro Tyr Thr
1               5

<210>  43
<211>  5
<212>  PRT
<213>  Artificial

<220>
<223>  Synthetic peptide

<220>
<221>  misc_feature
<222>  (3)..(3)
<223>  Xaa is Thr, Asp or Glu

<400>  43

Gly Tyr Xaa Ile His

1                   5


<210>  44
<211>  17
<212>  PRT
<213>  Artificial

<220>
<223>  Synthetic peptide


<220>
<221>  misc_feature
<222>  (1)..(1)
<223>  Xaa is Tyr or Glu

<220>
<221>  misc_feature
<222>  (3)..(3)
<223>  Xaa is Asn,Leu,Ile or Val

<220>
<221>  misc_feature
<222>  (6)..(6)
<223>  Xaa is Ser,Gly or Ala

<400>  44

Xaa Ile Xaa Pro Ser Xaa Thr Tyr Thr Lys Phe Asn Gln Lys Phe Lys
1               5                   10                  15


Asp




<210>  45
<211>  14
<212>  PRT
<213>  Artificial

<220>
<223>  Synthetic peptide


<220>
<221>  misc_feature
<222>  (4)..(4)
<223>  Xaa is Arg or Asn

<220>
<221>  misc_feature
<222>  (6)..(6)
<223>  Xaa is Tyr or Phe

<220>
<221>  misc_feature
<222>  (7)..(7)
<223>  Xaa is Ser or Phe

<220>
<221>  misc_feature

<222>  (14)..(14)
<223>  Xaa is Val or Arg

<400>  45

Ala Arg Glu Xaa Ile Xaa Xaa Asn Tyr Trp Phe Phe Asp Xaa
1               5                   10


<210>  46
<211>  11
<212>  PRT
<213>  Artificial

<220>
<223>  Synthetic peptide


<220>
<221>  misc_feature
<222>  (8)..(8)
<223>  Xaa is Thr or Trp

<220>
<221>  misc_feature
<222>  (9)..(9)
<223>  Xaa is Ala or Glu

<220>
<221>  misc_feature
<222>  (11)..(11)
<223>  Xaa is Ala,Asp or Val

<400>  46

Lys Ala Ser Gln Asn Val Tyr Xaa Xaa Val Xaa
1               5                   10


<210>  47
<211>  7
<212>  PRT
<213>  Artificial

<220>
<223>  Synthetic peptide


<220>
<221>  misc_feature
<222>  (1)..(1)
<223>  Xaa is Ser, Glu or Gln

<220>
<221>  misc_feature
<222>  (2)..(2)
<223>  Xaa is Ala or Met

<220>
<221>  misc_feature
<222>  (3)..(3)
<223>  Xaa is Ser or Val

<400>  47

Xaa Xaa Xaa Asn Arg Tyr Thr
1               5


<210>  48
<211>  9
<212>  PRT
<213>  Artificial

<220>
<223>  Synthetic peptide


<220>
<221>  misc_feature
<222>  (3)..(3)
<223>  Xaa is Tyr,Phe or Leu

<220>
<221>  misc_feature
<222>  (5)..(5)
<223>  Xaa is Ser or Trp

<220>
<221>  misc_feature
<222>  (6)..(6)
<223>  Xaa is Tyr,Phe,Gln or Ser

<220>
<221>  misc_feature
<222>  (8)..(8)
<223>  Xaa is Tyr,Asp or Glu

<400>  48

Gln Gln Xaa Ser Xaa Xaa Pro Xaa Thr
1               5


<210>  49
<211>  121
<212>  PRT
<213>  Artificial

<220>
<223>  h011-3050-VH

<400>  49

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Gly Tyr
            20                  25                  30


Thr Ile His Trp Val Arg Gln Ala Pro Gly Gln Gly Leu Glu Trp Met
        35                  40                  45

Gly Glu Ile Leu Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120

<210>  50
<211>  121
<212>  PRT
<213>  Artificial

<220>
<223>  h011-3058-VH

<400>  50

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Glu Ile Asn Pro Ser Gly Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120

<210>  51

<211>  121
<212>  PRT
<213>  Artificial

<220>
<223>  h011-3065-VH

<400>  51

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Asp Ile His Trp Val Arg Gln Ala Pro Gly Gln Gly Leu Glu Trp Met
        35                  40                  45

Gly Tyr Ile Asn Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120


<210>  52
<211>  121
<212>  PRT
<213>  Artificial

<220>
<223>  h011-3070-VH

<400>  52

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Tyr Ile Asn Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Asn Ile Tyr Phe Asn Tyr Trp Phe Phe Asp Arg Trp Gly
            100                 105                 110

Gln Gly Thr Thr Val Thr Val Ser Ser
        115                 120

<210>  53
<211>  121
<212>  PRT
<213>  Artificial

<220>
<223>  h011-3073-VH

<400>  53

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Tyr Ile Asn Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Asn Ile Phe Ser Asn Tyr Trp Phe Phe Asp Arg Trp Gly
            100                 105                 110

Gln Gly Thr Thr Val Thr Val Ser Ser
        115                 120

<210>  54

<211>  121
<212>  PRT
<213>  Artificial

<220>
<223>  h011-3133-VH

<400>  54

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Glu Ile His Trp Val Arg Gln Ala Pro Gly Gln Gly Leu Glu Trp Met
        35                  40                  45

Gly Tyr Ile Asn Pro Ser Ala Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120


<210>  55
<211>  121
<212>  PRT
<213>  Artificial

<220>
<223>  h011-3147-VH

<400>  55

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Glu Ile Ile Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120

<210>  56
<211>  121
<212>  PRT
<213>  Artificial

<220>
<223>  h011-3195-VH

<400>  56

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Tyr Ile Asn Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Phe Ser Asn Tyr Trp Phe Phe Asp Val Trp Gly
            100                 105                 110

Gln Gly Thr Thr Val Thr Val Ser Ser
        115                 120

<210>  57

<211>  121
<212>  PRT
<213>  Artificial

<220>
<223>  h011-3190-VH

<400>  57

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Glu Ile Asn Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120


<210>  58
<211>  121
<212>  PRT
<213>  Artificial

<220>
<223>  h011-3191-VH

<400>  58

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Thr Ile His Trp Val Arg Gln Ala Pro Gly Gln Gly Leu Glu Trp Met
        35                  40                  45

Gly Tyr Ile Val Pro Ser Ser Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser
        115                 120

<210>  59
<211>  107
<212>  PRT
<213>  Artificial

<220>
<223>  h011-3093-VL

<400>  59

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Glu Met Val Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105

<210>  60
<211>  107
<212>  PRT
<213>  Artificial

<220>
<223>  h011-3095-VL

<400>  60

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Glu Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105

<210>  61
<211>  107
<212>  PRT
<213>  Artificial

<220>
<223>  h011-3111-VL

<400>  61

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Trp Glu
            20                  25                  30

Val Asp Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105

<210>  62
<211>  107
<212>  PRT
<213>  Artificial

<220>
<223>  h011-3118-VL

<400>  62

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Trp Glu
            20                  25                  30

Val Val Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105

<210>  63
<211>  107
<212>  PRT
<213>  Artificial

<220>
<223>  h011-3120-VL

<400>  63

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Phe Ser Trp Phe Pro Tyr
                85                  90                  95

Thr Phe Gly Gly Gly Thr Lys Val Glu Ile Lys
            100                 105

<210>  64
<211>  107
<212>  PRT
<213>  Artificial

<220>
<223>  h011-3121-VL

<400>  64

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Leu Ser Ser Gln Pro Glu
                85                  90                  95

Thr Phe Gly Gly Gly Thr Lys Val Glu Ile Lys
            100                 105

<210>  65
<211>  107
<212>  PRT
<213>  Artificial

<220>
<223>  h011-3174-VL

<400>  65

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Gln Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105


<210>  66
<211>  107
<212>  PRT
<213>  Artificial

<220>
<223>  h011-3181-VL

<400>  66

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Leu Ser Ser Tyr Pro Asp
                85                  90                  95

Thr Phe Gly Gly Gly Thr Lys Val Glu Ile Lys
            100                 105

<210>  67
<211>  107
<212>  PRT
<213>  Artificial

<220>
<223>  h011-3187-VL

<400>  67

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Leu Ser Ser Ser Pro Glu
                85                  90                  95

Thr Phe Gly Gly Gly Thr Lys Val Glu Ile Lys
            100                 105

<210>  68
<211>  107
<212>  PRT
<213>  Artificial

<220>
<223>  h011-3192-VL

<400>  68

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Glu
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105

<210>  69
<211>  107
<212>  PRT
<213>  Artificial

<220>
<223>  h011-3193-VL

<400>  69

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Asp Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys
            100                 105

<210>  70
<211>  107
<212>  PRT

<213>  Artificial

<220>
<223>  h011-3194-VL

<400>  70

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Leu Ser Ser Tyr Pro Tyr
                85                  90                  95

Thr Phe Gly Gly Gly Thr Lys Val Glu Ile Lys
            100                 105


<210>  71
<211>  451
<212>  PRT
<213>  Artificial

<220>
<223>  h011-3133-IgG1

<400>  71

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Glu Ile His Trp Val Arg Gln Ala Pro Gly Gln Gly Leu Glu Trp Met
        35                  40                  45

Gly Tyr Ile Asn Pro Ser Ala Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile

325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  72
<211>  1413
<212>  DNA
<213>  Artificial

<220>
<223>  h011-3133-IgG1

<400>  72
atggagtttg ggctgagctg gctttttctt gtcgcgattc ttaagggtgt ccagtgccag      60

gtgcagctgg tgcagtctgg cgccgaggtg aagaagcccg gagcatctgt gaaggtgtct     120

tgtaaggcct ctggctatac ctttaccggc tacgagatcc actgggtgcg gcaggcaccc     180

gggcagggcc tggagtggat gggctacatc aacccctctg ctacctacac caagtttaac     240

cagaagttca aggaccgggt gaccatgacc cgggacacct ctatctctac cgcctacatg     300

gagctgtctc ggctgcggtc tgacgacacc gccgtgtact actgcgcacg cgaacggatc     360

tactctaact actggttctt cgacgtgtgg ggccagggca ccaccgtgac cgtgtcttct     420

gcttcgacca agggcccatc ggtcttcccc ctggcaccct cctccaagag cacctctggg     480

ggcacagcgg ccctgggctg cctggtcaag gactacttcc ccgaaccggt gacggtgtcg     540

tggaactcag gcgccctgac cagcggcgtg cacaccttcc cggctgtcct acagtcctca     600

ggactctact ccctcagcag cgtggtgacc gtgccctcca gcagcttggg cacccagacc 660

tacatctgca acgtgaatca caagcccagc aacaccaagg tggacaagaa agttgagccc 720

aaatcttgtg acaaaactca cacatgccca ccgtgcccag cacctgaact cctgggggga 780

ccgtcagtct tcctcttccc cccaaaaccc aaggacaccc tcatgatctc ccggacccct 840

gaggtcacat gcgtggtggt ggacgtgagc cacgaagacc ctgaggtcaa gttcaactgg 900

tacgtggacg gcgtggaggt gcataatgcc aagacaaagc cgcgggagga gcagtacaac 960

agcacgtacc gtgtggtcag cgtcctcacc gtcctgcacc aggactggct gaatggcaag 1020

gagtacaagt gcaaggtctc caacaaagcc ctcccagccc ccatcgagaa aaccatctcc 1080

aaagccaaag ggcagccccg agaaccacag gtgtacaccc tgcccccatc ccgggatgag 1140

ctgaccaaga accaggtcag cctgacctgc ctggtcaaag gcttctatcc cagcgacatc 1200

gccgtggagt gggagagcaa tgggcagccg gagaacaact acaagaccac gcctcccgtg 1260

ctggactccg acggctcctt cttcctctac agcaagctca ccgtggacaa gagcaggtgg 1320

cagcagggga acgtcttctc atgctccgtg atgcatgagg ctctgcacaa ccactacacg 1380

cagaagagcc tctccctgtc tccgggtaaa tga 1413

<210> 73
<211> 214
<212> PRT
<213> Artificial

<220>
<223> h011-3133-kappa

<400> 73

Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asn Val Tyr Thr Ala
            20                  25                  30

Val Ala Trp Tyr Gln Gln Lys Pro Gly Lys Ser Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Ser Tyr Pro Tyr
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  74
<211>  711
<212>  DNA
<213>  Artificial

<220>
<223>  h011-3133-kappa

<400>  74
atggacatgc gcgtgcccgc ccagctgctg ggcctgctgc tgctgtggtt ccccggctcg      60

cgatgcgaca tcgtgatgac ccagtctccc tcatctctga gtgcctctgt tggcgaccgg     120

gtgaccatca cctgcaaagc ctctcagaac gtatacacag ccgtggcctg gtatcaacag     180

aagcccggca agtcccccaa gctgctgatt tactctgcct ctaaccggta caccggcgtg     240

ccctctcggt tctctggctc tggttctggc accgacttca ccctgactat ctcttctctg     300

cagcccgagg acttcgccac ctacttctgc cagcagtact cttcttaccc ctacaccttc     360

ggcggaggca ccaaggtgga gatcaagcgt acggtggctg caccatctgt cttcatcttc     420

ccgccatctg atgagcagtt gaaatctgga actgcctctg ttgtgtgcct gctgaataac     480

ttctatccca gagaggccaa agtacagtgg aaggtggata acgccctcca atcgggtaac     540

tcccaggaga gtgtcacaga gcaggacagc aaggacagca cctacagcct cagcagcacc     600

ctgacgctga gcaaagcaga ctacgagaaa cacaaagtct acgcctgcga agtcacccat     660

cagggcctga gctcgcccgt cacaaagagc ttcaacaggg gagagtgttg a          711

<210>  75
<211>  451
<212>  PRT
<213>  Artificial

<220>
<223>  h011-3133-IgG1-YTE

<400>  75

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Glu Ile His Trp Val Arg Gln Ala Pro Gly Gln Gly Leu Glu Trp Met
        35                  40                  45

Gly Tyr Ile Asn Pro Ser Ala Thr Tyr Thr Lys Phe Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Glu Arg Ile Tyr Ser Asn Tyr Trp Phe Phe Asp Val Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His

195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Tyr
                245                 250                 255

Ile Thr Arg Glu Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  76
<211>  1413
<212>  DNA
<213>  Artificial

<220>
<223>  h011-3133-IgG1-YTE

<400>  76
atggagtttg ggctgagctg gctttttctt gtcgcgattc ttaagggtgt ccagtgccag      60

gtgcagctgg tgcagtctgg cgccgaggtg aagaagcccg gagcatctgt gaaggtgtct     120

tgtaaggcct ctggctatac ctttaccggc tacgagatcc actgggtgcg gcaggcaccc     180

gggcagggcc tggagtggat gggctacatc aacccctctg ctacctacac caagtttaac     240

cagaagttca aggaccgggt gaccatgacc cgggacacct ctatctctac cgcctacatg     300

gagctgtctc ggctgcggtc tgacgacacc gccgtgtact actgcgcacg cgaacggatc     360

tactctaact actggttctt cgacgtgtgg ggccagggca ccaccgtgac cgtgtcttct     420

gcttcgacca agggcccatc ggtcttcccc ctggcaccct cctccaagag cacctctggg     480

ggcacagcgg ccctgggctg cctggtcaag gactacttcc ccgaaccggt gacggtgtcg     540

tggaactcag gcgccctgac cagcggcgtg cacaccttcc cggctgtcct acagtcctca     600

ggactctact ccctcagcag cgtggtgacc gtgccctcca gcagcttggg cacccagacc     660

tacatctgca acgtgaatca caagcccagc aacaccaagg tggacaagaa agttgagccc     720

aaatcttgtg acaaaactca cacatgccca ccgtgcccag cacctgaact cctgggggga     780

ccgtcagtct tcctcttccc cccaaaaccc aaggacaccc tctacatcac ccgggagcct     840

gaggtcacat gcgtggtggt ggacgtgagc cacgaagacc ctgaggtcaa gttcaactgg     900

tacgtggacg gcgtggaggt gcataatgcc aagacaaagc cgcgggagga gcagtacaac     960

agcacgtacc gtgtggtcag cgtcctcacc gtcctgcacc aggactggct gaatggcaag    1020

gagtacaagt gcaaggtctc caacaaagcc ctcccagccc ccatcgagaa aaccatctcc    1080

aaagccaaag ggcagccccg agaaccacag gtgtacaccc tgcccccatc ccgggatgag    1140

ctgaccaaga accaggtcag cctgacctgc ctggtcaaag gcttctatcc cagcgacatc    1200

gccgtggagt gggagagcaa tgggcagccg gagaacaact acaagaccac gcctcccgtg    1260

ctggactccg acggctcctt cttcctctac agcaagctca ccgtggacaa gagcaggtgg    1320

cagcagggga acgtcttctc atgctccgtg atgcatgagg ctctgcacaa ccactacacg    1380

cagaagagcc tctccctgtc tccgggtaaa tga                                 1413